# EUROPEAN PATENT SPECIFICATION

(11) **EP 3 433 623 B1**
(45) Date of publication and mention of the grant of the patent: **07.10.2026**
(21) Application number: 17717568.4
(22) Date of filing: 24.03.2017
(51) Int. Cl.: G01N 33/92

(54) **BIOMARKERS OF PROTEOPATHIES AND USES THEREOF**
BIOMARKERN VON PROTEOPATHIEN UND DEREN VERWENDUNGEN
BIOMARKERS DE PROTÉOPATHIES ET UTILISATIONS DE CEUX-CI

(30) Priority: 25.03.2016 US 201662313638 P; 09.08.2016 US 201662372523 P
(43) Date of publication of application: 30.01.2019
(73) Proprietor: Genzyme Corporation, Cambridge, MA 02141 (US); The Brigham and Women's Hospital, Inc., Boston, MA 02115 (US)
(72) Inventor: SARDI, Sergio Pablo, Bridgewater, New Jersey 08807 (US); SCHERZER, Clemens, Boston, Massachusetts 02115 (US)
(74) Representative: Mathys & Squire
(86) International application number: PCT/US2017/024012
(87) International publication number: WO 2017/165766

(56) References cited:
- WO-A1-2012/177997
- WO-A2-01/97829
- WO-A2-2012/012714
- ANONYMOUS: "Cerdelga (eliglustat), Assessment report for an initial marketing authorisation application", 1 January 2015 (2015-01-01), pages 1 - 75, XP055306076, Retrieved from the Internet <URL:http://www.ema.europa.eu/docs/en_GB/document_library/EPAR_-_Public_assessment_report/human/003724/WC500182389.pdf> [retrieved on 20160928]
- GROENER ET AL: "Plasma glucosylceramide and ceramide in type 1 Gaucher disease patients: Correlations with disease severity and response to therapeutic intervention", BIOCHIMICA ET BIOPHYSICA ACTA (BBA) - MOLECULAR AND CELL BIOLOGY OF LIPIDS, ELSEVIER, AMSTERDAM, NL, vol. 1781, no. 1-2, 5 December 2007 (2007-12-05), pages 72 - 78, XP022459150, ISSN: 1388-1981, DOI: 10.1016/J.BBALIP.2007.11.004

## Description

This invention relates to methods of molecular medicine and molecular biology.

### BACKGROUND

Proteopathies (also known as proteinopathies, protein conformation disorders, or protein misfolding diseases) are a class of diseases caused by abnormal conformation and assembly of proteins. This class of diseases includes more than 40 types of disorders including, for example, Alzheimer's disease, Parkinson's disease, and Creutzfeldt-Jakob disease. Risk factors such as advancing age, genetic mutations, cardiovascular disease, education, and brain injury can increase the likelihood of developing a proteopathy.

Treating a proteopathy in a subject can cause changes in the level of gangliosides in the subject, and ganglioside levels have been linked with treatments for conditions such as Gaucher disease (e.g. glucosylceramide or ceremide levels - see international patent publication No. WO 2012/177997; Groener et al., Biochimica et Biophysica Acta, 2007, 1781:72-78; and the CHMP assessment report for Cerdelga^{®}, 2015, reference EMA/57549/2015), Fabry disease (e.g. globotriaosylceramide levels - see international patent publication No. WO 01/97829), and neurological or neuropsychiatric diseases or conditions (e.g. sphingomyelin levels - see international patent publication No. WO 2012/012714).

### SUMMARY

The present disclosure is based, at least in part, on the discovery that sphingolipid levels are elevated in subjects having a proteinopathy. In view of this discovery, described herein are methods of determining the efficacy of a treatment for a proteopathy, diagnosing a proteopathy in a subject, determining a subject's risk of developing a proteopathy, determining the stage of a proteopathy in a subject, monitoring a proteopathy in a subject, selecting a treatment for a proteopathy for a subject, and selecting a subject for a clinical trial that include determining a level of at least one sphingolipid in a sample, including a biological fluid from the subject.

The present invention provides a method of determining the efficacy of a glucosyl ceramide synthase inhibitor treatment for Parkinson's disease in a subject having Parkinson's disease, the method comprising:
(a) providing a first sample comprising plasma obtained from a subject having Parkinson's disease at a first time point;
(b) determining the level at least one sphingolipid in the sample of (a), wherein the at least one sphingolipid is selected from the group consisting of: sphingomyelin C24:1; sphingomyelin C24:0; and sphingomyelin C23:0;
(c) providing a second sample comprising plasma obtained from the subject at a second time point, wherein the subject has been administered said treatment between the first time point and the second time point, and performing step (b) on the second sample; and
(d) identifying the administered treatment as being effective when the level(s) of the at least one sphingolipid is increased at the second time point as compared to the first time point.

In some embodiments, the method of the invention further comprises: determining the level of at least one sphingolipid selected from the group consisting of: dihexosylceramide C24:1, dihexosylceramide C24:0, dihexosylceramide C23:0, dihexosylceramide C22:0, dihexosylceramide C20:0, dihexosylceramide C18:0, dihexosylceramide C16:0, lactosylceramide C24:1, lactosylceramide C24:0, lactosylceramide C23:0, lactosylceramide C22:0, lactosylceramide C20:0, lactosylceramide C18:0, ceramide C24:1, ceramide C24:0, ceramide C23:0, ceramide C22:0, ceramide C20:0, ceramide C18:0, ceramide C16:0, ceramide C14:0, globotriaosylceramide C24:1, globotriaosylceramide C24:0, globotriaosylceramide C23:0, globotriaosylceramide C22:0, globotriaosylceramide C20:0, globotriaosylceramide C18:0, globotriaosylceramide C16:0, galactosylceramide C24:1, galactosylceramide C24:0, galactosylceramide C23:0, galactosylceramide C22:0, galactosylceramide C20:0, galactosylceramide C18:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylsphingosine; and further identifying the administered treatment as being effective when the level(s) of the at least one sphingolipid selected from the group consisting of dihexosylceramide C24:1, dihexosylceramide C24:0, dihexosylceramide C23:0, dihexosylceramide C22:0, dihexosylceramide C20:0, dihexosylceramide C18:0, dihexosylceramide C16:0, lactosylceramide C24:1, lactosylceramide C24:0, lactosylceramide C23:0, lactosylceramide C22:0, lactosylceramide C20:0, lactosylceramide C18:0, ceramide C24:1, ceramide C24:0, ceramide C23:0, ceramide C22:0, ceramide C20:0, ceramide C18:0, ceramide C16:0, ceramide C14:0, globotriaosylceramide C24:1, globotriaosylceramide C24:0, globotriaosylceramide C23:0, globotriaosylceramide C22:0, globotriaosylceramide C20:0, globotriaosylceramide C18:0, globotriaosylceramide C16:0, galactosylceramide C24:1, galactosylceramide C24:0, galactosylceramide C23:0, galactosylceramide C22:0, galactosylceramide C20:0, galactosylceramide C18:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylsphingosine is decreased as compared to level(s) of the at least one sphingolipid present in a healthy subject.

In some embodiments, the method of the invention further comprises: further identifying the administered treatment as being effective when the level(s) of the at least one sphingolipid selected from the group consisting of sphingomyelin C24:1, sphingomyelin C24:0, and sphingomyelin C23:0 is also increased as compared to level(s) of the at least one sphingolipid present in a healthy subject.

In some embodiments of the method of the invention, the glucosyl ceramide synthase inhibitor is selected from the group consisting of: (i) eliglustat; (ii) miglustat; (iii) quinuclidin-3-yl (2-(4'-fluoro-[1,1'-biphenyl]-3-yl)propan-2-yl)carbamate; (iv) (S)-quinuclidin-3-yl (2-(2-(4-fluorophenyl)thiazol-4-yl)propan-2-yl)carbamate; and (v) (S)-quinuclidin-3-yl (2-(4'-(2-methoxyethoxy)-[1,1'-biphenyl]-4-yl)propan-2-yl)carbamate; and the pharmaceutically acceptable salts and prodrugs thereof.

More generally, described herein are methods of determining the efficacy of a treatment for a proteopathy in a subject having a proteopathy that include: (a) providing a first sample including a biological fluid obtained from a subject having a proteopathy at a first time point; (b) determining the level of at least one sphingolipid in the sample of (a), wherein the at least one sphingolipid is selected from the group consisting of: dihexosylceramide C24:1; dihexosylceramide C24:0; dihexosylceramide C23:0; dihexosylceramide C22:0; dihexosylceramide C20:0; dihexosylceramide C18:0; dihexosylceramide C16:0; lactosylceramide C24:1; lactosylceramide C24:0; lactosylceramide C23:0; lactosylceramide C22:0; lactosylceramide C20:0; lactosylceramide C18:0; lactosylceramide C16:0; ceramide C24:1; ceramide C24:0; ceramide C23:0; ceramide C22:0; ceramide C20:0; ceramide C18:0; ceramide C16:0; ceramide C14:0; globotriaosylceramide C24:1; globotriaosylceramide C24:0; globotriaosylceramide C23:0; globotriaosylceramide C22:0; globotriaosylceramide C20:0; globotriaosylceramide C18:0; globotriaosylceramide C16:0; galactosylceramide C24:1; galactosylceramide C24:0; galactosylceramide C23:0; galactosylceramide C22:0; galactosylceramide C20:0; galactosylceramide C18:0; galactosylceramide C16:0; glucosylceramide C24:1; glucosylceramide C24:0; glucosylceramide C23:0; glucosylceramide C22:0; glucosylceramide C20:0; glucosylceramide C18:0; glucosylceramide C16:0; and glucosylsphingosine; (c) administering a treatment for a proteopathy to the subject; (d) providing a second sample comprising a biological fluid obtained from the subject at a second time point after step (c), and performing step (b) on the second sample; and (e) determining that the administered treatment is effective or identifying the administered treatment as being effective when the level(s) of the at least one sphingolipid is decreased at the second time point as compared to the first time point.

Some embodiments of these methods further include: further identifying the administered treatment as being effective when the level(s) of the at least one sphingolipid is also decreased as compared to level(s) of the at least one sphingolipid present in a healthy subject. In some embodiments of these methods, steps (b) and (d) include determining the level(s) of at least one sphingolipid selected from the group of: dihexosylceramide C24: 1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24: 1, glucosylceramide C24:0, glucosylceramide C23:0, glucosylceramide C22:0, glucosylceramide C20:0, glucosylceramide C18:0, and glucosylceramide C16:0. In some embodiments of these methods, steps (b) and (d) include determining the levels of at least two sphingolipids selected from the group consisting of: dihexosylceramide C24: 1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24: 1, glucosylceramide C24:0, glucosylceramide C23:0, glucosylceramide C22:0, glucosylceramide C20:0, glucosylceramide C18:0, and glucosylceramide C16:0, and the administered treatment is identified as being effective when at least one or both of the two levels is decreased at the second time point as compared to the first time point. In some embodiments of these methods, steps (b) and (d) include detecting the levels of at least three sphingolipids selected from the group consisting of: dihexosylceramide C24: 1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24: 1, glucosylceramide C24:0, glucosylceramide C23:0, glucosylceramide C22:0, glucosylceramide C20:0, glucosylceramide C18:0, and glucosylceramide C16:0, and the administered treatment is identified as being effective when at least one or all three of the three levels is decreased at the second time point as compared to the first time point. In some embodiments of these methods, steps (b) and (d) include detecting the levels of at least four sphingolipids selected from the group consisting of: dihexosylceramide C24: 1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24: 1, glucosylceramide C24:0, glucosylceramide C23:0, glucosylceramide C22:0, glucosylceramide C20:0, glucosylceramide C18:0, and glucosylceramide C16:0, and the administered treatment is identified as being effective when at least one or all four of the four levels is decreased at the second time point as compared to the first time point.

Also described are methods of determining the efficacy of a treatment for a proteopathy in a subject having a proteopathy that include: (a) providing a first sample including a biological fluid obtained from a subject having a proteopathy at a first time point; (b) determining the level at least one sphingolipid in the sample of (a), where the at least one sphingolipid is selected from the group of: sphingomyelin C24:1, sphingomyelin C24:0, sphingomyelin C23:0, sphingomyelin C22:0, sphingomyelin C20:0, sphingomyelin C18:0, and sphingomyelin C16:0; (c) administering a treatment for a proteopathy to the subject; (d) providing a second sample including a biological fluid obtained from the subject at a second time point after step (c), and performing step (b) on the second sample; and (e) identifying the administered treatment as being effective when the level(s) of the at least one sphingolipid is increased at the second time point as compared to the first time point. In some embodiments of these methods, the method further includes: further identifying the administered treatment as being effective when the level(s) of the at least one sphingolipid is also increased as compared to level(s) of the at least one sphingolipid present in a healthy subject.

Also described are methods of determining the efficacy of a treatment for a proteopathy in a subject having a proteopathy that include: (a) providing a first sample including a biological fluid obtained from a subject having a proteopathy at a first time point; (b) determining at least one of total dihexosylceramide level, total lactosylceramide level, total globotriaosylceramide level, total galactosylceramide level, total glucosylceramide level, and total phosphatidylcholine level in the sample of step (a); (c) administering a treatment for the proteopathy to the subject; (d) providing a second sample including a biological fluid obtained from the subject at a second time point after step (c), and performing step (b) on the second sample; and (e) identifying the administered treatment as being effective when at least one of the total dihexosylceramide level, the total lactosylceramide level, the total globotriaosylceramide level, the total galactosylceramide level, the total glucosylceramide level, and the total phosphatidylcholine level is decreased at the second time point as compared to the first time point. In some embodiments of these methods, the method further includes further identifying the administered treatment as being effective when at least one of the total dihexosylceramide level, the total lactosylceramide level, the total globotriaosylceramide level, level, the total galactosylceramide level, the total glucosylceramide level, and the total phosphatidylcholine level is also decreased as compared to level(s) in a healthy subject. In some embodiments of these methods, steps (b) and (d) include determining one or both of the total galactosylceramide level and the total glucosylceramide level. In some embodiments of these methods, steps (b) and (d) include determining both of the total galactosylceramide level and the total glucosylceramide level, and the administered treatment is identified as being effective when one or both of the levels is decreased at the second time point as compared to the first time point.

Also described herein are methods of determining the efficacy of a treatment for a proteopathy in a subject having a proteopathy that include: (a) providing a first sample including a biological fluid obtained from a subject having a proteopathy at a first time point; (b) determining one or both of total ceramide level and total sphingomyelin level in the sample of step (a); (c) administering a treatment for a proteopathy to the subject; (d) providing a second sample including a biological fluid obtained from the subject at a second time point after step (c), and performing step (b) on the second sample; and (e) identifying the administered treatment as being effective when one or both of the total the total ceramide level and the total sphingomyelin level is increased at the second time point as compared to the first time point. In some embodiments of these methods, the method further includes: further identifying the administered treatment as being effective when one or both of the total ceramide level and the total sphingomyelin level is also increased as compared to level(s) in a healthy subject.

Also described herein are methods of determining the efficacy of a treatment for a proteopathy in a subject having a proteopathy that includes: (a) providing a first sample including a biological fluid obtained from a subject having a proteopathy at a first time point; (b) determining the ratio of glucosylceramide C24:0 to sphingomyelin C24:0 in the sample of step (a); (c) administering a treatment for a proteopathy to the subject; (d) providing a second sample includes a biological fluid obtained from the subject at a second time point after step (c), and performing step (b) on the second sample; and (e) identifying the administered treatment as being effective when the ratio of glucosylceramide C24:0 to sphingomyelin C24:0 is decreased at the second time point as compared to the first time point. Some embodiments of these methods further include identifying the administered treatment as being effective when the ratio of glucosylceramide C24:0 to sphingomyelin C24:0 is also decreased as compared to the ratio in a healthy subject.

In some embodiments of any of the methods described herein, the subject has previously been diagnosed as having a proteopathy. In some embodiments of any of the methods described herein, the first sample and the second sample include blood, serum, plasma, or cerebrospinal fluid. In the methods of the invention, the first sample comprises plasma and the second sample comprises plasma. In some embodiments of any of the methods described herein, the administered treatment is administration of a glucosyl ceramide synthase inhibitor or a recombinant enzyme. In the methods of the invention, the administered treatment is administration of a glucosyl ceramide synthase inhibitor. In some embodiments of any of these methods, the glucosyl ceramide synthase inhibitor is selected from the group of: (i) eliglustat; (ii) miglustat; (iii) quinuclidin-3-yl (2-(4'-fluoro-[1,1'-biphenyl]-3-yl)propan-2-yl)carbamate; (iv) (S)-quinuclidin-3-yl (2-(2-(4-fluorophenyl)thiazol-4-yl)propan-2-yl)carbamate; (v) (S)-quinuclidin-3-yl (2-(4'-(2-methoxyethoxy)-[1,1'-biphenyl]-4-yl)propan-2-yl)carbamate; and pharmaceutically acceptable salt and prodrugs thereof. The recombinant enzyme may be a recombinant glucocerebrosidase (e.g., imiglucerase, velaglucerase, or taliglucerase).

Some embodiments of any of these methods further include after (e), (f) administering additional doses of the administered treatment identified as being effective to the subject. In some embodiments of these methods, the administered treatment identified as being effective is a glucosyl ceramide synthase inhibitor or a recombinant enzyme, and in step (f) the subject is administered additional doses of the glucosyl ceramide synthase inhibitor or the recombinant enzyme. In some embodiments of these methods, in step (f) the subject is administered additional doses of the glucosyl ceramide synthase inhibitor, e.g., eliglustat; miglustat; quinuclidin-3-yl (2-(4'-fluoro-[1,1'-biphenyl]-3-yl)propan-2-yl)carbamate; (S)-quinuclidin-3-yl (2-(2-(4-fluorophenyl)thiazol-4-yl)propan-2-yl)carbamate; or (S)-quinuclidin-3-yl (2-(4'-(2-methoxyethoxy)-[1,1'-biphenyl]-4-yl)propan-2-yl)carbamate; and/or the pharmaceutically acceptable salts and prodrugs thereof. In step (f) the subject may be administered additional doses of the recombinant enzyme. The recombinant enzyme may be a recombinant glucocerebrosidase, e.g., imiglucerase, velaglucerase, or taliglucerase.

Described herein are methods of diagnosing a proteopathy in a subject that include: (a) providing a sample comprising a biological fluid obtained from a subject; (b) determining the level of at least one sphingolipid in the sample of (a), wherein the at least one sphingolipid is selected from the group consisting of: dihexosylceramide C24:1; dihexosylceramide C24:0; dihexosylceramide C23:0; dihexosylceramide C22:0; dihexosylceramide C20:0; dihexosylceramide C18:0; dihexosylceramide C16:0; lactosylceramide C24:1; lactosylceramide C24:0; lactosylceramide C23:0; lactosylceramide C22:0; lactosylceramide C20:0; lactosylceramide C18:0; lactosylceramide C16:0; ceramide C24:1; ceramide C24:0; ceramide C23:0; ceramide C22:0; ceramide C20:0; ceramide C18:0; ceramide C16:0; ceramide C14:0; globotriaosylceramide C24:1; globotriaosylceramide C24:0; globotriaosylceramide C23:0; globotriaosylceramide C22:0; globotriaosylceramide C20:0; globotriaosylceramide C18:0; globotriaosylceramide C16:0; galactosylceramide C24:1; galactosylceramide C24:0; galactosylceramide C23:0; galactosylceramide C22:0; galactosylceramide C20:0; galactosylceramide C18:0; galactosylceramide C16:0; glucosylceramide C24:1; glucosylceramide C24:0; glucosylceramide C23:0; glucosylceramide C22:0; glucosylceramide C20:0; glucosylceramide C18:0; glucosylceramide C16:0; and glucosylsphingosine; and (c) identifying the subject as having a proteopathy when the level(s) of the at least one sphingolipid is elevated as compared to a control level(s).

In some embodiments of these methods, step (b) includes determining the level(s) of at least one sphingolipid selected from the group consisting of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0. In some embodiments of these methods step (b) includes determining the levels of at least two sphingolipids selected from the group consisting of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24: 1, glucosylceramide C24:0, glucosylceramide C23:0, glucosylceramide C22:0, glucosylceramide C20:0, glucosylceramide C18:0, and glucosylceramide C16:0, and the subject is identified as having a proteopathy when at least one of the two levels is elevated as compared to control level(s).

In some embodiments of any of these methods, the subject is identified as having a proteopathy when both levels are increased as compared to control levels. In some embodiments of these methods step (b) includes detecting the levels of at least three sphingolipids selected from the group consisting of: dihexosylceramide C24: 1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24: 1, glucosylceramide C24:0, glucosylceramide C23:0, glucosylceramide C22:0, glucosylceramide C20:0, glucosylceramide C18:0, and glucosylceramide C16:0, and the subject is identified as having a proteopathy when at least one of the three levels is elevated as compared to control level(s). In some embodiments of any of these methods, the subject is identified as having a proteopathy when all three levels are elevated as compared to control levels. In some embodiments of these methods, step (b) includes detecting the levels of at least four sphingolipids selected from the group consisting of: dihexosylceramide C24: 1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24: 1, glucosylceramide C24:0, glucosylceramide C23:0, glucosylceramide C22:0, glucosylceramide C20:0, glucosylceramide C18:0, and glucosylceramide C16:0, and the subject is identified as having a proteopathy when at least one of the four levels is elevated as compared to control level(s). In some embodiments of these methods, the subject is identified as having a proteopathy when all four levels are elevated as compared to control levels.

Also described are methods of diagnosing a proteopathy in a subject that include that include: (a) providing a first sample including a biological fluid obtained from a subject having a proteopathy at a first time point; (b) determining the level at least one sphingolipid in the sample of (a), where the at least one sphingolipid is selected from the group of: sphingomyelin C24:1, sphingomyelin C24:0, sphingomyelin C23:0, sphingomyelin C22:0, sphingomyelin C20:0, sphingomyelin C18:0, and sphingomyelin C16:0; and (c) identifying the subject as having a proteopathy when the level(s) of the at least one sphingolipid is decreased as compared to a control level(s).

Described herein are methods of diagnosing a subject as having a proteopathy that include: (a) providing a sample comprising a biological fluid obtained from a subject; (b) determining at least one of total dihexosylceramide level, total lactosylceramide level, total globotriaosylceramide level, total galactosylceramide level, total glucosylceramide level, and total phosphatidylcholine level in the sample of step (a); and (c) identifying the subject as having a proteopathy when at least one of the total dihexosylceramide level, the total lactosylceramide level, the total globotriaosylceramide level, the total galactosylceramide level, the total glucosylceramide level, and the total phosphatidylcholine level is increased as compared to a control level(s).

In some embodiments of these methods, step (b) includes determining one or both of the total galactosylceramide level and the total glucosylceramide level. In some embodiments of these methods, step (b) includes determining both of the total galactosylceramide level and the total glucosylceramide level, and the subject is identified as having a proteopathy when one or both of the levels is increased as compared to control level(s). In some embodiments of any of these methods, the subject is identified as having a proteopathy when both levels are increased as compared to control levels.

Described herein are methods of diagnosing a subject as having a proteopathy that include: (a) providing a sample comprising a biological fluid obtained from a subject; (b) determining one or both of the total ceramide level and the total sphingomyelin level in the sample of step (a); and (c) identifying the subject as having a proteopathy when one or both of the total ceramide level and the total sphingomyelin level is decreased as compared to a control level(s).

Described herein are methods of diagnosing a subject as having a proteopathy that include: (a) providing a sample comprising a biological fluid obtained from a subject; (b) determining the ratio of glucosylceramide C24:0 to sphingomyelin C24:0 in the sample of step (a); and (c) identifying the subject as having a proteopathy when ratio of glucosylceramide C24:0 to sphingomyelin C24:0 is decreased as compared to a control ratio.

In any of these methods, the sample may comprise blood, serum, plasma, or cerebrospinal fluid. Some embodiments of any of these methods further include: detecting a mutation in a glucocerebrosidase (GBA) gene in a sample comprising genomic DNA from the subject, and further identifying a subject having a mutation in a GBA gene as having a proteopathy. In some embodiments of these methods the mutation in a GBA gene results in the expression of a GBA protein having one or more of the following mutations: V15L, C16S, Δ36T, F37V, E41K, G46E, R48W, L66P, K79N, A90T, S107L, N117D, I119T, R120W, R120Q, P122S, M123V, D127V, R131C, R131L, T134I, D140H, K156Q, P159T, P159L, R170C, R170P, P178S, P182L, N188S, G189V, A190T, A190E, G195W, L197F, K198E, G202R, M361I, F213I, F216Y, T231R, E233Stop, insertion of M between amino acids 241 and 242, S237P, F251L, H255Q, D409H, R257Q, P266A, P266R, P266L, S271N, R285H, P289L, Y304C, Y304Stop, H311R, W312C, G325R, E326K, A341T, C342G, C342Y, V352L, R353G, G355D, R359Q, S364R, S364T, S366G, N370S, L371V, V375L, G377S, D380A, G389E, G390R, N392I, V394L, N396T, F397S, V398L, V398F, D399N, P401L, I402F, I402T, L444P, A456P, D409V, D409G, K413Q, Q414R, P415R, K425E, R433G, L444R, A446P, N462K, R463C, T491I, R496C, and R496H. In some embodiments of these methods, the mutation in a GBA gene results in the expression of a GBA protein having one or more of the following mutations: S12I, insertion of SY between amino acids 13 and 14, frameshift mutation at amino acid 14, L157Q, V460M, and K416Q. In some embodiments of these methods, the mutation in a GBA gene results in the expression of a GBA protein having one or more of the following mutations: K(-27)R, 2(-4)X, G10S, V15M, C16S, D24N, G35S, S42N, T43I, R47X, R48W, R48Q, Q73X, K74X, V78A, M85T, L105R, S107L, F109V, E111K, G113E, G113A, I119S, V121A, P122L, M123T, T134P, Y135X, A136E, K157Q, K157N, I161N, I161S, H162P, R163X, Q169X, R170P, S173X, L174F, A176D, W179X, T180P, P182T, W184R, L185F, N188K, V191G, V191E, G195E, S196P, L197P, G202E, Y205C, W209R, A210V, Y212H, F213C, Y220C, E233X, E233D, G239V, G243V, Y244H, P245H, R257X, F259L, G265D, P266A, S271N, L279P, R285C, K303I, Y304X, V305L, A309V, W312R, Y313H, D315H, A318D, P319A, T323I, L324P, G325W, R329C, F331S, L336P, C342R, W348G, G349K, Q350X, R353W, S356F, R359X, Y363C, S364N, S366N, S366T, T369M, N370K, W378G, W378X, D380N, D380H, W381X, N382K, L383R, L385P, P387L, E388X, P391L, W393R, W393L, V394L, R395C, R395P, V398I, D399Y, F411I, Y412H, Q414X, M416V, F417V, Y418C, R433S, H451R, L461P, N462S, R463P, D474Y, G478S, L480P, I489T. In some embodiments of these methods, the mutation in a GBA gene includes one or more of the following insertion mutations: 84GG, 122CC, c.153-154insTACAGC, 155-156insACAGCT, D127X, 500insT, c.8410842insTGA, 1093-1094insG, 1098insA, c.1122-1123insTG, c1326insT, c.1515_1516insAGTGAGGGCAAT, and 1562-1585ins. In some embodiments of these methods, the mutation in a GBA gene results in the expression of a GBA protein having one or more of the following deletion mutations: c.42-65del24, 72delC, 203Cdel, del205-209ACCTT, c.222-224delTAC, del255-257GCG, c.330delA, 344delA, c.533delC, 534delT, 595-596delCT, c.708delC, V214X, 793delC, 898delG, 914Cdel, c.953delT, g5255delT, L354X, c.1214delGC, 1324-1326delATT, c.1439-1445del7, 1450del2, 1447-1466del20 insTG, and c.1510delT,C,T. In some embodiments of these methods, the mutation in a GBA gene is one or more of the following splice junction mutations: IVS2 +1G>A, IVS2+1G>T, IVS4 +1G>A, IVS5 +1G>T, g.4252C>G, g.4426A>G, IVS6-1G>C, g.5230G>A, IVS8+1, IVS8(-11delC)(-14T>A), IVS9-3C>G, IVS10-1G>A R463Q, IVS10+2T>A, and IVS10(+2). In some embodiments of these methods, the mutation in a GBA gene includes a IVS2+1 mutation. In some embodiments of these methods, the mutation in a GBA gene results in the expression of a GBA protein having one or more of the following mutations: c.(-203)A>G + IVS4-2a>g, S448P, c.1379G>A c.1469A>G, g.7319T>C + g.7741T>C, c.203-204insC, RecTL. In some embodiments of these methods, the mutation in a GBA gene results in the expression of a GBA protein having a Rec1 mutation (L444P, A456P, and V460M).

Some embodiments of any of these methods further include: further detecting a level of one or more of acid beta-glucocerebrosidase, acid sphingomyelinase, acid alpha-glucosidase, galactocerebrosidase, alpha-galactosidase A, and alpha-L-iduronidase, and further identifying a subject having a decrease in the level of at least one of acid beta-glucocerebrosidase, acid sphingomyelinase, acid alpha-glucosidase, galactocerebrosidase, alpha-galactosidase A, and alpha-L-iduronidase, as compared to control level(s) as having a proteopathy. These methods may further include: after step (c): (d) administering a treatment for a proteopathy to a subject identified as having a proteopathy.

In some embodiments of these methods, the treatment is administering a glucosyl ceramide synthase inhibitor or a recombinant enzyme. In some embodiments of these methods, the glucosyl ceramide synthase inhibitor is selected from the group consisting of: (i) eliglustat; (ii) miglustat; (iii) quinuclidin-3-yl (2-(4'-fluoro-[1,1'-biphenyl]-3-yl)propan-2-yl)carbamate; (iv) (S)-quinuclidin-3-yl (2-(2-(4-fluorophenyl)thiazol-4-yl)propan-2-yl)carbamate; (v) (S)-quinuclidin-3-yl (2-(4'-(2-methoxyethoxy)-[1,1'-biphenyl]-4-yl)propan-2-yl)carbamate; and the pharmaceutically acceptable salts and prodrugs thereof.

In some embodiments of these methods, the treatment includes administering a recombinant enzyme. In some embodiments of these methods, the recombinant enzyme is a recombinant glucocerebrosidase, e.g., imiglucerase, velaglucerase, or taliglucerase.

Described herein are methods of determining a subject's risk of developing a proteopathy that include: (a) providing a sample comprising a biological fluid obtained from a subject; (b) determining the level of at least one sphingolipid in the sample of (a), wherein the at least one sphingolipid is selected from the group consisting of: dihexosylceramide C24:1; dihexosylceramide C24:0; dihexosylceramide C23:0; dihexosylceramide C22:0; dihexosylceramide C20:0; dihexosylceramide C18:0; dihexosylceramide C16:0; lactosylceramide C24:1; lactosylceramide C24:0; lactosylceramide C23:0; lactosylceramide C22:0; lactosylceramide C20:0; lactosylceramide C18:0; lactosylceramide C16:0; ceramide C24:1; ceramide C24:0; ceramide C23:0; ceramide C22:0; ceramide C20:0; ceramide C18:0; ceramide C16:0; ceramide C14:0; globotriaosylceramide C24:1; globotriaosylceramide C24:0; globotriaosylceramide C23:0; globotriaosylceramide C22:0; globotriaosylceramide C20:0; globotriaosylceramide C18:0; globotriaosylceramide C16:0; galactosylceramide C24:1; galactosylceramide C24:0; galactosylceramide C23:0; galactosylceramide C22:0; galactosylceramide C20:0; galactosylceramide C18:0; galactosylceramide C16:0; glucosylceramide C24:1; glucosylceramide C24:0; glucosylceramide C23:0; glucosylceramide C22:0; glucosylceramide C20:0; glucosylceramide C18:0; glucosylceramide C16:0; and glucosylsphingosine; and (c) identifying a subject having an elevated level(s) of the at least one sphingolipid as compared to a control level(s) as having an increased risk of developing a proteopathy.

In some embodiments of these methods, step (b) includes determining the level(s) of at least one sphingolipid selected from the group consisting of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0.

In some embodiments of these methods, step (b) includes determining the levels of at least two sphingolipids selected from the group consisting of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0, and the subject is identified as having an increased risk of developing a proteopathy when at least one of the two levels is elevated as compared to control level(s).

In some embodiments of these methods, the subject is identified as having an increased risk of developing a proteopathy when both levels are increased as compared to control levels.

In some embodiments of these methods, step (b) includes detecting the levels of at least three sphingolipids selected from the group consisting of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0, and the subject is identified as having an increased risk of developing a proteopathy when at least one of the three levels is elevated as compared to control level(s).

In some embodiments of any of these methods, the subject is identified as having an increased risk of developing a proteopathy when all three levels are elevated as compared to control levels. In some embodiments of any of these methods, step (b) includes detecting the levels of at least four sphingolipids selected from the group consisting of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0, and the subject is identified as having an increased risk of developing a proteopathy when at least one of the four levels is elevated as compared to control level(s). In some embodiments of these methods, the subject is identified as having an increased risk of developing a proteopathy when all four levels are elevated as compared to control levels.

Described herein are methods of determining a subject's risk of developing a proteopathy that include: (a) providing a sample comprising a biological fluid obtained from a subject; (b) determining the level of at least one sphingolipid in the sample of (a), wherein the at least one sphingolipid is selected from the group consisting of: sphingomyelin C24: 1, sphingomyelin C24:0, sphingomyelin C23:0, sphingomyelin C22:0, sphingomyelin C20:0, sphingomyelin C18:0, and sphingomyelin C16:0; and (c) identifying a subject having a decreased level(s) of the at least one sphingolipid as compared to a control level(s) as having an increased risk of developing a proteopathy.

Described herein are methods of determining a subject's risk of developing a proteopathy that include: (a) providing a sample comprising a biological fluid obtained from a subject; (b) determining at least one of total dihexosylceramide level, total lactosylceramide level, total globotriaosylceramide level, total galactosylceramide level, total glucosylceramide level, and total phosphatidylcholine level in the sample of step (a); (c) identifying a subject having an elevated level(s) of at least one of the total dihexosylceramide level, the total lactosylceramide level, the total globotriaosylceramide level, the total galactosylceramide level, the total glucosylceramide level, and the total phosphatidylcholine level as compared to a control level(s), as having an increased risk of developing a proteopathy.

In some embodiments of any of these methods, step (b) includes determining one or both of the total galactosylceramide level and the total glucosylceramide level. In some embodiments of these methods, step (b) includes determining both the total galactosylceramide level and the total glucosylceramide level, and the subject is identified as having an increased risk of developing a proteopathy when one or both of the levels is increased as compared to control level(s). In some embodiments of these methods, the subject is identified as having an increased risk of developing a proteopathy when both levels are increased as compared to control levels.

Described herein are methods of determining a subject's risk of developing a proteopathy that include: (a) providing a sample comprising a biological fluid obtained from a subject; (b) determining one or both of total ceramide level and total sphingomyelin level in the sample of step (a); and (c) identifying a subject having a decreased level(s) of one or both of the the total ceramide level and the total sphingomyelin level as compared to a control level(s), as having an increased risk of developing a proteopathy. Described herein are methods of determining a subject's risk of developing a proteopathy that include: (a) providing a sample comprising a biological fluid obtained from a subject; (b) determining the ratio of glucosylceramide C24:0 to sphingomyelin C24:0 in the sample of step (a); and (c) identifying a subject having an increase in the ratio of glucosylceramide C24:0 to sphingomyelin C24:0 as compared to a control ratio, as having an increased risk of developing a proteopathy.

In some embodiments of any of these methods, the sample comprises blood, serum, plasma, or cerebrospinal fluid.

Some embodiments of any of these methods further include: further detecting a mutation in a glucocerebrosidase (GBA) gene in a sample comprising genomic DNA from the subject, and further identifying a subject having a mutation in a GBA gene as having an increased risk of developing a proteopathy. In some embodiments of these methods, the mutation in a GBA gene results in the expression of a GBA protein having one or more of the following mutations: V15L, C16S, Δ36T, F37V, E41K, G46E, R48W, L66P, K79N, A90T, S107L, N117D, I119T, R120W, R120Q, P122S, M123V, D127V, R131C, R131L, T134I, D140H, K156Q, P159T, P159L, R170C, R170P, P178S, P182L, N188S, G189V, A190T, A190E, G195W, L197F, K198E, G202R, M361I, F213I, F216Y, T231R, E233 Stop, insertion of M between amino acids 241 and 242, S237P, F251L, H255Q, D409H, R257Q, P266A, P266R, P266L, S271N, R285H, P289L, Y304C, Y304Stop, H311R, W312C, G325R, E326K, A341T, C342G, C342Y, V352L, R353G, G355D, R359Q, S364R, S364T, S366G, N370S, L371V, V375L, G377S, D380A, G389E, G390R, N392I, V394L, N396T, F397S, V398L, V398F, D399N, P401L, I402F, I402T, L444P, A456P, D409V, D409G, K413Q, Q414R, P415R, K425E, R433G, L444R, A446P, N462K, R463C, T491I, R496C, and R496H. In some embodiments of these methods, the mutation in a GBA gene results in the expression of a GBA protein having one or more of the following mutations: S12I, insertion of SY between amino acids 13 and 14, frameshift mutation at amino acid 14, L157Q, V460M, and K416Q. In some embodiments of these methods, the mutation in a GBA gene results in the expression of a GBA protein having one or more of the following mutations: K(-27)R, 2(-4)X, G10S, V15M, C16S, D24N, G35S, S42N, T43I, R47X, R48W, R48Q, Q73X, K74X, V78A, M85T, L105R, S107L, F109V, E111K, G113E, G113A, I119S, V121A, P122L, M123T, T134P, Y135X, A136E, K157Q, K157N, I161N, I161S, H162P, R163X, Q169X, R170P, S173X, L174F, A176D, W179X, T180P, P182T, W184R, L185F, N188K, V191G, V191E, G195E, S196P, L197P, G202E, Y205C, W209R, A210V, Y212H, F213C, Y220C, E233X, E233D, G239V, G243V, Y244H, P245H, R257X, F259L, G265D, P266A, S271N, L279P, R285C, K303I, Y304X, V305L, A309V, W312R, Y313H, D315H, A318D, P319A, T323I, L324P, G325W, R329C, F331S, L336P, C342R, W348G, G349K, Q350X, R353W, S356F, R359X, Y363C, S364N, S366N, S366T, T369M, N370K, W378G, W378X, D380N, D380H, W381X, N382K, L383R, L385P, P387L, E388X, P391L, W393R, W393L, V394L, R395C, R395P, V398I, D399Y, F411I, Y412H, Q414X, M416V, F417V, Y418C, R433S, H451R, L461P, N462S, R463P, D474Y, G478S, L480P, I489T. In some embodiments of these methods, the mutation in a GBA gene includes one or more of the following insertion mutations: 84GG, 122CC, c.153-154insTACAGC, 155-156insACAGCT, D127X, 500insT, c.8410842insTGA, 1093-1094insG, 1098insA, c.1122-1123insTG, c1326insT, c.1515_1516insAGTGAGGGCAAT, and 1562-1585ins. In some embodiments of these methods, the mutation in a GBA gene results in the expression of a GBA protein having one or more of the following deletion mutations: c.42-65del24, 72delC, 203Cdel, del205-209ACCTT, c.222-224delTAC, del255-257GCG, c.330delA, 344delA, c.533delC, 534delT, 595-596delCT, c.708delC, V214X, 793delC, 898delG, 914Cdel, c.953delT, g5255delT, L354X, c.1214delGC, 1324-1326delATT, c.1439-1445del7, 1450del2, 1447-1466del20 insTG, and c.1510delT,C,T. In some embodiments of these methods, the mutation in a GBA gene is one or more of the following splice junction mutations: IVS2 +1G>A, IVS2+1G>T, IVS4 +1G>A, IVS5 +1G>T, g.4252C>G, g.4426A>G, IVS6-1G>C, g.5230G>A, IVS8+1, IVS8(-11delC)(-14T>A), IVS9-3C>G, IVS10-1G>A R463Q, IVS10+2T>A, and IVS10(+2). In some embodiments of these methods, the mutation in a GBA gene includes a IVS2+1 mutation. In some embodiments of these methods, the mutation in a GBA gene results in the expression of a GBA protein having one or more of the following mutations: c.(-203)A>G + IVS4-2a>g, S448P, c.1379G>A c.1469A>G, g.7319T>C + g.7741T>C, c.203-204insC, RecTL. In some embodiments of these methods, the mutation in a GBA gene results in the expression of a GBA protein having a Rec1 mutation (L444P, A456P, and V460M).

Some embodiments of any of these methods further include: further detecting a level of one or more of acid beta-glucocerebrosidase, acid sphingomyelinase, acid alpha-glucosidase, galactocerebrosidase, alpha-galactosidase A, and alpha-L-iduronidase, and further identifying a subject having a decrease in the level of at least one of acid beta-glucocerebrosidase, acid sphingomyelinase, acid alpha-glucosidase, galactocerebrosidase, alpha-galactosidase A, and alpha-L-iduronidase, as compared to control level(s), as having an increased risk of developing a proteopathy. These methods may further include after step (c): (d) administering a treatment for a proteopathy to a subject identified as having an increased risk of developing a proteopathy.

In some embodiments of these methods, the treatment is administering a glucosyl ceramide synthase inhibitor or a recombinant enzyme. In some embodiments the glucosyl ceramide synthase inhibitor is selected from the group consisting of: (i) eliglustat; (ii) miglustat; (iii) quinuclidin-3-yl (2-(4'-fluoro-[1,1'-biphenyl]-3-yl)propan-2-yl)carbamate; (iv) (S)-quinuclidin-3-yl (2-(2-(4-fluorophenyl)thiazol-4-yl)propan-2-yl)carbamate; (v) (S)-quinuclidin-3-yl (2-(4'-(2-methoxyethoxy)-[1,1'-biphenyl]-4-yl)propan-2-yl)carbamate; and the pharmaceutically acceptable salts and prodrugs thereof.

In some embodiments of these methods, the treatment is administering a recombinant enzyme, e.g. a recombinant glucocerebrosidase. In some embodiments of these methods, the recombinant glucocerebrosidase is selected from the group consisting of: imiglucerase, velaglucerase, and taliglucerase.

Described herein are methods of determining the stage of a proteopathy in a subject that include: (a) providing a sample comprising a biological fluid obtained from a subject suspected of having a proteopathy or identified as having a proteopathy; (b) determining the level of at least one sphingolipid in the sample of (a), wherein the at least one sphingolipid is selected from the group consisting of: dihexosylceramide C24:1; dihexosylceramide C24:0; dihexosylceramide C23:0; dihexosylceramide C22:0; dihexosylceramide C20:0; dihexosylceramide C18:0; dihexosylceramide C16:0; lactosylceramide C24:1; lactosylceramide C24:0; lactosylceramide C23:0; lactosylceramide C22:0; lactosylceramide C20:0; lactosylceramide C18:0; lactosylceramide C16:0; ceramide C24:1; ceramide C24:0; ceramide C23:0; ceramide C22:0; ceramide C20:0; ceramide C18:0; ceramide C16:0; ceramide C14:0; globotriaosylceramide C24:1; globotriaosylceramide C24:0; globotriaosylceramide C23:0; globotriaosylceramide C22:0; globotriaosylceramide C20:0; globotriaosylceramide C18:0; globotriaosylceramide C16:0; sphingomyelin C24:1; sphingomyelin C24:0; sphingomyelin C23:0; sphingomyelin C22:0; sphingomyelin C20:0; sphingomyelin C18:0; sphingomyelin C16:0; galactosylceramide C24:1; galactosylceramide C24:0; galactosylceramide C23:0; galactosylceramide C22:0; galactosylceramide C20:0; galactosylceramide C18:0; galactosylceramide C16:0; glucosylceramide C24:1; glucosylceramide C24:0; glucosylceramide C23:0; glucosylceramide C22:0; glucosylceramide C20:0; glucosylceramide C18:0; glucosylceramide C16:0; and glucosylsphingosine; and (c) determining the stage of the proteopathy in the subject from the at least one level.

In some embodiments of these methods, step (b) includes determining the level(s) of at least one sphingolipid selected from the group consisting of: dihexosylceramide C24:1, dihexosylceramide C24:0, dihexosylceramide C20:0, ceramide C24:0, ceramide C23:0, globotriaosylceramide C24:1, globotriaosylceramide C16:0, sphingomyelin C24:0, sphingomyelin C24:1, sphingomyelin C23:0, sphingomyelin C22:0, sphingomyelin C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0.

In some embodiments of these methods, step (b) includes determining the levels of at least two sphingolipids selected from the group consisting of: dihexosylceramide C24:1, dihexosylceramide C24:0, dihexosylceramide C20:0, ceramide C24:0, ceramide C23:0, globotriaosylceramide C24:1, globotriaosylceramide C16:0, sphingomyelin C24:0, sphingomyelin C24:1, sphingomyelin C23:0, sphingomyelin C22:0, sphingomyelin C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0, and the stage of the proteopathy in the subject is determined from at least one of the two levels. In some embodiments of these methods, the stage of the proteopathy in the subject is determined from both levels.

In some embodiments of these methods, step (b) includes detecting the levels of at least three sphingolipids selected from the group consisting of: dihexosylceramide C24:1, dihexosylceramide C24:0, dihexosylceramide C20:0, ceramide C24:0, ceramide C23:0, globotriaosylceramide C24:1, globotriaosylceramide C16:0, sphingomyelin C24:0, sphingomyelin C24:1, sphingomyelin C23:0, sphingomyelin C22:0, sphingomyelin C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0, and the stage of the proteopathy in the subject is determined from at least one of the three levels. In some embodiments of these methods, the stage of the proteopathy in the subject is determined from all three levels.

In some embodiments of these methods, step (b) includes detecting the levels of at least four sphingolipids selected from the group consisting of: dihexosylceramide C24:1, dihexosylceramide C24:0, dihexosylceramide C20:0, ceramide C24:0, ceramide C23:0, globotriaosylceramide C24:1, globotriaosylceramide C16:0, sphingomyelin C24:0, sphingomyelin C24:1, sphingomyelin C23:0, sphingomyelin C22:0, sphingomyelin C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0, and the stage of the proteopathy in the subject is determined from at least one of the four levels. In some embodiments of these methods, the stage of the proteopathy in the subject is determined from all four levels.

Described herein are methods of determining the stage of a proteopathy in a subject that include: (a) providing a sample comprising a biological fluid obtained from a subject suspected of having a proteopathy or identified as having a proteopathy; (b) determining at least one of total dihexosylceramide level, total lactosylceramide level, total ceramide level, total globotriaosylceramide level, total sphingomyelin level, total galactosylceramide level, total glucosylceramide level, and total phosphatidylcholine level in the sample of step (a); and (c) determining the stage of the proteopathy in the subject from at least one of the total dihexosylceramide level, the total lactosylceramide level, the total ceramide level, the total globotriaosylceramide level, the total sphingomyelin level, the total galactosylceramide level, the total glucosylceramide level, and the total phosphatidylcholine level.

In some embodiments of any of these methods, step (b) includes determining at least one of the total ceramide level, the total sphingomyelin level, the total galactosylceramide level, and the total glucosylceramide level. In some embodiments of any of these methods, step (b) includes determining at least two of the total ceramide level, the total sphingomyelin level, the total galactosylceramide level, and the total glucosylceramide level, and the stage of the proteopathy in the subject is determined from at least one of the two levels. In some embodiments of any of these methods, the stage of the proteopathy in the subject is determined from both levels.

In some embodiments of these methods, step (b) includes determining at least three of the total ceramide level, the total sphingomyelin level, the total galactosylceramide level, and the total glucosylceramide level, and the stage of the proteopathy in the subject is determined from at least one of the three levels. In some embodiments of these methods, the stage of the proteopathy in the subject is determined from all three levels.

In some embodiments of any of these methods, step (b) includes determining the total ceramide level, the total sphingomyelin level, the total galactosylceramide level, and the total glucosylceramide level, and the stage of the proteopathy in the subject is determined from at least one of the four levels. In some embodiments of these methods, the stage of the proteopathy in the subject is determined from all four levels.

In any of these methods, the sample may comprise blood, serum, plasma, or cerebrospinal fluid. These methods may further include: after (c): (d) administering a treatment for stage I, stage II, stage III, stage IV, or stage V of the proteopathy to a subject identified to have stage I, stage II, stage III, stage IV, or stage V of the proteopathy, respectively.

Described herein are methods of monitoring a proteopathy in a subject, that include: (a) providing a first sample comprising a biological fluid obtained from a subject having a proteopathy at a first time point; (b) determining the level at least one sphingolipid in the sample of (a), wherein the at least one sphingolipid is selected from the group consisting of: dihexosylceramide C24:1; dihexosylceramide C24:0; dihexosylceramide C23:0; dihexosylceramide C22:0; dihexosylceramide C20:0; dihexosylceramide C18:0; dihexosylceramide C16:0; lactosylceramide C24:1; lactosylceramide C24:0; lactosylceramide C23:0; lactosylceramide C22:0; lactosylceramide C20:0; lactosylceramide C18:0; lactosylceramide C16:0; ceramide C24:1; ceramide C24:0; ceramide C23:0; ceramide C22:0; ceramide C20:0; ceramide C18:0; ceramide C16:0; ceramide C14:0; globotriaosylceramide C24:1; globotriaosylceramide C24:0; globotriaosylceramide C23:0; globotriaosylceramide C22:0; globotriaosylceramide C20:0; globotriaosylceramide C18:0; globotriaosylceramide C16:0; galactosylceramide C24:1; galactosylceramide C24:0; galactosylceramide C23:0; galactosylceramide C22:0; galactosylceramide C20:0; galactosylceramide C18:0; galactosylceramide C16:0; glucosylceramide C24: 1; glucosylceramide C24:0; glucosylceramide C23:0; glucosylceramide C22:0; glucosylceramide C20:0; glucosylceramide C18:0; glucosylceramide C16:0; and glucosylsphingosine; (c) providing a second sample comprising a biological fluid obtained from the subject at a second time point after the first time point, and performing step (b) on the second sample; and (d) identifying the subject as having improving or static proteopathy when the level(s) is not elevated at the second time point as compared to the level(s) at the first time point.

In some embodiments of these methods, steps (b) and (c) include determining the level(s) of at least one sphingolipid selected from the group consisting of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0.

In some embodiments of these methods, steps (b) and (c) include determining the levels of at least two sphingolipids selected from the group consisting of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0, and the subject is identified as having improving or static proteopathy when at least one of the two levels is not elevated at the second time point as compared to the first time point. In some embodiments of these methods, the subject is identified as having improving or static proteopathy when both levels are elevated at the second time point as compared to the first time point.

In some embodiments of these methods, steps (b) and (c) include detecting the levels of at least three sphingolipids selected from the group consisting of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0, and the subject is identified as having improving or static proteopathy when at least one of the three levels is not elevated at the second time point as compared to the first time point. In some embodiments of these methods, the subject is identified as having improving or static proteopathy when all three levels are not elevated at the second time point as compared to the first time point.

In some embodiments of these methods, steps (b) and (c) include detecting the levels of at least four sphingolipids selected from the group consisting of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24: 1, glucosylceramide C24:0, glucosylceramide C23:0, glucosylceramide C22:0, glucosylceramide C20:0, glucosylceramide C18:0, and glucosylceramide C16:0, and the subject is identified as having improving or static proteopathy when at least one of the four levels is not elevated at the second time point as compared to the first time point.

In some embodiments of any of these methods, the subject is identified as having improving or static proteopathy when all four levels are not elevated at the second time point as compared to the first time point.

Described herein are methods of monitoring a proteopathy in a subject, that include: (a) providing a first sample comprising a biological fluid obtained from a subject having a proteopathy at a first time point; (b) determining the level at least one sphingolipid in the sample of (a), wherein the at least one sphingolipid is selected from the group consisting of: sphingomyelin C24:1; sphingomyelin C24:0; sphingomyelin C23:0; sphingomyelin C22:0; sphingomyelin C20:0; sphingomyelin C18:0; and sphingomyelin C16:0; (c) providing a second sample comprising a biological fluid obtained from the subject at a second time point after the first time point, and performing step (b) on the second sample; and (d) identifying the subject as having improving or static proteopathy when the level(s) is elevated at the second time point as compared to the level(s) at the first time point.

Described herein are methods of monitoring a proteopathy in a subject, that include: (a) providing a first sample comprising a biological fluid obtained from a subject having a proteopathy at a first time point; (b) determining at least one of total dihexosylceramide level, total lactosylceramide level, total globotriaosylceramide level, total galactosylceramide level, total glucosylceramide level, and total phosphatidylcholine level in the sample of step (a); (c) providing a second sample comprising a biological fluid obtained from the subject at a second time point after the first time point, and performing step (b) on the second sample; and (d) identifying the subject as having improving or static proteopathy when at least one of the total dihexosylceramide level, the total lactosylceramide level, the total globotriaosylceramide level, the total galactosylceramide level, the total glucosylceramide level, and the total phosphatidylcholine level is not elevated at the second time point as compared to the first time point.

In some embodiments of these methods, steps (b) and (c) include determining one or both of the total galactosylceramide level and the total glucosylceramide level. In some embodiments of these methods, steps (b) and (c) include determining both the total galactosylceramide level and the total glucosylceramide level, and the subject is identified as having improving or static proteopathy when one or both is not elevated at the second time point as compared to the first time point. In some embodiments of these methods, the subject is identified as having improving or static proteopathy when both levels are not elevated at the second time point as compared to the first time point.

Described herein are methods of monitoring a proteopathy in a subject, that include: (a) providing a first sample comprising a biological fluid obtained from a subject having a proteopathy at a first time point; (b) determining one or both of the total ceramide level and the total sphingomyelin level in the sample of step (a); and (c) providing a second sample comprising a biological fluid obtained from the subject at a second time point after the first time point, and performing step (b) on the second sample; and (d) identifying the subject as having improving or static proteopathy when one or both of the total ceramide level and the total sphingomyelin level is elevated at the second time point as compared to the first time point.

Described herein are methods of monitoring a proteopathy in a subject, that include: (a) providing a first sample comprising a biological fluid obtained from a subject having a proteopathy at a first time point; (b) determining the ratio of glucosylceramide C24:0 to sphingomyelin C24:0 in the sample of step (a); and (c) providing a second sample comprising a biological fluid obtained from the subject at a second time point after the first time point, and performing step (b) on the second sample; and (d) identifying the subject as having improving or static proteopathy when the ratio of glucosylceramide C24:0 to sphingomyelin C24:0 is elevated at the second time point as compared to the first time point.

In some embodiments of any of these methods, the first sample and the second sample comprise blood, plasma, serum, or cerebrospinal fluid.

Described herein are methods of selecting a treatment for a proteopathy for a subject, that include: (a) providing a sample comprising a biological fluid obtained from a subject; (b) determining the level of at least one sphingolipid in the sample of (a), wherein the at least one sphingolipid is selected from the group consisting of: dihexosylceramide C24: 1; dihexosylceramide C24:0; dihexosylceramide C23:0; dihexosylceramide C22:0; dihexosylceramide C20:0; dihexosylceramide C18:0; dihexosylceramide C16:0; lactosylceramide C24:1; lactosylceramide C24:0; lactosylceramide C23:0; lactosylceramide C22:0; lactosylceramide C20:0; lactosylceramide C18:0; lactosylceramide C16:0; ceramide C24:1; ceramide C24:0; ceramide C23:0; ceramide C22:0; ceramide C20:0; ceramide C18:0; ceramide C16:0; ceramide C14:0; globotriaosylceramide C24:1; globotriaosylceramide C24:0; globotriaosylceramide C23:0; globotriaosylceramide C22:0; globotriaosylceramide C20:0; globotriaosylceramide C18:0; globotriaosylceramide C16:0; galactosylceramide C24:1; galactosylceramide C24:0; galactosylceramide C23:0; galactosylceramide C22:0; galactosylceramide C20:0; galactosylceramide C18:0; galactosylceramide C16:0; glucosylceramide C24:1; glucosylceramide C24:0; glucosylceramide C23:0; glucosylceramide C22:0; glucosylceramide C20:0; glucosylceramide C18:0; glucosylceramide C16:0; and glucosylsphingosine; and (c) selecting a treatment for a proteopathy for a subject when the level(s) of the at least one sphingolipid is elevated as compared to a control level(s).

In some embodiments of these methods, step (b) includes determining the level(s) of at least one sphingolipid selected from the group consisting of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0.

In some embodiments of these methods, step (b) includes determining the levels of at least two sphingolipids selected from the group consisting of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0, and a treatment for a proteopathy is selected for a subject when at least one of the two levels is elevated as compared to control level(s). In some embodiments of these methods, a treatment for a proteopathy is selected for the subject when both levels are increased as compared to control levels.

In some embodiments of any of these methods, step (b) includes detecting the levels of at least three sphingolipids selected from the group consisting of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0, and a treatment for a proteopathy is selected for a subject when at least one of the three levels is elevated as compared to control level(s). In some embodiments of these methods a treatment for a proteopathy is selected for the subject when all three levels are increased as compared to control levels.

In some embodiments of any of these methods, step (b) includes detecting the levels of at least four sphingolipids selected from the group consisting of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0, and a treatment for a proteopathy is selected for the subject when at least one of the four levels is elevated as compared to control level(s). In some embodiments of these methods, a treatment for a proteopathy is selected for the subject when all four levels are increased as compared to control levels.

Described herein are methods of selecting a treatment for a proteopathy for a subject, that include: (a) providing a sample comprising a biological fluid obtained from a subject; (b) determining the level of at least one sphingolipid in the sample of (a), wherein the at least one sphingolipid is selected from the group consisting of: sphingomyelin C24:1; sphingomyelin C24:0; sphingomyelin C23:0; sphingomyelin C22:0; sphingomyelin C20:0; sphingomyelin C18:0; and sphingomyelin C16:0; and (c) selecting a treatment for a proteopathy for a subject when the level(s) of the at least one sphingolipid is decreased as compared to a control level(s).

Described herein are methods of selecting a treatment for a proteopathy for a subject, that include: (a) providing a sample comprising a biological fluid obtained from a subject; (b) determining at least one of total dihexosylceramide level, total lactosylceramide level, total globotriaosylceramide level, total galactosylceramide level, total glucosylceramide level, and total phosphatidylcholine level in the sample of step (a); (c) selecting a treatment for a proteopathy for a subject when at least one of the total dihexosylceramide level, the total lactosylceramide level, the total globotriaosylceramide level, the total galactosylceramide level, the total glucosylceramide level, and the total phosphatidylcholine level is elevated as compared to a control level(s).

In some embodiments of any of these methods, step (b) includes determining one or both of the total galactosylceramide level and the total glucosylceramide level. In some embodiments of these methods, step (b) includes determining both the total galactosylceramide level and the total glucosylceramide level, and a treatment for a proteopathy is selected for the subject when one or both of the levels is increased as compared to control level(s). In some embodiments of these methods, a treatment for a proteopathy is selected for the subject when both levels are increased as compared to control levels.

Described herein are methods of selecting a treatment for a proteopathy for a subject, that include: (a) providing a sample comprising a biological fluid obtained from a subject; (b) determining one or both of total ceramide level and total sphingomyelin level in the sample of step (a); (c) selecting a treatment for a proteopathy for a subject when one or both of the the total ceramide level and the total sphingomyelin level is decreased as compared to a control level(s).

Described herein are methods of selecting a treatment for a proteopathy for a subject, that include: (a) providing a sample comprising a biological fluid obtained from a subject; (b) determining the ratio of glucosylceramide C24:0 to sphingomyelin C24:0 in the sample of step (a); (c) selecting a treatment for a proteopathy for a subject when the ratio of glucosylceramide C24:0 to sphingomyelin C24:0 is decreased as compared to a control ratio.

In some embodiments of any of these methods, the sample comprises blood, serum, plasma, or cerebrospinal fluid. Some embodiments of any of these methods further include: further detecting a mutation in a glucocerebrosidase (GBA) gene in a sample comprising genomic DNA from the subject, and further selecting a treatment for a proteopathy for a subject having a mutation in a GBA gene. In some embodiments of these methods, the mutation in a GBA gene results in the expression of a GBA protein having one or more of the following mutations: V15L, C16S, Δ36T, F37V, E41K, G46E, R48W, L66P, K79N, A90T, S107L, N117D, I119T, R120W, R120Q, P122S, M123V, D127V, R131C, R131L, T134I, D140H, K156Q, P159T, P159L, R170C, R170P, P178S, P182L, N188S, G189V, A190T, A190E, G195W, L197F, K198E, G202R, M361I, F213I, F216Y, T231R, E233 Stop, insertion of M between amino acids 241 and 242, S237P, F251L, H255Q, D409H, R257Q, P266A, P266R, P266L, S271N, R285H, P289L, Y304C, Y304Stop, H311R, W312C, G325R, E326K, A341T, C342G, C342Y, V352L, R353G, G355D, R359Q, S364R, S364T, S366G, N370S, L371V, V375L, G377S, D380A, G389E, G390R, N392I, V394L, N396T, F397S, V398L, V398F, D399N, P401L, I402F, I402T, L444P, A456P, D409V, D409G, K413Q, Q414R, P415R, K425E, R433G, L444R, A446P, N462K, R463C, T491I, R496C, and R496H. In some embodiments of these methods, the mutation in a GBA gene results in the expression of a GBA protein having one or more of the following mutations: S12I, insertion of SY between amino acids 13 and 14, frameshift mutation at amino acid 14, L157Q, V460M, and K416Q. In some embodiments of these methods, the mutation in a GBA gene results in the expression of a GBA protein having one or more of the following mutations: K(-27)R, 2(-4)X, G10S, V15M, C16S, D24N, G35S, S42N, T43I, R47X, R48W, R48Q, Q73X, K74X, V78A, M85T, L105R, S107L, F109V, E111K, G113E, G113A, I119S, V121A, P122L, M123T, T134P, Y135X, A136E, K157Q, K157N, I161N, I161S, H162P, R163X, Q169X, R170P, S173X, L174F, A176D, W179X, T180P, P182T, W184R, L185F, N188K, V191G, V191E, G195E, S196P, L197P, G202E, Y205C, W209R, A210V, Y212H, F213C, Y220C, E233X, E233D, G239V, G243V, Y244H, P245H, R257X, F259L, G265D, P266A, S271N, L279P, R285C, K303I, Y304X, V305L, A309V, W312R, Y313H, D315H, A318D, P319A, T323I, L324P, G325W, R329C, F331S, L336P, C342R, W348G, G349K, Q350X, R353W, S356F, R359X, Y363C, S364N, S366N, S366T, T369M, N370K, W378G, W378X, D380N, D380H, W381X, N382K, L383R, L385P, P387L, E388X, P391L, W393R, W393L, V394L, R395C, R395P, V398I, D399Y, F411I, Y412H, Q414X, M416V, F417V, Y418C, R433S, H451R, L461P, N462S, R463P, D474Y, G478S, L480P, I489T. In some embodiments of these methods, the mutation in a GBA gene includes one or more of the following insertion mutations: 84GG, 122CC, c.153-154insTACAGC, 155-156insACAGCT, D127X, 500insT, c.8410842insTGA, 1093-1094insG, 1098insA, c.1122-1123insTG, c1326insT, c.1515_1516insAGTGAGGGCAAT, and 1562-1585ins. In some embodiments of these methods, the mutation in a GBA gene results in the expression of a GBA protein having one or more of the following deletion mutations: c.42-65del24, 72delC, 203Cdel, del205-209ACCTT, c.222-224delTAC, del255-257GCG, c.330delA, 344delA, c.533delC, 534delT, 595-596delCT, c.708delC, V214X, 793delC, 898delG, 914Cdel, c.953delT, g5255delT, L354X, c.1214delGC, 1324-1326delATT, c.1439-1445del7, 1450del2, 1447-1466del20 insTG, and c.1510delT,C,T. In some embodiments of these methods, the mutation in a GBA gene is one or more of the following splice junction mutations: IVS2 +1G>A, IVS2+1G>T, IVS4 +1G>A, IVS5 +1G>T, g.4252C>G, g.4426A>G, IVS6-1G>C, g.5230G>A, IVS8+1, IVS8(-11delC)(-14T>A), IVS9-3C>G, IVS10-1G>A R463Q, IVS10+2T>A, and IVS10(+2). In some embodiments of these methods, the mutation in a GBA gene includes a IVS2+1 mutation. In some embodiments of these methods, the mutation in a GBA gene results in the expression of a GBA protein having one or more of the following mutations: c.(-203)A>G + IVS4-2a>g, S448P, c.1379G>A c.1469A>G, g.7319T>C + g.7741T>C, c.203-204insC, RecTL. In some embodiments of these methods, the mutation in a GBA gene results in the expression of a GBA protein having a Rec1 mutation (L444P, A456P, and V460M).

Some embodiments of any of these methods, further include: further detecting a level of one or more of acid beta-glucocerebrosidase, acid sphingomyelinase, acid alpha-glucosidase, galactocerebrosidase, alpha-galactosidase A, and alpha-L-iduronidase, and further selecting a treatment for a proteopathy for a subject having a decrease in the level of at least one of acid beta-glucocerebrosidase, acid sphingomyelinase, acid alpha-glucosidase, galactocerebrosidase, alpha-galactosidase A, and alpha-L-iduronidase, as compared to control level(s). Some embodiments of any of these methods, further include: after step (c): (d) administering the selected treatment to the subject. In some embodiments of these methods, the selected treatment is a glucosyl ceramide synthase inhibitor or a recombinant enzyme. In some embodiments of these methods, the glucosyl ceramide synthase inhibitor is selected from the group consisting of: (i) eliglustat; (ii) miglustat; (iii) quinuclidin-3-yl (2-(4'-fluoro-[1,1'-biphenyl]-3-yl)propan-2-yl)carbamate; (iv) (S)-quinuclidin-3-yl (2-(2-(4-fluorophenyl)thiazol-4-yl)propan-2-yl)carbamate; (v) (S)-quinuclidin-3-yl (2-(4'-(2-methoxyethoxy)-[1,1'-biphenyl]-4yl)propan-2-yl)carbamate; and the pharmaceutically acceptable salts and prodrugs thereof. In some embodiments of these methods, the treatment is administering a recombinant enzyme. In some embodiments of these methods, the recombinant enzyme is a recombinant glucocerebrosidase, e.g., imiglucerase, velaglucerase, or taliglucerase.

Described herein are methods of selecting a subject for a clinical trial that include the administration of a treatment for a proteopathy that include: (a) providing a sample comprising a biological fluid obtained from a subject; (b) determining the level of at least one sphingolipid in the sample of (a), wherein the at least one sphingolipid is selected from the group consisting of: dihexosylceramide C24:1; dihexosylceramide C24:0; dihexosylceramide C23:0; dihexosylceramide C22:0; dihexosylceramide C20:0; dihexosylceramide C18:0; dihexosylceramide C16:0; lactosylceramide C24:1; lactosylceramide C24:0; lactosylceramide C23:0; lactosylceramide C22:0; lactosylceramide C20:0; lactosylceramide C18:0; lactosylceramide C16:0; ceramide C24:1; ceramide C24:0; ceramide C23:0; ceramide C22:0; ceramide C20:0; ceramide C18:0; ceramide C16:0; ceramide C14:0; globotriaosylceramide C24:1; globotriaosylceramide C24:0; globotriaosylceramide C23:0; globotriaosylceramide C22:0; globotriaosylceramide C20:0; globotriaosylceramide C18:0; globotriaosylceramide C16:0; galactosylceramide C24:1; galactosylceramide C24:0; galactosylceramide C23:0; galactosylceramide C22:0; galactosylceramide C20:0; galactosylceramide C18:0; galactosylceramide C16:0; glucosylceramide C24:1; glucosylceramide C24:0; glucosylceramide C23:0; glucosylceramide C22:0; glucosylceramide C20:0; glucosylceramide C18:0; glucosylceramide C16:0; and glucosylsphingosine; and (c) selecting a subject for participation in the clinical trial when the level(s) of the at least one sphingolipid is elevated as compared to a control level(s).

In some embodiments of these methods, step (b) includes determining a level(s) of at least one sphingolipid selected from the group consisting of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0.

In some embodiments of these methods, step (b) includes determining the levels of at least two sphingolipids selected from the group consisting of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0, and the subject is selected for participation in the clinical trial when at least one of the two levels is elevated as compared to control level(s). In some embodiments of these methods, the subject is selected for participation in the clinical trial when both levels are increased as compared to control levels.

In some embodiments of any of these methods, step (b) includes detecting the levels of at least three sphingolipids selected from the group consisting of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0, and the subject is selected for participation in the clinical trial when at least one of the three levels is elevated as compared to control level(s). In some embodiments of these methods, the subject is selected for participation in the clinical trial when all three levels are increased as compared to control levels.

In some embodiments of these methods, step (b) includes detecting the levels of at least four sphingolipids selected from the group consisting of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0, and the subject is selected for participation in the clinical trial when at least one of the four levels is elevated as compared to control level(s). In some embodiments of these methods, a subject is selected for participation in the clinical trial when all four levels are increased as compared to control levels.

Described herein are methods of selecting a subject for a clinical trial that include the administration of a treatment for a proteopathy that include: (a) providing a sample comprising a biological fluid obtained from a subject; (b) determining the level of at least one sphingolipid in the sample of (a), wherein the at least one sphingolipid is selected from the group consisting of: sphingomyelin C24:1; sphingomyelin C24:0; sphingomyelin C23:0; sphingomyelin C22:0; sphingomyelin C20:0; sphingomyelin C18:0; and sphingomyelin C16:0; and (c) selecting a subject for participation in the clinical trial when the level(s) of the at least one sphingolipid is decreased as compared to a control level(s).

Described herein are methods of selecting a subject for a clinical trial that include the administration of a treatment for a proteopathy that includes: (a) providing a sample comprising a biological fluid obtained from a subject; (b) determining at least one of total dihexosylceramide level, total lactosylceramide level, total globotriaosylceramide level, total galactosylceramide level, total glucosylceramide level, and total phosphatidylcholine level in the sample of step (a); (c) selecting a subject for participation in the clinical trial when at least one of the total dihexosylceramide level, the total lactosylceramide level, the total globotriaosylceramide level, the total galactosylceramide level, the total glucosylceramide level, and the total phosphatidylcholine level is elevated as compared to a control level(s).

In some embodiments of these methods, step (b) includes determining one or both of the total galactosylceramide level and the total glucosylceramide level. In some embodiments of these methods, step (b) includes determining both the total galactosylceramide level and the total glucosylceramide level, and the subject is selected for participation in the clinical trial when one or both of the levels is increased as compared to control level(s). In some embodiments of these methods, a subject is selected for participation in a clinical trial when both levels are increased as compared to control levels.

Described herein are methods of selecting a subject for a clinical trial that include the administration of a treatment for a proteopathy that includes: (a) providing a sample comprising a biological fluid obtained from a subject; (b) determining one or both of total ceramide level and total sphingomyelin level in the sample of step (a); and (c) selecting a subject for participation in the clinical trial when one or both of the total ceramide level and the total sphingomyelin level is decreased as compared to a control level(s).

Described herein are methods of selecting a subject for a clinical trial that include the administration of a treatment for a proteopathy that includes: (a) providing a sample comprising a biological fluid obtained from a subject; (b) determining a ratio of glucosylceramide C24:0 to sphingomyelin C24:0 in the sample of step (a); and (c) selecting a subject for participation in the clinical trial the ratio of glucosylceramide C24:0 to sphingomyelin C24:0 is increased as compared to a control ratio.

In some embodiments of any of these methods, the sample comprises blood, serum, plasma, or cerebrospinal fluid.

Some embodiments of these methods, further include: further detecting a mutation in a glucocerebrosidase (GBA) gene in a sample comprising genomic DNA from the subject, and further selecting a subject having a mutation in a GBA gene for participation in the clinical trial. In some embodiments of these methods, the mutation in a GBA gene results in the expression of a GBA protein having one or more of the following mutations: V15L, C16S, Δ36T, F37V, E41K, G46E, R48W, L66P, K79N, A90T, S107L, N117D, I119T, R120W, R120Q, P122S, M123V, D127V, R131C, R131L, T134I, D140H, K156Q, P159T, P159L, R170C, R170P, P178S, P182L, N188S, G189V, A190T, A190E, G195W, L197F, K198E, G202R, M361I, F213I, F216Y, T231R, E233Stop, insertion of M between amino acids 241 and 242, S237P, F251L, H255Q, D409H, R257Q, P266A, P266R, P266L, S271N, R285H, P289L, Y304C, Y304Stop, H311R, W312C, G325R, E326K, A341T, C342G, C342Y, V352L, R353G, G355D, R359Q, S364R, S364T, S366G, N370S, L371V, V375L, G377S, D380A, G389E, G390R, N392I, V394L, N396T, F397S, V398L, V398F, D399N, P401L, I402F, I402T, L444P, A456P, D409V, D409G, K413Q, Q414R, P415R, K425E, R433G, L444R, A446P, N462K, R463C, T491I, R496C, and R496H. In some embodiments of these methods, the mutation in a GBA gene results in the expression of a GBA protein having one or more of the following mutations: S12I, insertion of SY between amino acids 13 and 14, frameshift mutation at amino acid 14, L157Q, V460M, and K416Q. In some embodiments of these methods, the mutation in a GBA gene results in the expression of a GBA protein having one or more of the following mutations: K(-27)R, 2(-4)X, G10S, V15M, C16S, D24N, G35S, S42N, T43I, R47X, R48W, R48Q, Q73X, K74X, V78A, M85T, L105R, S107L, F109V, E111K, G113E, G113A, I119S, V121A, P122L, M123T, T134P, Y135X, A136E, K157Q, K157N, I161N, I161S, H162P, R163X, Q169X, R170P, S173X, L174F, A176D, W179X, T180P, P182T, W184R, L185F, N188K, V191G, V191E, G195E, S196P, L197P, G202E, Y205C, W209R, A210V, Y212H, F213C, Y220C, E233X, E233D, G239V, G243V, Y244H, P245H, R257X, F259L, G265D, P266A, S271N, L279P, R285C, K303I, Y304X, V305L, A309V, W312R, Y313H, D315H, A318D, P319A, T323I, L324P, G325W, R329C, F331S, L336P, C342R, W348G, G349K, Q350X, R353W, S356F, R359X, Y363C, S364N, S366N, S366T, T369M, N370K, W378G, W378X, D380N, D380H, W381X, N382K, L383R, L385P, P387L, E388X, P391L, W393R, W393L, V394L, R395C, R395P, V398I, D399Y, F411I, Y412H, Q414X, M416V, F417V, Y418C, R433S, H451R, L461P, N462S, R463P, D474Y, G478S, L480P, I489T. In some embodiments of these methods, the mutation in a GBA gene includes one or more of the following insertion mutations: 84GG, 122CC, c.153-154insTACAGC, 155-156insACAGCT, D127X, 500insT, c.8410842insTGA, 1093-1094insG, 1098insA, c.1122-1123insTG, c1326insT, c.1515_1516ins AGTGAGGGCAAT, and 1562-1585ins. In some embodiments of these methods, the mutation in a GBA gene results in the expression of a GBA protein having one or more of the following deletion mutations: c.42-65del24, 72delC, 203Cdel, del205-209ACCTT, c.222-224delTAC, del255-257GCG, c.330delA, 344delA, c.533delC, 534delT, 595-596delCT, c.708delC, V214X, 793delC, 898delG, 914Cdel, c.953delT, g5255delT, L354X, c.1214delGC, 1324-1326delATT, c.1439-1445del7, 1450del2, 1447-1466del20 insTG, and c.1510delT,C,T. In some embodiments of these methods, the mutation in a GBA gene is one or more of the following splice junction mutations: IVS2 +1G>A, IVS2+1G>T, IVS4 +1G>A, IVS5 +1G>T, g.4252C>G, g.4426A>G, IVS6-1G>C, g.5230G>A, IVS8+1, IVS8(-11delC)(-14T>A), IVS9-3C>G, IVS10-1G>A R463Q, IVS10+2T>A, and IVS10(+2). In some embodiments of these methods, the mutation in a GBA gene includes a IVS2+1 mutation. In some embodiments of these methods, the mutation in a GBA gene results in the expression of a GBA protein having one or more of the following mutations: c.(-203)A>G + IVS4-2a>g, S448P, c.1379G>A c.1469A>G, g.7319T>C + g.7741T>C, c.203-204insC, RecTL. In some embodiments of these methods, the mutation in a GBA gene results in the expression of a GBA protein having a Rec1 mutation (L444P, A456P, and V460M).

Some embodiments of any of these methods, further include: further detecting a level of one or more of acid beta-glucocerebrosidase, acid sphingomyelinase, acid alpha-glucosidase, galactocerebrosidase, alpha-galactosidase A, and alpha-L-iduronidase, and further selecting a subject having a decrease in the level of at least one of acid beta-glucocerebrosidase, acid sphingomyelinase, acid alpha-glucosidase, galactocerebrosidase, alpha-galactosidase A, and alpha-L-iduronidase, as compared to control level(s), for participation in the clinical trial.

In some embodiments of these methods, the treatment for the proteopathy is administering a glucosyl ceramide synthase inhibitor or a recombinant enzyme. In some embodiments of these methods, the glucosyl ceramide synthase inhibitor is selected from the group consisting of: (i) eliglustat; (ii) miglustat; (iii) quinuclidin-3-yl (2-(4'-fluoro-[1,1'-biphenyl]-3-yl)propan-2-yl)carbamate; (iv) (S)-quinuclidin-3-yl (2-(2-(4-fluorophenyl)thiazol-4-yl)propan-2-yl)carbamate; (v) (S)-quinuclidin-3-yl (2-(4'-(2-methoxyethoxy)-[1,1'-biphenyl]-4-yl)propan-2-yl)carbamate; and the pharmaceutically acceptable salts and prodrugs thereof.

In some embodiments of these methods, the treatment is administering a recombinant enzyme. In some embodiments of these methods, the recombinant enzyme is a recombinant glucocerebrosidase, e.g., imiglucerase, velaglucerase, or taliglucerase.

In some embodiments of any of these methods, the proteopathy is synucleinopathy. In some embodiments of these methods the synucleinopathy is Parkinson's disease. In the methods of the invention, the proteopathy is Parkinson's disease.

Also disclosed herein are methods in which the proteopathy is selected from the group consisting of: Mild Cognitive Impairment, Alzheimer's disease, Lewy body dementia, multiple system atrophy, cerebral β-amyloid angiopathy, retinal ganglion cell degeneration, prion disease, a tauopathy, frontotemporal lobar degeneration, FTLD-FUS, amyotrophic lateral sclerosis, Huntington's disease, familial British dementia, familial Danish dementia, hereditary cerebral hemorrhage with amyloidosis, CADASIL, Alexander disease, a seipinopathy, familial amyloidotic neuropathy, a serpinopathy, AL (light chain) amyloidosis, AA (secondary) amyloidosis, type II diabetes, aortic medial amyloidosis, ApoAI amyloidosis, ApoAII amyloidosis, ApoAIV amyloidosis, familial amyloidosis of the Finnish type (FAF), lysozyme amyloidosis, fibrinogen amyloidosis, dialysis amyloidosis, inclusion body myositis/myopathy, cataracts, retinitis pigmentosa with rhodopsin mutations, medullary thyroid carcinoma, cardiac atrial amyloidosis, pituitary prolactinoma, hereditary lattice corneal dystrophy, cutaneous lichen amyloidosis, Mallory bodies, corneal lactoferrin amyloidosis, pulmonary alveolar proteinosis, odontogenic (Pindborg) tumor amyloid, seminal vesicle amyloid, cystic fibrosis, sickle cell disease, and critical illness myopathy (CIM).

As used herein, the word "a" before a noun represents one or more of the particular noun. For example, the phrase "a sphingolipid" represents "one or more sphingolipids."

The term "subject" means a vertebrate, including any member of the class mammalia, including humans, domestic and farm animals, and zoo, sports or pet animals, such as mouse, rabbit, pig, sheep, goat, cattle, horse (e.g., race horse), and higher primates. In some embodiments, the subject is a human.

The term "biological fluid" means any fluid obtained from a mammalian subject (e.g., blood, plasma, serum, or other blood fractions, lymph, urine, cerebrospinal fluid, ascites, saliva, breast milk, tears, vaginal discharge, amniotic fluid, lavage, semen, glandular secretions, exudate, and contents of cysts or feces). In some instances, the biological fluid is blood, serum, or plasma. In some instances, the biological fluid is cerebrospinal fluid.

The phrase "enriching a sample for lipids" is art known and means one or more manipulations of a sample to increase the concentration of lipids. The step of enriching a sample for lipids can, for example, include one or both of the following: centrifuging the sample to remove particular matter and extracting lipids using one or more organic solvents (e.g., any of the exemplary solvents described in the Examples section). Exemplary methods of enriching a sample for lipids are described herein and additional methods are known in the art.

The term "pharmaceutically acceptable excipient" encompasses any of the standard pharmaceutical excipients, including carriers such as a phosphate buffered saline solution, water, and emulsions, such as an oil/water or water/oil emulsion, and various types of wetting agents. Pharmaceutical compositions also can include stabilizers and preservatives. For examples of carriers, stabilizers and adjuvants, see Remington's Pharmaceutical Sciences (20th ed., Mack Publishing Co. 2000).

The term "prodrug" means a pharmacological derivative of a parent drug molecule that requires biotransformation, either spontaneous or enzymatic, within the organism to release the active drug. For example, prodrugs are variations or derivatives of the quinuclidine compounds described herein that have groups cleavable under certain metabolic conditions, which when cleaved, become the quinuclidine compounds described herein, *e.g.* a compound of Formula I. Such prodrugs then are pharmaceutically active *in vivo* when they undergo solvolysis under physiological conditions or undergo enzymatic degradation. Prodrug compounds herein may be called single, double, triple, *etc.,* depending on the number of biotransformation steps required to release the active drug within the organism, and the number of functionalities present in a precursor-type form. Prodrug forms can offer advantages of solubility, tissue compatibility, or delayed release in the mammalian organism (Bundgard, Design of Prodrugs, pp. 7-9, 21-24, Elsevier, Amsterdam 1985 and Silverman, "The Organic Chemistry of Drug Design and Drug Action" pp. 352-401, Academic Press, San Diego, Calif., 1992).

Prodrugs commonly known in the art include well-known acid derivatives, such as, for example, esters prepared by reaction of acid compounds with a suitable alcohol, amides prepared by reaction of acid compounds with an amine, basic groups reacted to form an acylated base derivative, *etc..* Other prodrug derivatives may be combined with other features disclosed herein to enhance bioavailability. As such, those of skill in the art will appreciate that certain of the presently disclosed compounds having, for example, free amino or hydroxy groups can be converted into prodrugs. Prodrugs include compounds having an amino acid residue, or a polypeptide chain of two or more (e.g. two, three or four) amino acid residues which are covalently joined through peptide bonds to free amino, hydroxy or carboxylic acid groups of the presently disclosed compounds. The amino acid residues include the 20 naturally occurring amino acids commonly designated by three letter symbols and also include non-naturally occurring amino acids including, for example, 4-hydroxyproline, hydroxylysine, demosine, isodemosine, 3-methylhistidine, norvalin, beta-alanine, gamma-aminobutyric acid, citrulline, homocysteine, homoserine, ornithine and methionine sulfone. Prodrugs also include compounds having a carbonate, carbamate, amide or alkyl ester moiety covalently bonded to any of the substituents disclosed herein.

The term "pharmaceutically acceptable salt" means a pharmaceutically acceptable acid addition salt or a pharmaceutically acceptable base addition salt of a currently disclosed compound that may be administered without any resultant substantial undesirable biological effect(s) or any resultant deleterious interaction(s) with any other component of a pharmaceutical composition in which it may be contained.

The term "C₁₋₆-alkyl" means a saturated linear or branched free radical consisting essentially of 1 to 6 carbon atoms and a corresponding number of hydrogen atoms. Exemplary C₁₋₆-alkyl groups include methyl, ethyl, n-propyl, isopropyl, n-butyl, isobutyl, etc. Other C₁₋₆-alkyl groups will be readily apparent to those of skill in the art given the benefit of the present disclosure. The terms "C₁₋₃-alkyl", "C₁₋₄-alkyl", etc., have equivalent meanings, *i.e.* saturated linear or branched free radical consisting essentially of 1 to 3 (or 4) carbon atoms and a corresponding number of hydrogen atoms.

The term "C₂₋₆-alkenyl" means an unsaturated linear or branched free radical consisting essentially of 2 to 6 carbon atoms and a corresponding number of hydrogen atoms, which free radical comprises at least one carbon-carbon double bond. Exemplary C₂₋₆-alkenyl groups include ethenyl, prop-1-enyl, prop-2-enyl, isopropenyl, but-1-enyl, 2-methyl-prop-1-enyl, 2-methyl-prop-2-enyl, *etc..* Other C₂₋₆-alkenyl groups will be readily apparent to those of skill in the art given the benefit of the present disclosure.

The term "C₂₋₆-alkynyl" means an unsaturated linear or branched free radical consisting essentially of 2 to 6 carbon atoms and a corresponding number of hydrogen atoms, which free radical comprises at least one carbon-carbon triple bond. Exemplary C₂₋₆-alkynyl groups include ethynyl, prop-1-ynyl, prop-2-ynyl, but-1-ynyl, 3-methyl-but-1-ynyl, *etc.* Other C₂₋₆-alkynyl groups will be readily apparent to those of skill in the art given the benefit of the present disclosure.

The term "C₁₋₆-alkyloxy" means a saturated linear or branched free radical consisting essentially of 1 to 6 carbon atoms (and a corresponding number of hydrogen atoms) and an oxygen atom. A C₁₋₆-alkyloxy group is attached via the oxygen atom. Exemplary C₁₋₆-alkyloxy groups include methyloxy, ethyloxy, n-propyloxy, isopropyloxy, n-butyloxy, isobutyloxy, *etc..* Other C₁₋₆-alkyloxy groups will be readily apparent to those of skill in the art given the benefit of the present disclosure. The terms "C₁₋₃-alkyloxy", "C₁₋₄-alkyloxy", and the like, have an equivalent meaning, *i.e.* a saturated linear or branched free radical consisting essentially of 1 to 3 (or 4) carbon atoms (and a corresponding number of hydrogen atoms) and an oxygen atom, wherein the group is attached via the oxygen atom.

The term "C₂₋₆-alkenyloxy" means an unsaturated linear or branched free radical consisting essentially of 2 to 6 carbon atoms (and a corresponding number of hydrogen atoms) and an oxygen atom, which free radical comprises at least one carbon-carbon double bond. A C₂₋₆-alkenyloxy group is attached via the oxygen atom. An exemplary C₂₋₆-alkenyloxy group is ethenyloxy; others will be readily apparent to those of skill in the art given the benefit of the present disclosure.

The term "C₂₋₆-alkynyloxy" means an unsaturated linear or branched free radical consisting essentially of 2 to 6 carbon atoms (and a corresponding number of hydrogen atoms) and an oxygen atom, which free radical comprises at least one carbon-carbon triple bond. A C₂₋₆-alkenyloxy group is attached via the oxygen atom. An exemplary C₂₋₆-alkenyloxy group is ethynyloxy; others will be readily apparent to those of skill in the art given the benefit of the present disclosure.

The term "heteroaryl" means an aromatic free radical having 5 or 6 atoms (*i.e.* ring atoms) that form a ring, wherein 1 to 5 of the ring atoms are carbon and the remaining 1 to 5 ring atom(s) (*i.e.* hetero ring atom(s)) is selected independently from the group consisting of nitrogen, sulfur, and oxygen. Exemplary 5-membered heteroaryl groups include furyl, thienyl, thiazolyl (e.g. thiazol-2-yl), pyrazolyl, isothiazolyl, oxazolyl, isoxazolyl, pyrrolyl, triazolyl, imidazolyl, oxadiazolyl and thiadiazolyl. Exemplary 6-membered heteroaryl groups include pyridyl, pyrimidyl, pyrazinyl, pyridazinyl, 1,2,4-triazinyl, benzoxazolyl, benzothiazolyl, benzisothiazolyl, benzisoxazolyl, benzimidazolyl, *etc..* Other heteroaryl groups will be readily apparent to those of skill in the art given the benefit of the present disclosure. In general, the heteroaryl group typically is attached to the main structure via a carbon atom. However, those of skill in the art will realize that certain other atoms, *e.g.* hetero ring atoms, can be attached to the main structure.

The term "aryl" means an aromatic free radical having 5 or 6 atoms (*i.e.* ring atoms) that form a ring, wherein all of the ring atoms are carbon. An exemplary aryl group is benzyl.

The term "aliphatic" means a non-aromatic compound containing carbon and hydrogen atoms, *e.g.* containing 1 to 9 carbon atoms. Aliphatic compounds may be straight-chained or branched, may contain one or more ring structures, and may contain one or more carbon-carbon double bond (provided that the compound does not contain an unsaturated ring structure having aromatic character). Examples of aliphatic compounds include ethane, propylene, cyclobutane, cyclohexadiene, *etc..*

As used herein, the terms "halo" and "halogen" mean fluorine, chlorine, bromine, or iodine. These terms are used interchangeably and may refer to a halogen free radical group or to a halogen atom as such. Those of skill in the art will readily be able to ascertain the identification of which, in view of the context in which this term is used in the present disclosure.

The term "cyano" means a free radical having a carbon atom linked to a nitrogen atom via a triple bond. The cyano radical is attached via its carbon atom.

The term "nitro" means an NO₂ radical which is attached via its nitrogen atom.

As used herein, the terms "hydroxy" and "hydroxyl" mean an OH radical which is attached via its oxygen atom. The term "thio" means an SH radical which is attached via its sulphur atom.

The term "amino" means a free radical having a nitrogen atom and 1 or 2 hydrogen atoms. As such, the term "amino" generally refers to primary and secondary amines. In that regard, as used herein and in the appended claims, a tertiary amine is represented by the general formula RR'N-, wherein R and R' are carbon radicals that may or may not be identical. Nevertheless, the term "amino" generally may be used herein to describe a primary, secondary, or tertiary amine, and those of skill in the art will readily be able to ascertain the identification of which in view of the context in which this term is used in the present disclosure.

The term "oxo" means an oxygen radical which is attached via a double bond. Where an atom bonded to this oxygen is a carbon atom, the bond is a carbon-oxygen double bond which may be denoted as -(C=O)- and which may be referred to as a ketone.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

Other features and advantages of the invention and the disclosure will be apparent from the following detailed description and figures, and from the claims.

### DESCRIPTION OF DRAWINGS

FIG. 1 is a diagram illustrating that the combination of glucosylceramide and sphingomyelin levels in biofluids provides a biomarker that distinguishes PD from healthy controls (HC) and GBA-PD from both PD and healthy controls. The asterisks represent p < 0.01.
FIGS. 2A-B are diagrams illustrating diagnostic accuracy of sphingolipid panels. FIG. 2A illustrates the diagnostic accuracy of sphingolipid panels for diagnosing PD. FIG. 2B illustrates the diagnostic accuracy of sphingolipid panels for diagnosing PD patients carrying a GBA mutation.
FIG. 3 is a heatmap diagram illustrating how patterns of combinations of these sphingolipids can be used as "molecular barcodes" to distinguish patients with PD and patients with PD with a GBA mutation (GBA-PD) from healthy controls (HC). (Abbreviations as described in Example 1).
FIG. 4 is a graph showing the fold change in the levels of specific sphingolipid isoforms in plasma from patients having sporadic Parkinson's disease (PD) to healthy patients (HC). Fold changes are shown on a log-scale. A p value of < 0.05 is indicated with a single asterisk (*). A p value of <0.01 is indicated with double asterisks (**). (Abbreviations as described in Example 1).
FIG. 5 is a graph showing the fold change in the levels of specific sphingolipid isoforms in plasma from patients having sporadic Parkinson's disease that carry a GBA mutation (GBA-PD) to healthy patients (HC). Fold changes are shown on a log-scale. A p value of < 0.05 is indicated with a single asterisk (*). A p value of <0.01 is indicated with double asterisks (**). (Abbreviations as described in Example 1).
FIG. 6 is a graph showing the total ceramide level in cerebral spinal fluid from healthy patients (HC) (n = 59) and patients having sporadic Parkinson's disease (PD) (n = 48). A p value of <0.01 is indicated with double asterisks (**).
FIG. 7 is a graph showing the mean ratio of C24-glucoceramide to C24-sphingomyelin over time for up to 8 years in healthy patients (HC) (circles), patients having sporadic Parkinson's disease (PD) (triangles), and patients having Parkinson's disease that carry a mutation in GBA (GBA-PD) (squares).

### DETAILED DESCRIPTION

Described herein are methods of determining the efficacy of a treatment for a proteopathy, diagnosing a proteopathy in a subject, determining a subject's risk of developing a proteopathy, determining the stage of a proteopathy in a subject, monitoring a proteopathy in a subject, selecting a treatment for a proteopathy for a subject, and selecting a subject for a clinical trial that include determining a level of at least one sphingolipid in a sample including a biological fluid from the subject. The invention provides a method of determining the efficacy of a glucosyl ceramide synthase inhibitor treatment for Parkinson's disease in a subject. Non-limiting aspects of these methods are described below. As can be appreciated in the art, the various aspects described below can be used in any combination without limitation.

### Proteopathies

The methods described herein can further include a step of identifying or diagnosing a subject as having a proteopathy. Non-limiting examples of methods of diagnosing a subject as having a proteopathy are described below.

In other examples, a subject is identified as having a proteopathy based on the observation or assessment of one or more symptoms of the following symptoms in a subject: depression, thyroid problems, memory loss, difficulty concentrating, difficulty planning, difficulty problem solving, problems finishing tasks, confusion, visual and space difficulties, language problems, excess use of alcohol, vitamin deficiencies, animated expressions, tremor of appendages when at rest or extended, stiffness in limbs or neck, difficulty walking, imbalance, and loss of motor or sensory skills. A proteopathy can also be diagnosed in a subject by performing mental status testing, neuropsychological testing, brain imaging studies (e.g., computed tomography, electroencephalogram, magnetic resonance imaging, ultrasound, single-photon emission computerized tomography scan, positron emission tomography), protein analysis of cerebrospinal fuid, tonsil or brain biopsy to detect prions, and genetic testing.

Diagnostic kits for Alzheimer's disease are commercially available. Enzyme-linked immunosorbent assays (ELISAs) are commercially available for a number of protein biomarkers of Alzheimer's disease, including, for example, an oligomeric amyloid-beta ELISA (Biosensis; Thebarton, Australia), a Tau protein ELISA (Anogen; Mississauga, Ontario), a Aβ42 N-terminus, C-terminus ELISA (Mississauga, Ontario), and a β-amyloid (1-40) ELISA (AnaSpec; Fremont, CA).

Genetic testing can also be used to determine whether a subject has or has an elevated risk of developing Alzheimer's disease. For example, the detection of one or two alleles of the APOE-ε4 gene can be used to identify a subject that has or has an elevated risk of developing Alzheimer's disease (see, e.g., Kaya et al., Turk. J. Med. Sci. 45:1057-1072, 2015).

Diagnostic kits for Parkinson's disease are commercially available. Enzyme-linked immunosorbent assays (ELISAs) are commercially available for a number of protein biomarkers of Parkinson's disease, including, for example, a PARK7 ELISA (MyBioSource; San Diego, CA), a PARK2 ELISA (Fivephoton Biochemicals; San Diego, CA), and an α-synuclein ELISA (BioLegend; San Diego, CA).

Genetic testing can also be used to determine whether a subject has or has an elevated risk of developing Parkinson's disease. For example, the detection of mutations in α-synuclein, parkin, DJ1, PINK1, LRRK2, VPS₃₅, and EIF4G1 can be used to identify a subject that has or has an elevated risk of developing Parkinson's disease (see, e.g., McInerney-Leo et al., Mov. Disord. 20:1-10, 2005; Anfossi et al., J. Alzheimers Dis. 38:351-357, 2014; Dhungel et al., Neuron 85:76-87, 2015; and Deng et al., Acta Neurol. Scand. 132:73-78, 2015).

Diagnostic kits for amyotrophic lateral sclerosis (ALS) are also commercially available. ELISAs are commercially available for a number of protein markers of ALS, including, for example, a TDP-43 ELISA (Proteintech; Rosemont, IL), an ALS2 ELISA (Antibodies-Online; Atlanta, GA), and a cystatin C ELISA (Boster; Pleasanton, CA). Genetic testing can also be used to determine whether a subject has or has an elevated risk of developing ALS. For example, the detection of mutations in the SOD1 gene, ALS2 gene, SETX gene, SPG11 gene, and FUS gene can be used to identify a subject that has or has an elevated risk of developing ALS (see, e.g., Zou et al., Amyotroph. Lateral Scler Frontotemporal Degener. 17:249-252, 2016; and Eisen et al., Amyotroph Lateral Scler. 9:108-119, 2008).

Subjects having a proteopathy can be diagnosed or identified using any of the methods described herein, or any methods known in the art. A subject (e.g., a subject having a proteopathy or a subject being diagnosed or identified as having a proteopathy) in any of the methods described herein can be in utero (e.g., a fetus with a gestational age greater than 14 weeks, 15 weeks, 17 weeks, 20 weeks, 25 weeks, 30 weeks, or 35 weeks), an infant, an adolescent (between 13 and 18 years old (e.g., between 13 and 15 years old or between 15 and 18 years old), or an adult (greater than 18 years old (e.g., greater than 20, 22, 24, 26, 28, 30, 32, 34, 36, 38, 40, 45, 50, 55, 60, 65, 70, 75, 80, 85, 90, or 95 years old). A subject (e.g., a subject having a proteopathy) in any of the methods described herein can be a female (e.g., a pregnant female) or can be a male. A subject having a proteopathy may already be receiving a treatment for a proteopathy. In other examples, a subject having a proteopathy may not have received a treatment for a proteopathy. In additional examples, a subject having a proteopathy may have received a previous treatment for a proteoatphy, and the previous treatment was therapeutically unsuccessful (e.g., lead to the development of negative adverse side effects and/or did not reduce the rate of development of a proteopathy). A subject having a proteopathy may be a participant in a clinical study.

A proteopathy can be, e.g., a synucleinopathy (e.g., Parkinson's disease). Additional non-limiting examples of proteopathies include: Mild Cognitive Impairment, Alzheimer's disease, Lewy body dementia, multiple system atrophy, cerebral β-amyloid angiopathy, retinal ganglion cell degeneration, prion disease, a tauopathy, frontotemporal lobar degeneration (FTLD), FTLD-FUS, amyotrophic lateral sclerosis, Huntington's disease, familial British dementia, familial Danish dementia, hereditary cerebral hemorrhage with amyloidosis, CADASIL, Alexander disease, a seipinopathy, familial amyloidotic neuropathy, a serpinopathy, AL (light chain) amyloidosis, AA (secondary) amyloidosis, type II diabetes, aortic medial amyloidosis, ApoAI amyloidosis, ApoAII amyloidosis, ApoAIV amyloidosis, familial amyloidosis of the Finnish type (FAF), lysozyme amyloidosis, fibrinogen amyloidosis, dialysis amyloidosis, inclusion body myositis/myopathy, cataracts, retinitis pigmentosa with rhodopsin mutations, medullary thyroid carcinoma, cardiac atrial amyloidosis, pituitary prolactinoma, hereditary lattice corneal dystrophy, cutaneous lichen amyloidosis, Mallory bodies, corneal lactoferrin amyloidosis, pulmonary alveolar proteinosis, odontogenic (Pindborg) tumor amyloid, seminal vesicle amyloid, cystic fibrosis, sickle cell disease, and critical illness myopathy (CIM).

The severity of a proteopathy can be determined, e.g., by determining the severity, number, and/or frequency of symptom(s) in a subject. In other examples, the severity of a proteopathy in a subject can be determined using an art-accepted scoring system. For example, the severity of Parkinson's disease in a subject can be determined using the Unified Parkinson's disease rating scale (UPDRS), the severity of Alzheimer's disease in a subject can be determined using the Alzheimer's Disease Assessment Scale (ADAS-Cog), and the severity of ALS in a subject can be determined using the ALS Functional Rating Scale (ALSFRS) or the Revised ALSFRS (ALSFRS-R).

### Treatments for a Proteopathy

Some examples of a treatment for a proteopathy is the administration of one or more glucosyl ceramide synthase inhibitor. Non-limiting examples of glycosyl ceramide synthase inhibitors include: (i) eliglustat; (ii) miglustat; (iii) quinuclidin-3-yl (2-(4'-fluoro-[1,1'-biphenyl]-3-yl)propan-2-yl)carbamate; (iv) (S)-quinuclidin-3-yl (2-(2-(4-fluorophenyl)thiazol-4-yl)propan-2-yl)carbamate; (v) (S)-quinuclidin-3-yl (2-(4'-(2-methoxyethoxy)-[1,1'-biphenyl]-4-yl)propan-2-yl)carbamate; and the pharmaceutically acceptable salts and prodrugs thereof.

Additional examples of glucosyl ceramide synthase inhibitors are compounds of Formula I, 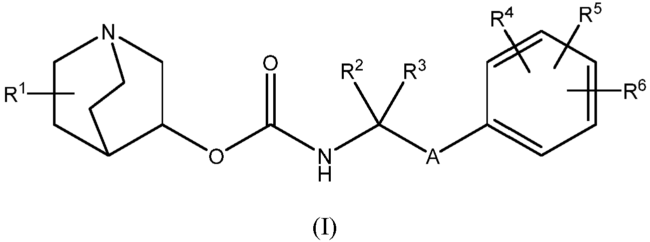 or a pharmaceutically acceptable salt or prodrug thereof, where:
R¹ is hydrogen;
   a halogen, or a cyano, nitro, hydroxy, thio, or amino group; or
   a C₁₋₆-alkyl, C₂₋₆-alkenyl, C₂₋₆-alkynyl, C₁₋₆-alkyloxy, C₂₋₆-alkenyloxy or
   C₂₋₆-alkynyloxy group, optionally substituted by one or more *(e.g.* 1, 2 or 3) groups independently selected from a halogen, and a cyano, nitro, hydroxy, thio, or amino group;
R² and R³ are each independently selected from a C₁₋₃-alkyl group, optionally substituted by one or more (*e.g.* 1, 2 or 3) halogens; or
   R² and R³ together form a cyclopropyl or cyclobutyl group, optionally substituted by one or more (*e.g.* 1 or 2) halogens;
R⁴, R⁵, and R⁶ are each independently selected from hydrogen; a halogen; a nitro, hydroxy, thio, or amino group; and a C₁₋₆-alkyl or C₁₋₆-alkyloxy group, optionally substituted by one or more (*e.g.* 1, 2, or 3) groups selected from a halogen; a hydroxy or cyano group; and a C₁₋₆-alkyloxy group; and
A is a 5- or 6-membered aryl or heteroaryl group.

In some examples of the compounds of Formula I, R¹ can be a hydrogen; a halogen; or a C₁₋₄-alkyl or C₁₋₄-alkyloxy group, optionally substituted by one or two groups selected independently from a halogen; and a cyano, nitro, hydroxy, thio or amino group. In some examples of compounds of Formula I, R¹ can be hydrogen; fluorine; or a methyl or ethyl group optionally substituted by a halogen, or a hydroxy, thio or amino group. In some examples of compounds of Formula I, R¹ can be hydrogen; or a methyl group optionally substituted by one or more (*e.g.* 1, 2, or 3) halogens. In some examples of compounds of Formula I, R¹ can be hydrogen. In some examples of the compounds of Formula I, R¹ is not attached to the nitrogen atom of the quinuclidine moiety.

In some examples of the compounds of Formula I, R² and R³ are each independently selected from C₁₋₃-alkyl groups, optionally substituted with one or more halogens. In some examples of the compounds of Formula I, R² and R³ are each independently selected from methyl and ethyl groups, optionally substituted with one or more fluorine atoms. In some examples of compounds of Formula I, R² and R³ are each methyl, optionally substituted with one to three fluorine atoms. In some examples of the compounds of Formula I, either both R² and R³ are methyl groups, or R² and R³ together form a cyclopropyl group. In some examples of the compounds of Formula I, R² and R³ are both methyl groups.

In some examples of the compounds of Formula I, R⁶ is hydrogen. In some examples of the compounds of Formula I, R⁵ and R⁶ are both hydrogen. In some examples of the compounds of Formula I, at least one of R⁴, R⁵, and R⁶ is not hydrogen. In some examples of the compounds of Formula I, R⁴ is selected from a halogen; and a C₁₋₃-alkyl or C₁₋₃-alkyloxy group, optionally substituted by one or more groups selected from a halogen; and a cyano or C₁₋₃-alkyloxy group. In some examples of the compounds of Formula I, R⁴ is selected from a halogen; and a C₁₋₃-alkyl or C₁₋₃-alkyloxy group, optionally substituted by one or more groups selected from a halogen; and a C₁₋₃-alkyloxy group. In some examples of the compounds of Formula I, R⁴ is selected from fluorine; and a C₁₋₃-alkyloxy group, optionally substituted by one or more groups selected from a halogen; and a cyano or C₁₋₃-alkyloxy group. In some examples of the compounds of Formula I, R⁴ is selected from fluorine; and a C₁₋₃-alkyloxy group, optionally substituted by one or more groups selected from a halogen; and a cyano or C₁₋₃-alkyloxy group; and R⁵ and R⁶ are both hydrogen. In some examples of the compounds of Formula I, wherein R⁵ and R⁶ are both hydrogen, R⁴ may be fluorine or a 2-methoxyethoxy group, *e.g.* fluorine.

In some examples of the compounds of Formula I, wherein all of R⁴, R⁵ and R⁶ are other than hydrogen (i.e., each of R⁴, R⁵, and R⁶ not hydrogen), these three groups may be attached to the benzene ring, for example, at positions 2, 4 and 6 (relative to the group A being attached to position 1). In some examples of the compounds of Formula I, where only one of R⁴, R⁵ and R⁶ is hydrogen, the other two groups may be attached to the benzene ring, for example, at positions 2 and 3, positions 3 and 4, or positions 3 and 5, *e.g.* at positions 3 and 5 (relative to the group A being attached to position 1). In some examples of the compounds of Formula I, where two of R⁴, R⁵ and R⁶ are hydrogen, the other group may be attached to the benzene ring at position 2, 3 or 4, *e.g.* at position 4 (*i.e.* at the position *para* to the group A). In one embodiment, R⁴ is in a position on the benzene ring *para* to the group A.

In some examples of the compounds of Formula I, A can be a 6-membered aryl group or a 5-membered heteroaryl group. Non-limiting examples of 6-membered aryl groups and 5-membered heteroaryl groups include benzyl, furyl, thienyl, thiazolyl, pyrazolyl, isothiazolyl, oxazolyl, isoxazolyl, pyrrolyl, triazolyl, imidazolyl, oxadiazolyl and thiadiazolyl. In one embodiment, the 6-membered aryl group or 5-membered heteroaryl group is selected from benzyl, thienyl, thiazolyl, pyrrolyl and imidazolyl. In some examples, the 6-membered aryl group or 5-membered heteroaryl group is selected from benzyl and thiazolyl.

In some examples of the compounds of Formula I, A is benzyl, optionally substituted with 1, 2, or 3 groups independently selected from a halogen; and a hydroxy, thio, amino, nitro, oxy or methyl group. In some examples of the compounds of Formula I, A is benzyl, optionally substituted with 1 or 2 halogens. In some examples of the compounds of Formula I, A is benzyl, optionally substituted with a halogen, *e.g.* fluorine. In some examples of the compounds of Formula I, A is an unsubstituted benzyl group.

In some examples of the compounds of Formula I, where A is 6-membered aryl or heteroaryl, the attached groups -C(R²R³)- and -(C₆H₂R⁴R⁵R⁶) may be in a 1,2- or 1,3- or 1,4-relationship, *i.e.* ortho, meta or para to each other. In some examples of the compounds of Formula I, the attached groups -C(R²R³)- and -(C₆H₂R⁴R⁵R⁶) are in a 1,3- relationship. In some examples of the compounds of Formula I, the attached groups are in a 1,4- relationship.

In some examples of the compounds of Formula I, A is a 5-membered heteroaryl group which contains 1, 2, or 3 heteroatoms selected from N, O and S. In some examples of the compounds of Formula I, A is a 5-membered heteroaryl group which contains 1 or 2 heteroatoms selected from N and S. In some examples of the compounds of Formula I, A is a 5-membered heteroaryl group which contains 2 heteroatoms selected from N and S. In some examples of the compounds of Formula I, A is a 5-membered heteroaryl group which contains 2 heteroatoms wherein one heteroatom is N and the other heteroatom is S. In some examples of the compounds of Formula I, A is a thiazolyl group.

In some examples of the compounds of Formula I, where A is a 5-membered heteroaryl group, at least one of the attached groups -C(R²R³)- and -(C₆H₂R⁴R⁵R⁶) may be bonded directly to a carbon atom of the heteroaryl group. In some examples of the compounds of Formula I, both of the attached groups -C(R²R³)- and -(C₆H₂R⁴R⁵R⁶) are bonded directly to a carbon atom of the heteroaryl group. In some examples of the compounds of Formula I, the attached groups -C(R²R³)- and -(C₆H₂R⁴R⁵R⁶) are in a 1,3-relationship to each other, *e.g.* they are bonded directly to carbon atoms of the heteroaryl group which are separated by a single intervening atom, *e.g.* heteroatom. In some examples of the compounds of Formula I, where A is a thiazolyl group, the attached groups -C(R²R³)- and -(C₆H₂R⁴R⁵R⁶) may be bonded directly at the 4- and 2- positions, respectively.

In some examples, the compound of Formula I is a compound of Formula II: 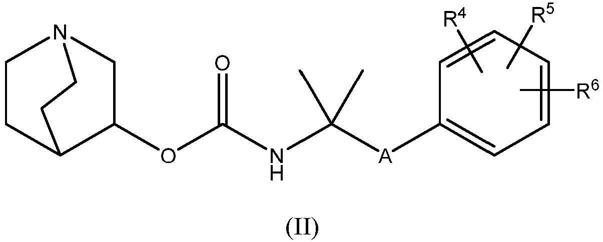 or a pharmaceutically acceptable salt or prodrug thereof, wherein R⁴, R⁵, R⁶ and A are as described herein.

In some examples, the compound of Formula I is a compound of Formula III: 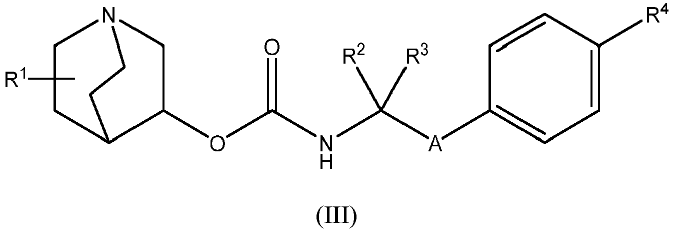 or a pharmaceutically acceptable salt or prodrug thereof, wherein R¹ to R⁴, and A are as described herein.

In some examples, the compound of Formula I is a compound of Formula IV: 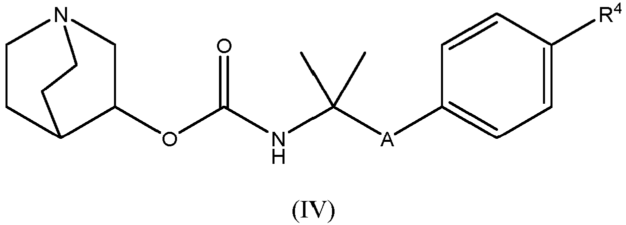 or a pharmaceutically acceptable salt or prodrug thereof, wherein R⁴ and A are as described herein.

In some examples of the compounds of Formula I, R⁴ can be a halogen, e.g. fluorine. Accordingly, a compound of Formula I can be a compound of Formula V: 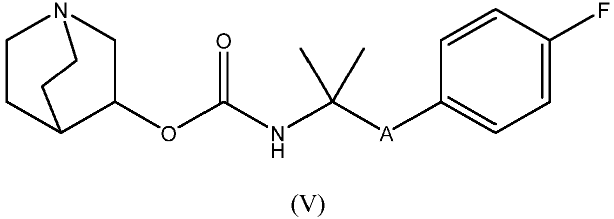 or a pharmaceutically acceptable salt or prodrug thereof, wherein A is as described herein.

In some examples, the compound of Formula I can be a compound of formula VI: 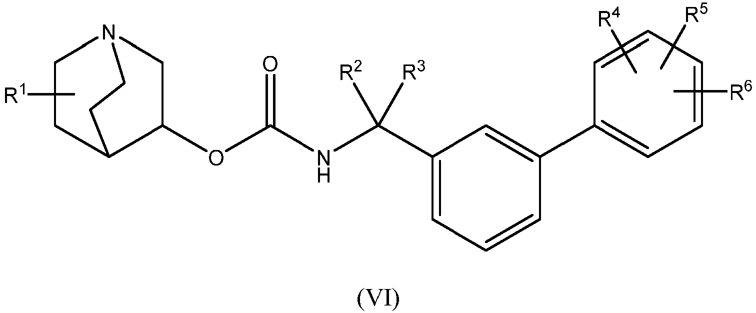 or a pharmaceutically acceptable salt or prodrug thereof, wherein R¹ to R⁶ are as described herein.

In some examples, the compound of Formula I can be a compound of Formula VII: 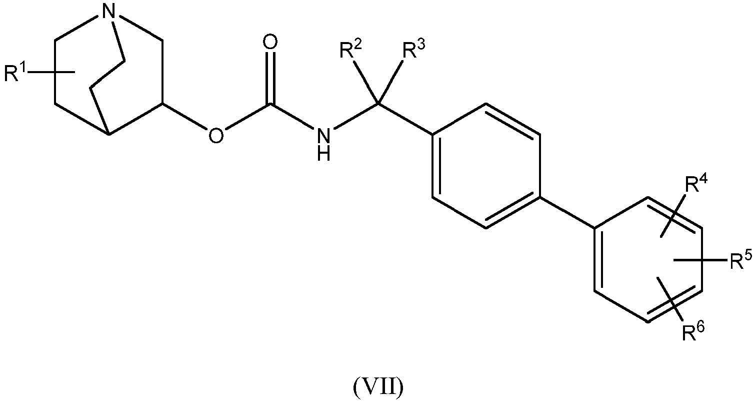 or a pharmaceutically acceptable salt or prodrug thereof, wherein R¹ to R⁶ are as described herein.

In some examples, the compound of Formula I can be a compound of Formula VIII: 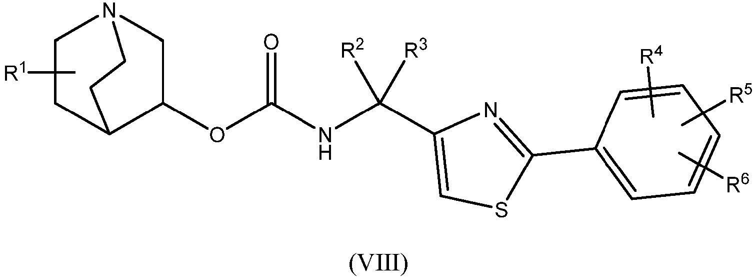 or a pharmaceutically acceptable salt or prodrug thereof, wherein R¹ to R⁶ are as described herein.

In some examples, the compound of Formula I can be a compound of Formula IX: 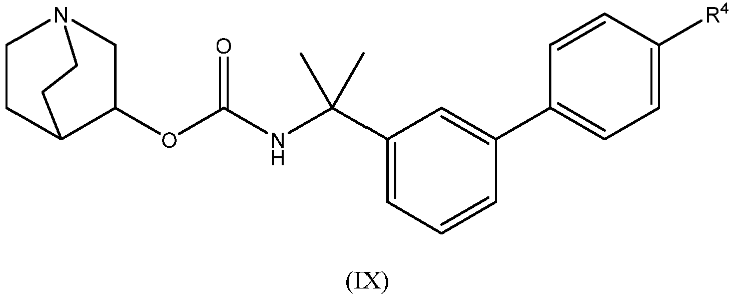 or a pharmaceutically acceptable salt or prodrug thereof, wherein R⁴ is as described herein.

In some examples of the compounds of Formula I, R⁴ can be a halogen, e.g. fluorine. Accordingly, a compound of Formula I can be a compound of Formula X: 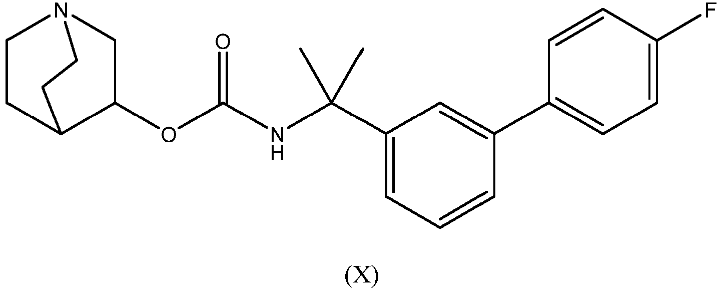 or a pharmaceutically acceptable salt or prodrug thereof.

In some examples, a compound of Formula I can be a compound of Formula XI: 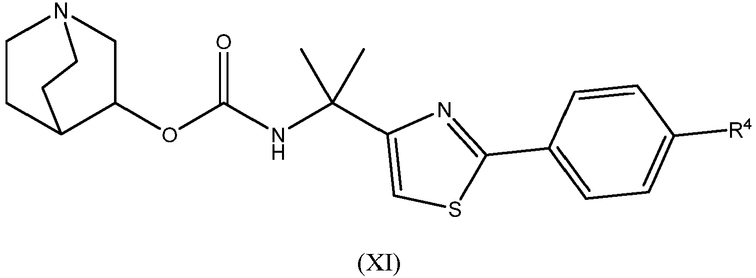 or a pharmaceutically acceptable salt or prodrug thereof, wherein R⁴ is as described herein.

In some examples of the compounds of Formula I, R⁴ can be a halogen, e.g. fluorine. Accordingly, a compound of Formula I can be a compound of Formula XII: 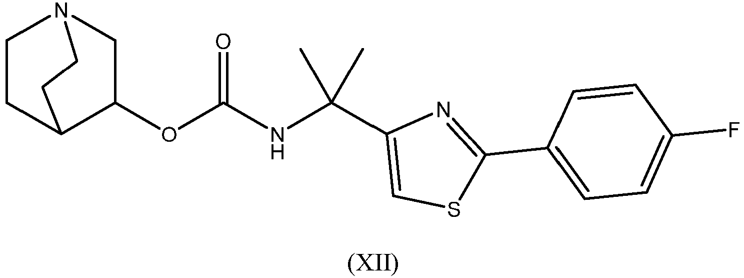 or a pharmaceutically acceptable salt or prodrug thereof.

Non-limiting examples of compounds of Formula I are Compound Nos. 1-23:

| Compound No. | Compound |
|---|---|
| 1 | Quinuclidin-3-yl (2-(4'-fluoro-[1,1'-biphenyl]-3-yl)propan-2-yl)carbamate |
| 2 | (S)-quinuclidin-3-yl (2-(2-(4-fluorophenyl)thiazol-4-yl)propan-2-yl)carbamate |
| 3 | (S)-quinuclidin-3-yl (2-(4'-(2-methoxyethoxy)-[1,1'-biphenyl]-4-yl)propan-2-yl)carbamate |
| 4 | 1-azabicyclo[2.2.2]oct-3-yl [2-(biphenyl-3-yl)propan-2-yl]carbamate |
| 5 | (*S*)-quinuclidin-3-yl 2-(biphenyl-4-yl)propan-2-ylcarbamate |
| 6 | Quinuclidin-3-yl 1-(biphenyl-4-yl)cyclopropylcarbamate |
| 7 | (S)-quinuclidin-3-yl 1-(4'-fluorobiphenyl-4-yl)cyclopropylcarbamate |
| 8 | (*S*)-1-azabicyclo[2.2.2]oct-3-yl [1-(2',4'-difluorobiphenyl-4-yl)cyclopropyl]carbamate |
| 9 | 1-azabicyclo[2.2.2]oct-3-yl [1-(4'-methoxybiphenyl-4-yl)cyclopropyl]carbamate |
| 10 | Quinuclidin-3-yl 2-(5-(4-fluorophenyl)thiophen-3-yl)propan-2-ylcarbamate |
| 11 | (S)-quinuclidin-3-yl 2-(3-(4-fluorophenyl)isothiazol-5-yl)propan-2-ylcarbamate |
| 12 | (S)-quinuclidin-3-yl 2-(4-(4-fluorophenyl)thiazol-2-yl)propan-2-ylcarbamate |
| 13 | Quinuclidin-3-yl (2-(4'-(2-methoxyethoxy)-[1,1'-biphenyl]-4-yl)propan-2-yl)carbamate |
| 14 | (S)-quinuclidin-3-yl (2-(3'-(2-methoxyethoxy)-[1,1'-biphenyl]-4-yl)propan-2-yl)carbamate |
| 15 | Quinuclidin-3-yl (2-(4'-(2-methoxyethoxy)-[1,1'-biphenyl]-3-yl)propan-2-yl)carbamate |
| 16 | Quinuclidin-3-yl (2-(4'-(3-methoxypropoxy)-[1,1'-biphenyl]-4-yl)propan-2-yl)carbamate |
| 17 | Quinuclidin-3-yl (2-(4'-(hydroxymethyl)-[1,1'-biphenyl]-4-yl)propan-2-yl)carbamate |
| 18 | Quinuclidin-3-yl (2-(4'-(2-hydroxyethyl)-[1,1'-biphenyl]-4-yl)propan-2-yl)carbamate |
| 19 | Quinuclidin-3-yl (2-(2-(4-(3-methoxypropoxy)phenyl)thiazol-4-yl)propan-2-yl)carbamate |
| 20 | Quinuclidin-3-yl (2-(2-(4-(2-methoxyethoxy)phenyl)thiazol-4-yl)propan-2-yl)carbamate |
| 21 | Quinuclidin-3-yl 2-(5-(4-(2-methoxyethoxy)phenyl)pyridin-2-yl)propan-2-ylcarbamate |
| 22 | Quinuclidin-3-yl (2-(4'-(3-cyanopropoxy)-[1,1'-biphenyl]-4-yl)propan-2-yl)carbamate |
| 23 | Quinuclidin-3-yl (2-(4'-(cyanomethoxy)-[1,1'-biphenyl]-4-yl)propan-2-yl)carbamate |

and the pharmaceutically acceptable salts and prodrugs thereof. Additional examples of glucosyl ceramide synthase inhibitors are known in the art. See, e.g., PCT/US2010/023080; PCT/US2013/058896; PCT/US2012/029417; PCT/US2014/069338; PCT/US2010/023168; PCT/US2014/056555; U.S. Patent Appln. Serial No. 13/652,016; U.S. Patent Appln. Serial No. 11/077,556; U.S. Patent Appln. Serial No. 10/839,497; U.S. Patent No. 9,126,993; U.S. Patent No. 8,309,593; and U.S. Patent No. 8,304,447.

Additional examples of a treatment for a proteopathy is the administration of one or more recombinant enzymes. Non-limiting examples of recombinant enzymes that can be used to treat a proteopathy include imiglucerase, velaglucerase, and taliglucerase. Additional non-limiting examples of recombinant proteins and enzymes that can be used to treat a proteopathy include α-galactosidase A (α-gal A), human cytochrome P450 enzymes, IL-33, and neprilysin (see, e.g., Blom, D., et al., Am. J. Hum. Genet. 72(1):23-31, 2003; Blurton-Jones, M., et al., Stem Cell Research & Therapy 5:46, 2014; Coune, P.G., et al., Cold Spring Harb Perspect. Med. 2(4): a009431, 2012; U.S. Patent Appln Serial No. 10/096,723; Fu, A.K.Y, et al., PNAS April 2016; Hidestrand M, et al., Drug Metab Dispos. 29(11):1480-4, 2001; Spencer B., et al., J. Biol. Chem. 289(25):17917-17931, 2014). Additional treatments for proteopathies are known in the art and include, for example, RILUTEK (riluzole), Donepezil (Aricept), Galantamine (Razadyne), Memantine (Namenda), Rivastigmine (Exelon), Baclofen, nutritional supplements including branched-chain amino acids (BCAAs), phenytoin, tricyclic antidepressants, antidepressants, cell-derived neurotrophic factor, Amantadine, Benztropine, Carbidopa/Levodopa, Selegiline (Eldepryl), Rotigotine, Entacapone (Comtan), Ropinirole (Requip), Rasagiline (Azilect), Bromocriptine (Parlodel ), Cabergoline, Tolcapone (Tasmar), and Pramipexole (Mirapex).

### Sphingolipids

Some of the methods described herein include the determination of the level(s) of at least one (e.g., 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or 51) sphingolipid in a sample including a biological fluid obtained from a subject (e.g., any of the subjects described herein, e.g., a subject having a proteopathy) (e.g., a sample including a biological fluid obtained from a subject that has been enriched for lipids) selected from the group of: dihexosylceramide C24:1; dihexosylceramide C24:0; dihexosylceramide C23:0; dihexosylceramide C22:0; dihexosylceramide C20:0; dihexosylceramide C18:0; dihexosylceramide C16:0; lactosylceramide C24: 1; lactosylceramide C24:0; lactosylceramide C23:0; lactosylceramide C22:0; lactosylceramide C20:0; lactosylceramide C18:0; lactosylceramide C16:0; ceramide C24:1; ceramide C24:0; ceramide C23:0; ceramide C22:0; ceramide C20:0; ceramide C18:0; ceramide C16:0; ceramide C14:0; globotriaosylceramide C24:1; globotriaosylceramide C24:0; globotriaosylceramide C23:0; globotriaosylceramide C22:0; globotriaosylceramide C20:0; globotriaosylceramide C18:0; globotriaosylceramide C16:0; sphingomyelin C24: 1; sphingomyelin C24:0; sphingomyelin C23:0; sphingomyelin C22:0; sphingomyelin C20:0; sphingomyelin C18:0; sphingomyelin C16:0; galactosylceramide C24:1; galactosylceramide C24:0; galactosylceramide C23:0; galactosylceramide C22:0; galactosylceramide C20:0; galactosylceramide C18:0; galactosylceramide C16:0; glucosylceramide C24: 1; glucosylceramide C24:0; glucosylceramide C23:0; glucosylceramide C22:0; glucosylceramide C20:0; glucosylceramide C18:0; glucosylceramide C16:0; and glucosylsphingosine. In the methods of the invention, the level of at least one sphingolipid is determined, wherein the at least one sphingolipid is selected from the group consisting of: sphingomyelin C24:1; sphingomyelin C24:0; and sphingomyelin C23:0.

Some of the methods described herein include the determination of at least one (e.g., 2, 3, 4, 5, 6, 7, or 8) of total dihexosylceramide level, total lactosylceramide level, total ceramide level, total globotriaosylceramide level, total sphingomyelin level, total galactosylceramide level, total glucosylceramide level, and total phosphatidylcholine level in a sample including a biological fluid obtained from a subject (e.g., any of the subjects described herein, e.g., a subject having a proteopathy) (e.g., a sample including a biological fluid obtained from a subject that has been enriched for lipids).

Methods for determining the levels of the sphingolipids described herein are well understood in the art. Methods of detecting a level(s) of any of the sphingolipids described herein can be performed using a variety of standard and advanced methods of analyte detection. For example, a level(s) of any of the sphingolipids described herein can be detected and quantified using techniques such as chromatography, chromatography coupled with mass spectroscopy, fluorometric assays, and immunochemical methodologies, e.g., thin layer chromatography, liquid chromatography coupled with mass spectroscopy (LC MS), LC-MS coupled with MS (LC-MS/MS), hydrophilic interaction chromatography (HILIC) coupled with MS, high performance liquid chromatography (HPLC) coupled with MS, HPLC coupled with fluorometric assays, anti-ceramide antibodies, etc. (see, e.g., the methods described in Butter et al., J. Chromatogr. B Analyt. Technol. Biomed. Life Sci. 824(1-2):65-70, 2005; Cell Biolabs, INC. "Product Manual: Sphingomyelin Assay Kit" Catalog Number STA-601; Farwanah et al., J. Chromatogr. B Analyt. Technol. Biomed. Life Sci. 877(27):2976-82, 2009; Haynes et al., J. Chromatogr. B Analyt. Technol. Biomed. Life Sci. 877(26):2696-2708, 2009; Mano et al., Anal. Biochem. 244(2):291-300, 1997; Markham JE., Methods Mol Biol. 1009:92-101, 2013; and Scherer et al., J. Lipid Res. 51(7): 2001-2011, 2010).

In some examples, a sample (e.g., a sample including a biological fluid) from a subject can be stored for a period of time (e.g., stored at least 1 hour (e.g., at least 2, 4, 6, 8, 12, or 24 hours, or at least 1, 2, 3, 4, 5, 6, 7, 14, or 21 days, e.g., at a temperature of about 10 °C, about 0 °C, about -20 °C, about -40°C, about -70 °C, or about -80 °C) before the level(s) of the at least one sphingolipid are determined in the sample. For example, a sample comprising a biological fluid and enriched for lipids can be stored for a period of time (e.g., stored for any of the times and/or temperatures described herein) before the level(s) of the at least one sphingolipid are determined in the sample. Some examples further include a step of enriching a sample containing a biological fluid for lipids before the level(s) of the at least one sphingolipid (e.g., any sphingolipid or any combination of sphingolipids described herein) is determined. Non-limiting examples of methods of enriching a sample containing a biological fluid for lipids are described herein. In some examples, a biological sample containing a biological fluid that is enriched for lipids is stored for a period of time (e.g., stored for any of the times and/or temperatures described herein) before the level(s) of the at least one sphingolipid is determined in the sample.

The level(s) of any sphingolipid, any parameter, or any combination of sphingolipids or parameters described herein can be determined in a sample (e.g., a sample containing a biological fluid or a sample containing a biological fluid that has been enriched for lipids) in any of the methods described herein.

The level(s) of any sphingolipid, any parameter, or any combination of sphingolipids or parameters described herein can be compared to a control level of any sphingolipid, any parameter, or any combination of sphingolipids or parameters described herein. The control level(s) can be, e.g., a level(s) in a sample from a subject not presenting with one or more symptoms of a proteopathy and/or not diagnosed as having a proteopathy, a level(s) in a sample from a healthy subject or a population of healthy subjects, or a threshold level(s) (e.g., a level(s) above which indicates that the subject has a proteopathy or an increased risk of developing a proteopathy). The control level(s) can be, e.g., a level in a sample from a subject or a population of subjects that has/have no history of a proteopathy and, optionally, also does/do not have a genetically-related family member diagnosed or identified as having a proteopathy. The control level(s) can be, e.g., a threshold level(s) that is indicative of a subject having a proteopathy or a subject having an increased risk of developing a proteopathy when the measured level is above the threshold level. Additional exemplary control level(s) of any of the sphingolipid(s) are described herein.

### Enriching a Sample for Lipids

Some embodiments of the methods described herein include a step of enriching a sample containing a biological fluid (e.g., any of the biological fluids described herein) for lipids, e.g., before a level(s) of at least one of any of the sphingolipids described herein is determined in the sample. Methods for enriching a sample including a biological fluid obtained from a subject for lipids can include, e.g., one or both of the following: centrifuging the sample to remove particulate matter and extracting lipids using one or more organic solvents (e.g., any of the exemplary solvents described in the Examples section or known in the art). Non-limiting examples of one or more organic solvents that can be used to extract lipids and enrich a sample for lipids include: acetonitrile, methanol, and acetic acid. In some examples, the enriching of a sample including a biological fluid obtained from a subject can include the use of an extraction solvent including one or more organic solvents (e.g., any organic solvent or any combination of organic solvents described herein) and water. In some examples, the enriching of a sample including a biological fluid obtained from a subject can include the use of an extraction solvent including one or more organic solvents (e.g., any organic solvent or any combination of organic solvents described herein), water, and ammonium acetate. Non-limiting specific examples of extraction solvents that can be used to enrich a sample including a biological fluid obtained from a subject are described in the Examples section. As is known in the art, enriching a sample including a biological fluid obtained from a subject for lipids can be performed at temperature of about 10 °C to about 25 °C, about 22 °C, about 18 °C, about 16 °C, about 14 °C, or about 12 °C; about 12 °C to about 25 °C, about 22 °C, about 20 °C, about 18 °C, about 16 °C, or about 14 °C; about 14 °C to about 25 °, about 22 °C, about 20 °C, about 18 °C, or about 16 °C; about 16 °C to about 25 °, about 22 °C, about 20 °C, or about 18 °C; about 18 ° C to about 25 °, about 22 °C, or about 20 °C; or about 20 °C to about 22 °C or about 25 °C.

Exemplary methods for enriching a sample including a biological fluid obtained from a subject for lipids are also described in the Example. Additional methods for enriching a sample including a biological fluid obtained from a subject for lipids are known in the art.

### Methods of Determining the Efficacy of a Treatment of a Proteopathy

Described herein are methods of determining the efficacy of a treatment for a proteopathy in a subject having a proteopathy. In some examples, these methods include: (a) providing a first sample including a biological fluid (e.g., serum, plasma, blood, or cerebrospinal fluid) obtained from a subject having a proteopathy at a first time point; (b) determining the level(s) of at least one (e.g., 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, or 44) in any combination) sphingolipid in the sample of (a), where the at least one sphingolipid is selected from the group of: dihexosylceramide C24:1; dihexosylceramide C24:0; dihexosylceramide C23:0; dihexosylceramide C22:0; dihexosylceramide C20:0; dihexosylceramide C18:0; dihexosylceramide C16:0; lactosylceramide C24:1; lactosylceramide C24:0; lactosylceramide C23:0; lactosylceramide C22:0; lactosylceramide C20:0; lactosylceramide C18:0; lactosylceramide C16:0; ceramide C24:1; ceramide C24:0; ceramide C23:0; ceramide C22:0; ceramide C20:0; ceramide C18:0; ceramide C16:0; ceramide C14:0; globotriaosylceramide C24:1; globotriaosylceramide C24:0; globotriaosylceramide C23:0; globotriaosylceramide C22:0; globotriaosylceramide C20:0; globotriaosylceramide C18:0; globotriaosylceramide C16:0; galactosylceramide C24:1; galactosylceramide C24:0; galactosylceramide C23:0; galactosylceramide C22:0; galactosylceramide C20:0; galactosylceramide C18:0; galactosylceramide C16:0; glucosylceramide C24:1; glucosylceramide C24:0; glucosylceramide C23:0; glucosylceramide C22:0; glucosylceramide C20:0; glucosylceramide C18:0; glucosylceramide C16:0; and glucosylsphingosine; (c) administering a treatment for a proteopathy to the subject; (d) providing a second sample including a biological fluid obtained from the subject at a second time point after step (c), and perfoming step (b) on the second sample; and (e) identifying the administered treatment as being effective when the level(s) of the at least one sphingolipid is decreased at the second time point as compared to the first time point. In some examples, the method further includes: further identifying the administered treatment as being effective when the level(s) of the at least one sphingolipid is also decreased as compared to level(s) of the at least one sphingolipid present in a healthy subject or a population of healthy subjects. In some examples, the method further includes: further identifying the administered treatment as being effective when the level(s) of the at least one sphingolipid is also decreased as compared to level(s) of the at least one sphingolipid present in a healthy subject or a population of healthy subjects, level(s) of the at least one sphingolipid in a sample from a subject or population of subjects not presenting one or more symptoms of a proteopathy and/or not diagnosed as having a proteopathy, a threshold level(s) of the at least one sphingolipid (e.g., level(s) above which indicates that the subject has a proteopathy), level(s) of the at least one sphingolipid in a subject or a population of subjects that has/have no history of a proteopathy, and, optionally, also does/do not have a genetically-related family member diagnosed or identified as having a proteopathy, a threshold level(s) that is indicative of a subject having a proteopathy when the measured level(s) is above the threshold level(s), or a threshold level(s) that is indicative of a subject having a proteopathy that has been effectively treated when the measured level(s) is below the threshold level(s). Some embodiments of any of the methods described herein further include a step of enriching the sample of (a) for lipids prior to the step of determining the level of the at least one sphingolipid.

In some embodiments, the steps (b) and (d) include determining the level(s) of at least one (e.g., 2, 3, 4, 5, 6, 7, 8, 9, 10, 11, 12, 13, 14, 15, 16, or 17) sphingolipid selected from the group of: dihexosylceramide C24: 1, dihexosylceramide C24:0, dihexosylceramide C20:0, ceramide C24:0, ceramide C23:0, globotriaosylceramide C24: 1, globotriaosylceramide C16:0, \ galactosylceramide C24:0, galactosylceramide C23:0, galactosylceramide C16:0, glucosylceramide C24: 1, glucosylceramide C24:0, glucosylceramide C23:0, glucosylceramide C22:0, glucosylceramide C20:0, glucosylceramide C18:0, and glucosylceramide C16:0.

In some embodiments, steps (b) and (d) include determining the levels of at least two (e.g., 3, 4, 5, 6, 7, 8, 9, 10, 11, 12, 13, 14, 15, 16, or 17) sphingolipids selected from the group of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24: 1, glucosylceramide C24:0, glucosylceramide C23:0, glucosylceramide C22:0, glucosylceramide C20:0, glucosylceramide C18:0, and glucosylceramide C16:0, and the administered treatment is identified as being effective when at least one or both of the two levels is decreased at the second time point as compared to the first time point.

In some embodiments, the steps (b) and (d) include determining the levels of at least three (e.g., 4, 5, 6, 7, 8, 9, 10, 11, 12, 13, 14, 15, 16, or 17) sphingolipids selected from the group of: dihexosylceramide C24: 1, dihexosylceramide C24:0, dihexosylceramide C20:0, ceramide C24:0, ceramide C23:0, globotriaosylceramide C24: 1, globotriaosylceramide C16:0, galactosylceramide C24:0, galactosylceramide C23:0, galactosylceramide C16:0, glucosylceramide C24: 1, glucosylceramide C24:0, glucosylceramide C23:0, glucosylceramide C22:0, glucosylceramide C20:0, glucosylceramide C18:0, and glucosylceramide C16:0, and the administered treatment is identified as being effective when at least one, two, or all three of the three levels is decreased at the second time point as compared to the first time point.

In some embodiments, the steps (b) and (d) include determining the levels of at least four (e.g., 5, 6, 7, 8, 9, 10, 11, 12, 13, 14, 15, 16, or 17) sphingolipids selected from the group of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24: 1, glucosylceramide C24:0, glucosylceramide C23:0, glucosylceramide C22:0, glucosylceramide C20:0, glucosylceramide C18:0, and glucosylceramide C16:0, and the administered treatment is identified as being effective when at least one, two, three, or all four of the four levels is decreased at the second time point as compared to the first time point.

Also described are methods of determining the efficacy of a treatment for a proteopathy in a subject having a proteopathy that include: (a) providing a first sample including a biological fluid (e.g., serum, plasma, blood, or cerebrospinal fluid) obtained from a subject having a proteopathy at a first time point; (b) determining the level(s) of at least one (e.g., 2, 3, 4, 5, 6, or 7) in any combination) sphingolipid in the sample of (a), where the at least one sphingolipid is selected from the group of: sphingomyelin C24:1; sphingomyelin C24:0; sphingomyelin C23:0; sphingomyelin C22:0; sphingomyelin C20:0; sphingomyelin C18:0; and sphingomyelin C16:0; (c) administering a treatment for a proteopathy to the subject; (d) providing a second sample including a biological fluid obtained from the subject at a second time point after step (c), and perfoming step (b) on the second sample; and (e) identifying the administered treatment as being effective when the level(s) of the at least one (e.g., 2, 3, 4, 5, 6, or 7) sphingolipid is increased at the second time point as compared to the first time point. In the methods of the invention, the efficacy of a glucosyl ceramide synthase inhibitor treatment for for Parkinson's disease in a subject having Parkinson's disease is determined, wherein the first sample comprises plasma, the second sample comprises plasma, and the at least one sphingolipid is selected from the group consisting of: sphingomyelin C24: 1; sphingomyelin C24:0; and sphingomyelin C23:0, and wherein the subject has been administered said glucosyl ceramide synthase inhibitor treatment between the first time point and the second time point.

In some examples, the method further includes: further identifying the administered treatment as being effective when the level(s) of the at least one sphingolipid is also increased as compared to level(s) of the at least one sphingolipid present in a healthy subject or a population of healthy subjects. In some examples, the method further includes: further identifying the administered treatment as being effective when the level(s) of the at least one sphingolipid is also increased as compared to level(s) of the at least one sphingolipid present in a healthy subject or a population of healthy subjects, level(s) of the at least one sphingolipid in a sample from a subject or population of subjects not presenting one or more symptoms of a proteopathy and/or not diagnosed as having a proteopathy, a threshold level(s) of the at least one sphingolipid (e.g., level(s) below which indicates that the subject has a proteopathy), level(s) of the at least one sphingolipid in a subject or a population of subjects that has/have no history of a proteopathy, and, optionally, also does/do not have a genetically-related family member diagnosed or identified as having a proteopathy, a threshold level(s) that is indicative of a subject having a proteopathy when the measured level(s) is below the threshold level(s), or a threshold level(s) that is indicative of a subject having a proteopathy that has been effectively treated when the measured level(s) is above the threshold level(s). Some embodiments of any of the methods described herein further include a step of enriching the sample of (a) for lipids prior to the step of determining the level of the at least one sphingolipid.

Also described are methods of determining the efficacy of a treatment for a proteopathy in a subject having a proteopathy that include: (a) providing a first sample including a biological fluid obtained from a subject having a proteopathy at a first time point; (b) determining at least one (e.g., 2, 3, 4, 5, or 6) of total dihexosylceramide level, total lactosylceramide level, total globotriaosylceramide level, total galactosylceramide level, total glucosylceramide level, and total phosphatidylcholine level in the sample of step (a); (c) administering a treatment for a proteopathy to the subject; (d) providing a second sample including a biological fluid obtained from the subject at a second time point after step (c), and performing step (b) on the second sample; and (e) identifying the administered treatment as being effective when at least one (e.g., 2, 3, 4, 5, or 6) of the total dihexosylceramide level, the total lactosylceramide level, the total globotriaosylceramide level, the total galactosylceramide level, the total glucosylceramide level, and the total phosphatidylcholine level is decreased at the second time point as compared to the first time point. In some examples, the method further includes identifying the administered treatment as being effective when at least one (e.g., 2, 3, 4, 5, or 6) of the total dihexosylceramide level, the total lactosylceramide level, the total globotriaosylceramide level, the total galactosylceramide level, the total glucosylceramide level, and the total phosphatidylcholine level is also decreased as compared to level(s) in a healthy subject or a population of healthy subjects. In some examples, the method further includes: further identifying the administered treatment as being effective when the level(s) of the at least one of the total dihexosylceramide level, the total lactosylceramide level, the total globotriaosylceramide level, the total galactosylceramide level, the total glucosylceramide level, and the total phosphatidylcholine level is also decreased as compared to level(s) of the at least one of the total dihexosylceramide level, the total lactosylceramide level, the total globotriaosylceramide level, the total galactosylceramide level, the total glucosylceramide level, and the total phosphatidylcholine level present in a healthy subject or a population of healthy subjects, level(s) of the at least one of the total dihexosylceramide level, the total lactosylceramide level, the total globotriaosylceramide level, the total galactosylceramide level, the total glucosylceramide level, and the total phosphatidylcholine level in a sample from a subject or population of subjects not presenting one or more symptoms of a proteopathy and/or not diagnosed as having a proteopathy, a threshold level(s) of the at least one of the total dihexosylceramide level, the total lactosylceramide level, the total globotriaosylceramide level, the total galactosylceramide level, the total glucosylceramide level, and the total phosphatidylcholine level (e.g., level(s) above which indicates that the subject has a proteopathy), level(s) of the at least one of the total dihexosylceramide level, the total lactosylceramide level, the total globotriaosylceramide level, the total galactosylceramide level, the total glucosylceramide level, and the total phosphatidylcholine level in a subject or a population of subjects that has/have no history of a proteopathy, and, optionally, also does/do not have a genetically-related family member diagnosed or identified as having a proteopathy, a threshold level(s) of the at least one of the total dihexosylceramide level, the total lactosylceramide level, the total globotriaosylceramide level, the total galactosylceramide level, the total glucosylceramide level, and the total phosphatidylcholine level that is indicative of a subject having a proteopathy when the measured level(s) is above the threshold level(s), or a threshold level(s) of the at least one of the total dihexosylceramide level, the total lactosylceramide level, the total globotriaosylceramide level, the total galactosylceramide level, the total glucosylceramide level, and the total phosphatidylcholine level that is indicative of a subject having a proteopathy that has been effectively treated when the measured level(s) is below the threshold level(s). Some embodiments of these methods further include a step of enriching the first or second sample for lipids prior to the step of determining the level(s) of at least one of the total dihexosylceramide level, the total lactosylceramide level, the total globotriaosylceramide level, the total galactosylceramide level, the total glucosylceramide level, and the total phosphatidylcholine level.

**In** some embodiments of these methods, steps (b) and (d) include determining one or both of the total galactosylceramide level and the total glucosylceramide level. **In** some embodiments, steps (b) and (d) include determining both of the total galactosylceramide level and the total glucosylceramide level, and the administered treatment is identified as being effective when one or both of the levels is decreased at the second time point as compared to the first time point.

Also described are methods of determining the efficacy of a treatment for a proteopathy in a subject having a proteopathy that include: (a) providing a first sample including a biological fluid obtained from a subject having a proteopathy at a first time point; (b) determining one or both of total ceramide level and total sphingomyelin level in the sample of step (a); (c) administering a treatment for a proteopathy to the subject; (d) providing a second sample including a biological fluid obtained from the subject at a second time point after step (c), and performing step (b) on the second sample; and (e) identifying the administered treatment as being effective when one or both of the total ceramide level and the total sphingomyelin level is increased at the second time point as compared to the first time point.

Also described are methods of determining the efficacy of a treatment for a proteopathy in a subject having a proteopathy that include: (a) providing a first sample including a biological fluid obtained from a subject having a proteopathy at a first time point; (b) determining the ratio of glucosylceramide C24:0 to sphingomyelin C24:0 in the sample of step (a); (c) administering a treatment for a proteopathy to the subject; (d) providing a second sample including a biological fluid obtained from the subject at a second time point after step (c), and performing step (b) on the second sample; and (e) identifying the administered treatment as being effective when the ratio of glucosylceramide C24:0 to sphingomyelin C24:0 is decreased at the second time point as compared to the first time point.

**In** some embodiments of any of these methods, the subject has previously been diagnosed as having a proteopathy. **In** some embodiments of any of these methods, the first sample and the second sample include blood, plasma, serum, or cerebrospinal fluid. **In** the methods of the invention, the first sample comprises plasma and the second sample comprises plasma.

The administered treatment can be, e.g., administration of a glucosyl ceramide synthase inhibitor (e.g., any of the glycosyl ceramide synthase inhibitors described herein) or a recombinant enzyme (e.g., any of the recombinant enzymes described herein). In the methods of the invention, the administered treatment is administration of a glucosyl ceramide synthase inhibitor. Non-limiting examples of glucosyl ceramide synthase inhibitors include: (i) eliglustat; (ii) miglustat; (iii) quinuclidin-3-yl (2-(4'-fluoro-[1,1'-biphenyl]-3-yl)propan-2-yl)carbamate; (iv) (S)-quinuclidin-3-yl (2-(2-(4-fluorophenyl)thiazol-4-yl)propan-2-yl)carbamate; (v) (S)-quinuclidin-3-yl (2-(4'-(2-methoxyethoxy)-[1,1'-biphenyl]-4-yl)propan-2-yl)carbamate; and pharmaceutically acceptable salt and prodrugs thereof. A non-limiting example of a recombinant enzyme is a recombinant glucocerebrosidase (e.g., imiglucerase, velaglucerase, or taliglucerase).

Some embodiments of any of these methods further include after (e): (f) administering an additional dose or additional doses of the administered treatment identified as being effective to the subject. In some embodiments, the administered treatment identified as being effective is a glucosyl ceramide synthase inhibitor or a recombinant enzyme, and in step (f) the subject is administered additional doses of the glucosyl ceramide synthase inhibitor (e.g., any of the glucosyl synthase inhibitors described herein) or the recombinant enzyme (e.g., any of recombinant enzymes described herein). For example, in step (f) the subject can be administered additional doses of the glucosyl ceramide synthase inhibitor selected from the group of: (i) eliglustat; (ii) miglustat; (iii) quinuclidin-3-yl (2-(4'-fluoro-[1,1'-biphenyl]-3-yl)propan-2-yl)carbamate; (iv) (S)-quinuclidin-3-yl (2-(2-(4-fluorophenyl)thiazol-4-yl)propan-2-yl)carbamate; (v) (S)-quinuclidin-3-yl (2-(4'-(2-methoxyethoxy)-[1,1'-biphenyl]-4-yl)propan-2-yl)carbamate; and the pharmaceutically acceptable salts and prodrugs thereof. In step (f) the subject may be administered additional doses of the recombinant enzyme (e.g., a recombinant glucocerebrosidase, e.g., imiglucerase, velaglucerase, or taliglucerase).

Some embodiments of any of the methods further include a step of selecting a subject having a proteopathy or diagnosing a subject having a proteopathy (e.g., using any of the exemplary methods of diagnosing a proteopathy described herein). The subject in any of these methods can be any of the subjects described herein. For example, a subject having a proteopathy can have previously been administered a treatment for a proteopathy and the treatment was unsuccessful. Some embodiments of any of the methods further include obtaining the first and/or second samples from the subject (e.g., a subject having a proteopathy).

Some embodiments further include recording the identified efficacy of the administered treatment in the subject's medical record (e.g., a computer readable medium). Some examples further include informing the subject, the subject's family, and/or the subject's primary care physician or attending physician of the identified efficacy of the administered treatment. Some embodiments further include authorization of a refill of an administered treatment identified as being effective.

The difference in time between the first and second time points can be, e.g., between 1 week and 40 weeks, between 1 week and 30 weeks, between 1 week and 20 weeks, between 1 week and 12 weeks, between 1 week and 8 weeks, between 1 week and 4 weeks, between 1 week and 2 weeks, between 2 weeks and 12 weeks, between 2 weeks and 8 weeks, or between 2 weeks and 4 weeks.

### Methods of Diagnosing a Proteopathy

Also described are methods of diagnosing a proteopathy in a subject that include: (a) providing a sample including a biological fluid obtained from a subject; (b) determining the level of at least one (e.g., 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, or 44) sphingolipid in the sample of (a), wherein the at least one sphingolipid is selected from the group of: dihexosylceramide C24:1; dihexosylceramide C24:0; dihexosylceramide C23:0; dihexosylceramide C22:0; dihexosylceramide C20:0; dihexosylceramide C18:0; dihexosylceramide C16:0; lactosylceramide C24: 1; lactosylceramide C24:0; lactosylceramide C23:0; lactosylceramide C22:0; lactosylceramide C20:0; lactosylceramide C18:0; lactosylceramide C16:0; ceramide C24:1; ceramide C24:0; ceramide C23:0; ceramide C22:0; ceramide C20:0; ceramide C18:0; ceramide C16:0; ceramide C14:0; globotriaosylceramide C24:1; globotriaosylceramide C24:0; globotriaosylceramide C23:0; globotriaosylceramide C22:0; globotriaosylceramide C20:0; globotriaosylceramide C18:0; globotriaosylceramide C16:0; galactosylceramide C24:1; galactosylceramide C24:0; galactosylceramide C23:0; galactosylceramide C22:0; galactosylceramide C20:0; galactosylceramide C18:0; galactosylceramide C16:0; glucosylceramide C24:1; glucosylceramide C24:0; glucosylceramide C23:0; glucosylceramide C22:0; glucosylceramide C20:0; glucosylceramide C18:0; glucosylceramide C16:0; and glucosylsphingosine; and (c) identifying the subject as having a proteopathy when the level(s) of the at least one (e.g., 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, or 44) sphingolipid is elevated as compared to a control level(s). Some embodiments of these methods further include a step of enriching the sample of (a) for lipids prior to the step of determining the level(s) of the at least one sphingolipid.

In some embodiments of these methods, step (b) includes determining the level(s) of at least one (e.g., 2, 3, 4, 5, 6, 7, 8, 9, 10, 11, 12, 13, 14, 15, 16, or 17) sphingolipid selected from the group of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0. In some embodiments of these methods, step (b) includes determining the levels of at least two (e.g., 3, 4, 5, 6, 7, 8, 9, 10, 11, 12, 13, 14, 15, 16, or 17) sphingolipids selected from the group of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0, and the subject is identified as having a proteopathy when at least one or both of the two levels is elevated as compared to control level(s).

In some embodiments of these methods, step (b) includes detecting the levels of at least three (e.g., 4, 5, 6, 7, 8, 9, 10, 11, 12, 13, 14, 15, 16, or 17) sphingolipids selected from the group of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0, and the subject is identified as having a proteopathy when at least one, at least two, or all three of the three levels is elevated as compared to control level(s). In some embodiments of these methods, step (b) includes detecting the levels of at least four (e.g., 5, 6, 7, 8, 9, 10, 11, 12, 13, 14, 15, 16, or 17) sphingolipids selected from the group of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0, and the subject is identified as having a proteopathy when at least one, at least two, at least three, or all four of the four levels is elevated as compared to control level(s).

Also described are methods of diagnosing a proteopathy in a subject that include: (a) providing a sample including a biological fluid obtained from a subject; (b) determining the level of at least one (e.g., 2, 3, 4, 5, 6, or 7) sphingolipid in the sample of (a), wherein the at least one sphingolipid is selected from the group of: sphingomyelin C24:1; sphingomyelin C24:0; sphingomyelin C23:0; sphingomyelin C22:0; sphingomyelin C20:0; sphingomyelin C18:0; and sphingomyelin C16:0; and (c) identifying the subject as having a proteopathy when the level(s) of the at least one (e.g., 2, 3, 4, 5, 6, or 7) sphingolipid is decreased as compared to a control level(s). Some embodiments of these methods further include a step of enriching the sample of (a) for lipids prior to the step of determining the level(s) of the at least one sphingolipid.

Also described are methods of diagnosing a subject as having a proteopathy that include: (a) providing a sample including a biological fluid obtained from a subject; (b) determining at least one (e.g., 2, 3, 4, 5, or 6) of total dihexosylceramide level, total lactosylceramide level, total globotriaosylceramide level, total galactosylceramide level, total glucosylceramide level, and total phosphatidylcholine level in the sample of step (a); and (c) identifying the subject as having a proteopathy when at least one (e.g., 2, 3, 4, 5, or 6) of the total dihexosylceramide level, the total lactosylceramide level, the total globotriaosylceramide level, the total galactosylceramide level, the total glucosylceramide level, and the total phosphatidylcholine level is increased as compared to a control level(s). Some embodiments of these methods further include enriching the sample of (a) for lipids prior to determining the level(s) of at least one of total dihexosylceramide level, total lactosylceramide level, total globotriaosylceramide level, total galactosylceramide level, total glucosylceramide level, and total phosphatidylcholine level in the sample of (a).

In some embodiments of these methods, step (b) includes determining one or both of the total galactosylceramide level and the total glucosylceramide level. In some embodiments of these methods, step (b) includes determining both the total galactosylceramide level and the total glucosylceramide level, and the subject is identified as having a proteopathy when at least one or both of the levels is increased as compared to control level(s).

Also described are methods of diagnosing a subject as having a proteopathy that include: (a) providing a sample including a biological fluid obtained from a subject; (b) determining one or both of total ceramide level and total sphingomyelin level in the sample of step (a); and (c) identifying the subject as having a proteopathy when one or both of the total ceramide level and the total sphingomyelin level is decreased as compared to a control level(s). Some embodiments of these methods further include enriching the sample of (a) for lipids prior to determining the level(s) of one or both of total ceramide level and total sphingomyelin level in the sample of (a).

Also described are methods of diagnosing a subject as having a proteopathy that include: (a) providing a sample including a biological fluid obtained from a subject; (b) determining the ratio of glucosylceramide C24:0 to sphingomyelin C24:0 in the sample of step (a); and (c) identifying the subject as having a proteopathy when the ratio of glucosylceramide C24:0 to sphingomyelin C24:0 is increased as compared to a control ratio. Some embodiments of these methods further include enriching the sample of (a) for lipids prior to determining the ratio of glucosylceramide C24:0 to sphingomyelin C24:0 in the sample of (a).

In some embodiments of any of the methods described herein, the sample includes blood, serum, plasma, or cerebrospinal fluid. Some embodiments of any of the methods described herein further include detecting a mutation in a glucocerebrosidase (GBA) gene in a sample including genomic DNA from the subject, and further identifying a subject having a mutation in a GBA gene as having a proteopathy. Non-limiting mutations in a GBA gene can result in the expression of a GBA having, e.g., one or more of the following mutations: V15L, C16S, Δ36T, F37V, E41K, G46E, R48W, L66P, K79N, A90T, S107L, N117D, I119T, R120W, R120Q, P122S, M123V, D127V, R131C, R131L, T134I, D140H, K156Q, P159T, P159L, R170C, R170P, P178S, P182L, N188S, G189V, A190T, A190E, G195W, L197F, K198E, G202R, M361I, F213I, F216Y, T231R, E233Stop, insertion of M between amino acids 241 and 242, S237P, F251L, H255Q, D409H, R257Q, P266A, P266R, P266L, S271N, R285H, P289L, Y304C, Y304Stop, H311R, W312C, G325R, E326K, A341T, C342G, C342Y, V352L, R353G, G355D, R359Q, S364R, S364T, S366G, N370S, L371V, V375L, G377S, D380A, G389E, G390R, N392I, V394L, N396T, F397S, V398L, V398F, D399N, P401L, I402F, I402T, L444P, A456P, D409V, D409G, K413Q, Q414R, P415R, K425E, R433G, L444R, A446P, N462K, R463C, T491I, R496C, and R496H. In some embodiments of these methods, the mutation in a GBA gene results in the expression of a GBA protein having one or more of the following mutations: S12I, insertion of SY between amino acids 13 and 14, frameshift mutation at amino acid 14, L157Q, V460M, and K416Q. In some embodiments of these methods, the mutation in a GBA gene results in the expression of a GBA protein having one or more of the following mutations: K(-27)R, 2(-4)X, G10S, V15M, C16S, D24N, G35S, S42N, T43I, R47X, R48W, R48Q, Q73X, K74X, V78A, M85T, L105R, S107L, F109V, E111K, G113E, G113A, I119S, V121A, P122L, M123T, T134P, Y135X, A136E, K157Q, K157N, I161N, I161S, H162P, R163X, Q169X, R170P, S173X, L174F, A176D, W179X, T180P, P182T, W184R, L185F, N188K, V191G, V191E, G195E, S196P, L197P, G202E, Y205C, W209R, A210V, Y212H, F213C, Y220C, E233X, E233D, G239V, G243V, Y244H, P245H, R257X, F259L, G265D, P266A, S271N, L279P, R285C, K303I, Y304X, V305L, A309V, W312R, Y313H, D315H, A318D, P319A, T323I, L324P, G325W, R329C, F331S, L336P, C342R, W348G, G349K, Q350X, R353W, S356F, R359X, Y363C, S364N, S366N, S366T, T369M, N370K, W378G, W378X, D380N, D380H, W381X, N382K, L383R, L385P, P387L, E388X, P391L, W393R, W393L, V394L, R395C, R395P, V398I, D399Y, F411I, Y412H, Q414X, M416V, F417V, Y418C, R433S, H451R, L461P, N462S, R463P, D474Y, G478S, L480P, I489T. In some embodiments of these methods, the mutation in a GBA gene includes one or more of the following insertion mutations: 84GG, 122CC, c.153-154insTACAGC, 155-156insACAGCT, D127X, 500insT, c.8410842insTGA, 1093-1094insG, 1098insA, c.1122-1123insTG, c1326insT, c.1515_1516ins AGTGAGGGCAAT, and 1562-1585ins. In some embodiments of these methods, the mutation in a GBA gene results in the expression of a GBA protein having one or more of the following deletion mutations: c.42-65del24, 72delC, 203Cdel, del205-209ACCTT, c.222-224delTAC, del255-257GCG, c.330delA, 344delA, c.533delC, 534delT, 595-596delCT, c.708delC, V214X, 793delC, 898delG, 914Cdel, c.953delT, g5255delT, L354X, c.1214delGC, 1324-1326delATT, c.1439-1445del7, 1450del2, 1447-1466del20 insTG, and c.1510delT,C,T. In some embodiments of these methods, the mutation in a GBA gene is one or more of the following splice junction mutations: IVS2 +1G>A, IVS2+1G>T, IVS4 +1G>A, IVS5 +1G>T, g.4252C>G, g.4426A>G, IVS6-1G>C, g.5230G>A, IVS8+1, IVS8(-11delC)(-14T>A), IVS9-3C>G, IVS10-1G>A R463Q, IVS10+2T>A, and IVS10(+2). In some embodiments of these methods, the mutation in a GBA gene includes a IVS2+1 mutation. In some embodiments of these methods, the mutation in a GBA gene results in the expression of a GBA protein having one or more of the following mutations: c.(-203)A>G + IVS4-2a>g, S448P, c.1379G>A c.1469A>G, g.7319T>C + g.7741T>C, c.203-204insC, RecTL. In some embodiments of these methods, the mutation in a GBA gene results in the expression of a GBA protein having a Rec1 mutation (L444P, A456P, and V460M). Additional examples of mutations in a GBA gene and methods of detecting mutations in a GBA gene are described in, e.g., Beutler et al., Blood Cells, Molecules, and Diseases 35 (2005) 355-364; Gan-Or et al., Neurology 70(24):2277-83, 2008; Hruska et al., Human Mutation 29(5), 567-583, 2008; and Tsuang D., et al., Neurology 6; 79(19): 1944-1950, 2012.

Additional exemplary mutations in a GBA gene and exemplary methods for detecting a mutation in a GBA gene are described, e.g., in Hruska et al., Human Mutation 29(5):567-583, 2008; and Beautler et al., Blood Cells, Molecules, and Diseases 35:355-364, 2005. Non-limiting examples of methods for detecting a mutation in a GBA gene include the techniques of restriction fragment length polymorphism (RFLP); amplification refractory mutation system (ARMS) PCR; allele-specific amplification (ASA); multiplex PCR; nested PCR; reverse transcriptase (RT) PCR; real-time PCR; multiplex ligation-dependent probe amplification (MLPA); denaturing gradient gel electrophoresis (DGGE); denaturing high-performance liquid chromatography (DHPLC); temperature gradient gel electrophoresis (TGGE); single strand conformational polymorphism (SSCP); heteroduplex analysis (HET); single nucleotide primer extension (i.e., mini sequencing); chemical cleavage of mismatch (CCM); TaqMan and molecular beacons; enzyme mismatch cleavage (EMC); protein truncation test (PTT); oligonucleotide ligation assay (OLA); fluorescence in situ hybridization (FISH); cleavage fragment length polymorphism (CFLP); mismatch binding proteins (e.g., MutS); allele-specific oligonucleotide (ASO) hybridization; differential reactivity with sodium bisulfite; base excision sequencing scanning (BESS); and ribonuclease mismatch cleavage (e.g., NIRCA). Additional methods for detecting a mutation in a GBA gene are described in, e.g., Mahdieh et al., Iran J. Pediatr. 23(4):375-388, 2013.

Some embodiments of any of the methods described herein further include detecting a level of one or more (e.g., 2, 3, 4, 5, or 6) of acid beta-glucocerebrosidase, acid sphingomyelinase, acid alpha-glucosidase, galactocerebrosidase, alpha-galactosidase A, and alpha-L-iduronidase, and further identifying a subject having a decrease in the level of at least one (e.g., 2, 3, 4, 5, or 6) of acid beta-glucocerebrosidase, acid sphingomyelinase, acid alpha-glucosidase, galactocerebrosidase, alpha-galactosidase A, and alpha-L-iduronidase, as compared to control level(s) as having a proteopathy. Exemplary methods for detecting the levels of acid beta-glucocerebrosidase, acid sphingomyelinase, acid alpha-glucosidase, galactocerebrosidase, alpha-galactosidase A, and alpha-L-iduronidase in a sample including a biological fluid are described in, e.g., U.S. Patent Application Publication No. 2008/0248513 and WO 13/070953. Kits for detecting the level of acid beta-glucocerebrosidase, acid sphingomyelinase, acid alpha-glucosidase, galactocerebrosidase, alpha-galactosidase A, and alpha-L-iduronidase in a sample including a biological fluid are also commercially available.

Some embodiments of any of the methods described herein further include detecting the level of one or more of amyloid beta, Tau protein, Aβ42, PARK7, PARK2, TDP-43, ALS2, and cystatin C in a biological sample (e.g., a sample comprising a biological fluid) from a subject, and further identifying a subject having an elevated level(s) of one or more of amyloid beta, Tau protein, Aβ42, PARK7, PARK2, TDP-43, ALS2, and cystatin C (as compared to a control level(s), e.g., any of the control levels described herein) as having a proteopathy. Some embodiments of any of the methods described herein further include detecting a mutation associated with a proteopathy in one or more genes selected from the group consisting of: α-synuclein, Parkin, DJ1, PINK1, LRRK2, VDS₃₅, EIF4G1, SOD1, FUS, ALS2, SETX, and SPG11 in a biological sample comprising genomic DNA from a subject, and further identifying a subject having a mutation associated with a proteopathy as having a proteopathy. Some embodiments of any of the methods described herein, further include detecting the presence of one or two alleles of APOE-ε4 in a biological sample comprising genomic DNA from a subject, and further identifying a subject having one or two alleles of APOE-ε4 in a subject as having a proteopathy.

The methods described herein may further include after step (c): (d) administering a treatment for a proteopathy to a subject identified as having a proteopathy. In some examples, the treatment is administering a glucosyl ceramide synthase inhibitor (e.g., any of the glucosyl ceramide synthase inhibitors described herein) or a recombinant enzyme (e.g., a recombinant glucocerebrosidase, e.g., imiglucerase, velaglucerase, or taliglucerase). In some examples, the treatment is administering a glucosyl ceramide synthase inhibitor selected from the group of: (i) eliglustat; (ii) miglustat; (iii) quinuclidin-3-yl (2-(4'-fluoro-[1,1'-biphenyl]-3-yl)propan-2-yl)carbamate; (iv) (S)-quinuclidin-3-yl (2-(2-(4-fluorophenyl)thiazol-4-yl)propan-2-yl)carbamate; (v) (S)-quinuclidin-3-yl (2-(4'-(2-methoxyethoxy)-[1,1'-biphenyl]-4-yl)propan-2-yl)carbamate; and the pharmaceutically acceptable salts and prodrugs thereof.

In any of these methods, a control level(s) can be, e.g., a level(s) in a subject not presenting with one or more symptoms of a proteopathy and/or not diagnosed as having a proteopathy, a level(s) in a subject that has no history of a proteopathy, a level(s) in a healthy subject or a population of healthy subjects, or a threshold level(s) (e.g., a level(s) above which indicates that the subject has a proteopathy or an increased risk of developing a proteopathy). The control level(s) can be, e.g., a level(s) in a sample from a subject or a population of subjects that has/have no history of a proteopathy and, optionally, also does/do not have a genetically-related family member diagnosed or identified as having a proteopathy. The control level(s) can be, e.g., a threshold level(s) that is indicative of a subject having a proteopathy or a subject having an increased risk of developing a proteopathy when the measured level is above the threshold level. Additional exemplary control level(s) of any of the sphingolipid(s) are described herein.

Some embodiments further include recording the identification of a proteopathy in the subject in the subject's medical record (e.g., a computer readable medium). Some examples further include informing the subject, the subject's family, and/or the subject's primary care physician or attending physician of the identification of a proteopathy in the subject. Some examples further include informing the subject's insurance provider of the identification of a proteoatphy in the subject.

### Methods of Determining a Subject's Risk of Developing a Proteopathy

Also described are methods of determining a subject's risk of developing a proteopathy that include: (a) providing a sample incluidng a biological fluid obtained from a subject; (b) determining the level of at least one (e.g., 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, or 44) sphingolipid in the sample of (a), where the at least one sphingolipid is selected from the group of: dihexosylceramide C24:1; dihexosylceramide C24:0; dihexosylceramide C23:0; dihexosylceramide C22:0; dihexosylceramide C20:0; dihexosylceramide C18:0; dihexosylceramide C16:0; lactosylceramide C24:1; lactosylceramide C24:0; lactosylceramide C23:0; lactosylceramide C22:0; lactosylceramide C20:0; lactosylceramide C18:0; lactosylceramide C16:0; ceramide C24:1; ceramide C24:0; ceramide C23:0; ceramide C22:0; ceramide C20:0; ceramide C18:0; ceramide C16:0; ceramide C14:0; globotriaosylceramide C24:1; globotriaosylceramide C24:0; globotriaosylceramide C23:0; globotriaosylceramide C22:0; globotriaosylceramide C20:0; globotriaosylceramide C18:0; globotriaosylceramide C16:0; galactosylceramide C24:1; galactosylceramide C24:0; galactosylceramide C23:0; galactosylceramide C22:0; galactosylceramide C20:0; galactosylceramide C18:0; galactosylceramide C16:0; glucosylceramide C24:1; glucosylceramide C24:0; glucosylceramide C23:0; glucosylceramide C22:0; glucosylceramide C20:0; glucosylceramide C18:0; glucosylceramide C16:0; and glucosylsphingosine; and (c) identifying a subject having an elevated level(s) of the at least one (e.g., 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, or 44) sphingolipid as compared to a control level(s) as having an increased risk of developing a proteopathy. Some embodiments of these methods further include enriching the sample of (a) before the step of determining the level(s) of the at least one sphingolipid.

In some embodiments of these methods, step (b) includes determining the level(s) of at least one (e.g., 2, 3, 4, 5, 6, 7, 8, 9, 10, 11, 12, 13, 14, 15, 16, or 17) sphingolipid selected from the group of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0. In some embodiments of these methods, step (b) includes determining the levels of at least two (e.g., 3, 4, 5, 6, 7, 8, 9, 10, 11, 12, 13, 14, 15, 16, or 17) sphingolipids selected from the group of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0, and the subject is identified as having an increased risk of developing a proteopathy when at least one or both of the two levels is elevated as compared to control level(s).

In some embodiments of these methods, step (b) includes detecting the levels of at least three (e.g., 4, 5, 6, 7, 8, 9, 10, 11, 12, 13, 14, 15, 16, or 17) sphingolipids selected from the group of: dihexosylceramide C24: 1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24: 1, glucosylceramide C24:0, glucosylceramide C23:0, glucosylceramide C22:0, glucosylceramide C20:0, glucosylceramide C18:0, and glucosylceramide C16:0, and the subject is identified as having an increased risk of developing a proteopathy when at least one, at least two, or all three of the three levels is elevated as compared to control level(s). In some embodiments of these methods, step (b) includes detecting the levels of at least four (e.g., 5, 6, 7, 8, 9, 10, 11, 12, 13, 14, 15, 16, or 17) sphingolipids selected from the group of: dihexosylceramide C24: 1, dihexosylceramide C24:0, dihexosylceramide C20:0, ceramide C24:0, ceramide C23:0, globotriaosylceramide C24: 1, globotriaosylceramide C16:0, galactosylceramide C24:0, galactosylceramide C23:0, galactosylceramide C16:0, glucosylceramide C24: 1, glucosylceramide C24:0, glucosylceramide C23:0, glucosylceramide C22:0, glucosylceramide C20:0, glucosylceramide C18:0, and glucosylceramide C16:0, and the subject is identified as having an increased risk of developing a proteopathy when at least one, at least two, at least three, or all four of the four levels is elevated as compared to control level(s).

Also described are methods of determining a subject's risk of developing a proteopathy that include: (a) providing a sample incluidng a biological fluid obtained from a subject; (b) determining the level of at least one (e.g., 2, 3, 4, 5, 6, or 7) sphingolipid in the sample of (a), where the at least one sphingolipid is selected from the group of: sphingomyelin C24:1; sphingomyelin C24:0; sphingomyelin C23:0; sphingomyelin C22:0; sphingomyelin C20:0; sphingomyelin C18:0; and sphingomyelin C16:0; and (c) identifying a subject having a decreased level(s) of the at least one (e.g., 2, 3, 4, 5, 6, or 7) sphingolipid as compared to a control level(s) as having an increased risk of developing a proteopathy. Some embodiments of these methods further include enriching the sample of (a) before the step of determining the level(s) of the at least one sphingolipid.

Also described are methods of determining a subject's risk of developing a proteopathy that include: (a) providing a sample include a biological fluid obtained from a subject; (b) determining at least one (e.g., 2, 3, 4, 5, or 6) of total dihexosylceramide level, total lactosylceramide level, total globotriaosylceramide level, total galactosylceramide level, total glucosylceramide level, and total phosphatidylcholine level in the sample of step (a); and (c) identifying a subject having an elevated level(s) of at least one (e.g., 2, 3, 4, 5, or 6) of the total dihexosylceramide level, the total lactosylceramide level, the total globotriaosylceramide level, the total galactosylceramide level, the total glucosylceramide level, and the total phosphatidylcholine level as compared to a control level(s), as having an increased risk of developing a proteopathy. Some embodiments of these methods further include a step of enriching the sample of (a) for lipids prior to the step of detecting the level(s) of at least one of total dihexosylceramide level, total lactosylceramide level, total globotriaosylceramide level, total galactosylceramide level, total glucosylceramide level, and total phosphatidylcholine level.

In some embodiments of these methods, step (b) includes determining one or both of the total galactosylceramide level and the total glucosylceramide level. In some embodiments of these methods, step (b) includes determining both the total galactosylceramide level and the total glucosylceramide level, and the subject is identified as having an increased risk of developing a proteopathy when at least one or both of the levels is increased as compared to control level(s).

Also described are methods of determining a subject's risk of developing a proteopathy that include: (a) providing a sample include a biological fluid obtained from a subject; (b) determining one or both of total ceramide level and total sphingomyelin level in the sample of step (a); and (c) identifying a subject having a decreased level(s) of one or both of the the total ceramide level and the total sphingomyelin level, as having an increased risk of developing a proteopathy. Some embodiments of these methods further include a step of enriching the sample of (a) for lipids prior to the step of detecting the level(s) of one or both of total ceramide level and total sphingomyelin level.

Also described are methods of determining a subject's risk of developing a proteopathy that include: (a) providing a sample include a biological fluid obtained from a subject; (b) determining the ratio of glucosylceramide C24:0 to sphingomyelin C24:0 in the sample of step (a); and (c) identifying a subject having an increased ratio of glucosylceramide C24:0 to sphingomyelin C24:0 as compared to a control ratio, as having an increased risk of developing a proteopathy. Some embodiments of these methods further include a step of enriching the sample of (a) for lipids prior to the step of determining the ratio of glucosylceramide C24:0 to sphingomyelin C24:0 in the sample of (a).

In any of the methods described herein, the sample may include blood, serum, plasma, or cerebrospinal fluid. Some embodiments of any of these methods further include detecting a mutation in a glucocerebrosidase (GBA) gene in a sample including genomic DNA from the subject, and further identifying a subject having a mutation in a GBA gene as having an increased risk of developing a proteopathy. Non-limiting mutations in a GBA gene can result in the expression of a GBA protein having, e.g., one or more of the following mutations: V15L, C16S, Δ36T, F37V, E41K, G46E, R48W, L66P, K79N, A90T, S107L, N117D, I119T, R120W, R120Q, P122S, M123V, D127V, R131C, R131L, T134I, D140H, K156Q, P159T, P159L, R170C, R170P, P178S, P182L, N188S, G189V, A190T, A190E, G195W, L197F, K198E, G202R, M361I, F213I, F216Y, T231R, E233Stop, insertion of M between amino acids 241 and 242, S237P, F251L, H255Q, D409H, R257Q, P266A, P266R, P266L, S271N, R285H, P289L, Y304C, Y304Stop, H311R, W312C, G325R, E326K, A341T, C342G, C342Y, V352L, R353G, G355D, R359Q, S364R, S364T, S366G, N370S, L371V, V375L, G377S, D380A, G389E, G390R, N392I, V394L, N396T, F397S, V398L, V398F, D399N, P401L, I402F, I402T, L444P, A456P, D409V, D409G, K413Q, Q414R, P415R, K425E, R433G, L444R, A446P, N462K, R463C, T491I, R496C, and R496H. In some embodiments of these methods, the mutation in a GBA gene results in the expression of a GBA protein having one or more of the following mutations: S12I, insertion of SY between amino acids 13 and 14, frameshift mutation at amino acid 14, L157Q, V460M, and K416Q. In some embodiments of these methods, the mutation in a GBA gene results in the expression of a GBA protein having one or more of the following mutations: K(-27)R, 2(-4)X, G10S, V15M, C16S, D24N, G35S, S42N, T43I, R47X, R48W, R48Q, Q73X, K74X, V78A, M85T, L105R, S107L, F109V, E111K, G113E, G113A, I119S, V121A, P122L, M123T, T134P, Y135X, A136E, K157Q, K157N, I161N, I161S, H162P, R163X, Q169X, R170P, S173X, L174F, A176D, W179X, T180P, P182T, W184R, L185F, N188K, V191G, V191E, G195E, S196P, L197P, G202E, Y205C, W209R, A210V, Y212H, F213C, Y220C, E233X, E233D, G239V, G243V, Y244H, P245H, R257X, F259L, G265D, P266A, S271N, L279P, R285C, K303I, Y304X, V305L, A309V, W312R, Y313H, D315H, A318D, P319A, T323I, L324P, G325W, R329C, F331S, L336P, C342R, W348G, G349K, Q350X, R353W, S356F, R359X, Y363C, S364N, S366N, S366T, T369M, N370K, W378G, W378X, D380N, D380H, W381X, N382K, L383R, L385P, P387L, E388X, P391L, W393R, W393L, V394L, R395C, R395P, V398I, D399Y, F411I, Y412H, Q414X, M416V, F417V, Y418C, R433S, H451R, L461P, N462S, R463P, D474Y, G478S, L480P, I489T. In some embodiments of these methods, the mutation in a GBA gene includes one or more of the following insertion mutations: 84GG, 122CC, c.153-154insTACAGC, 155-156insACAGCT, D127X, 500insT, c.8410842insTGA, 1093-1094insG, 1098insA, c.1122-1123insTG, c1326insT, c.1515_1516ins AGTGAGGGCAAT, and 1562-1585ins. In some embodiments of these methods, the mutation in a GBA gene results in the expression of a GBA protein having one or more of the following deletion mutations: c.42-65del24, 72delC, 203Cdel, del205-209ACCTT, c.222-224delTAC, del255-257GCG, c.330delA, 344delA, c.533delC, 534delT, 595-596delCT, c.708delC, V214X, 793delC, 898delG, 914Cdel, c.953delT, g5255delT, L354X, c.1214delGC, 1324-1326delATT, c.1439-1445del7, 1450del2, 1447-1466del20 insTG, and c.1510delT,C,T. In some embodiments of these methods, the mutation in a GBA gene is one or more of the following splice junction mutations: IVS2 +1G>A, IVS2+1G>T, IVS4 +1G>A, IVS5 +1G>T, g.4252C>G, g.4426A>G, IVS6-1G>C, g.5230G>A, IVS8+1, IVS8(-11delC)(-14T>A), IVS9-3C>G, IVS10-1G>A R463Q, IVS10+2T>A, and IVS10(+2). In some embodiments of these methods, the mutation in a GBA gene includes a IVS2+1 mutation. In some embodiments of these methods, the mutation in a GBA gene results in the expression of a GBA protein having one or more of the following mutations: c.(-203)A>G + IVS4-2a>g, S448P, c.1379G>A c.1469A>G, g.7319T>C + g.7741T>C, c.203-204insC, RecTL. In some embodiments of these methods, the mutation in a GBA gene results in the expression of a GBA protein having a Rec1 mutation (L444P, A456P, and V460M). Additional examples of mutations in a GBA gene and methods of detecting mutations in a GBA gene are described in, e.g., Beutler et al., Blood Cells, Molecules, and Diseases 35 (2005) 355-364; Gan-Or et al., Neurology 70(24):2277-83, 2008; Hruska et al., Human Mutation 29(5), 567-583, 2008; and Tsuang D., et al., Neurology 6; 79(19): 1944-1950, 2012.

Additional exemplary mutations in a GBA gene and exemplary methods for detecting a mutation in a GBA gene are described, e.g., in Hruska et al., Human Mutation 29(5):567-583, 2008; and Beautler et al., Blood Cells, Molecules, and Diseases 35:355-364, 2005.

Non-limiting examples of methods for detection a mutation in a GBA gene include the techniques of restriction fragment length polymorphism (RFLP); amplification refractory mutation system (ARMS) PCR; allele-specific amplification (ASA); multiplex PCR; nested PCR; reverse transcriptase (RT) PCR; real-time PCR; multiplex ligation-dependent probe amplification (MLPA); denaturing gradient gel electrophoresis (DGGE); denaturing high-performance liquid chromatography (DHPLC); temperature gradient gel electrophoresis (TGGE); single strand conformational polymorphism (SSCP); heteroduplex analysis (HET); single nucleotide primer extension (i.e., mini sequencing); chemical cleavage of mismatch (CCM); TaqMan and molecular beacons; enzyme mismatch cleavage (EMC); protein truncation test (PTT); oligonucleotide ligation assay (OLA); fluorescence in situ hybridization (FISH); cleavage fragment length polymorphism (CFLP); mismatch binding proteins (e.g., MutS); allele-specific oligonucleotide (ASO) hybridization; differential reactivity with sodium bisulfite; base excision sequencing scanning (BESS); and ribonuclease mismatch cleavage (e.g., NIRCA). Additional methods for detecting a mutation in a GBA gene are described in, e.g., Mahdieh et al., Iran J. Pediatr. 23(4):375-388, 2013.

Some embodiments of any of the methods described herein further include detecting a level of one or more (e.g., 2, 3, 4, 5, or 6) of acid beta-glucocerebrosidase, acid sphingomyelinase, acid alpha-glucosidase, galactocerebrosidase, alpha-galactosidase A, and alpha-L-iduronidase, and further identifying a subject having a decrease in the level of at least one (e.g., 2, 3, 4, 5, or 6) of acid beta-glucocerebrosidase, acid sphingomyelinase, acid alpha-glucosidase, galactocerebrosidase, alpha-galactosidase A, and alpha-L-iduronidase, as compared to control level(s), as having an increased risk of developing a proteopathy. Exemplary methods for detecting the levels of acid beta-glucocerebrosidase, acid sphingomyelinase, acid alpha-glucosidase, galactocerebrosidase, alpha-galactosidase A, and alpha-L-iduronidase in a sample including a biological fluid are described in, e.g., U.S. Patent Application Publication No. 2008/0248513 and WO 13/070953. Kits for detecting the level of acid beta-glucocerebrosidase, acid sphingomyelinase, acid alpha-glucosidase, galactocerebrosidase, alpha-galactosidase A, and alpha-L-iduronidase in a sample including a biological fluid are also commercially available.

Some embodiments of any of the methods described herein further include detecting the level of one or more of amyloid beta, Tau protein, Aβ42, PARK7, PARK2, TDP-43, ALS2, and cystatin C in a biological sample (e.g., a sample comprising a biological fluid) from a subject, and further identifying a subject having an elevated level(s) of one or more of amyloid beta, Tau protein, Aβ42, PARK7, PARK2, TDP-43, ALS2, and cystatin C (as compared to a control level(s), e.g., any of the control levels described herein) as having an increased risk of developing a proteopathy. Some embodiments of any of the methods described herein further include detecting a mutation associated with a proteopathy in one or more genes selected from the group consisting of: α-synuclein, Parkin, DJ1, PINK1, LRRK2, VDS35, EIF4G1, SOD1, FUS, ALS2, SETX, and SPG11 in a biological sample comprising genomic DNA from a subject, and further identifying a subject having a mutation associated with a proteopathy as having an increased risk of developing a proteopathy. Some embodiments of any of the methods described herein, further include detecting the presence of one or two alleles of APOE-ε4 in a biological sample comprising genomic DNA from a subject, and further identifying a subject having one or two alleles of APOE-ε4 in a subject as having an increased risk of developing a proteopathy.

The methods described herein may further include after step (c): (d) administering a treatment for a proteopathy to a subject identified as having an increased risk of developing a proteopathy. In some embodiments of these methods, the treatment is administering a glucosyl ceramide synthase inhibitor (e.g., any of the glucosyl ceramide synthase inhibitors described herein or known in the art) or a recombinant enzyme (e.g., a recombinant glucocerebrosidase, e.g., imiglucerase, velaglucerase, or taliglucerase). In some examples, the treatment is administering a glucosyl ceramide synthase inhibitor selected from the group of: (i) eliglustat; (ii) miglustat; (iii) quinuclidin-3-yl (2-(4'-fluoro-[1,1'-biphenyl]-3-yl)propan-2-yl)carbamate; (iv) (S)-quinuclidin-3-yl (2-(2-(4-fluorophenyl)thiazol-4-yl)propan-2-yl)carbamate; (v) (S)-quinuclidin-3-yl (2-(4'-(2-methoxyethoxy)-[1,1'-biphenyl]-4-yl)propan-2-yl)carbamate; and the pharmaceutically acceptable salts and prodrugs thereof.

In any of these methods, a control level(s) can be, e.g., a level(s) in a subject not presenting with one or more symptoms of a proteopathy and/or not diagnosed as having a proteopathy, a level(s) in a healthy subject or a population of healthy subjects, a threshold level(s) (e.g., a level(s) above which indicates that the subject has a proteopathy or is at risk for developing a proteopathy), a level(s) in a subject or population of subjects not identified as having a genetic risk (e.g., an elevated genetic risk) of developing a proteopathy, a level(s) in a sample from a subject or a population of subjects that has/have no history of a proteopathy and, optionally, also does/do not have a genetically-related family member diagnosed or identified as having a proteopathy, or a threshold level(s) that is indicative of a subject having a proteopathy or a subject having an increased risk of developing a proteopathy when the measured level is above the threshold level. Additional exemplary control level(s) of any of the sphingolipid(s) are described herein.

Some embodiments further include recording a subject's risk of developing a proteopathy in the subject's medical record (e.g., a computer readable medium). Some examples further include informing the subject, the subject's family, and/or the subject's primary care physician or attending physician of the subject's risk of developing a proteopathy. Some examples further include informing the subject's insurance provider of the subject's risk of developing a proteopathy.

### Methods of Determining the Stage of a Proteopathy

Also described are methods of determining the stage of a proteopathy in a subject that include: (a) providing a sample including a biological fluid obtained from a subject suspected of having a proteopathy or identified as having a proteopathy; (b) determining the level of at least one (e.g., 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or 51) sphingolipid in the sample of (a), wherein the at least one sphingolipid is selected from the group of: dihexosylceramide C24:1; dihexosylceramide C24:0; dihexosylceramide C23:0; dihexosylceramide C22:0; dihexosylceramide C20:0; dihexosylceramide C18:0; dihexosylceramide C16:0; lactosylceramide C24: 1; lactosylceramide C24:0; lactosylceramide C23:0; lactosylceramide C22:0; lactosylceramide C20:0; lactosylceramide C18:0; lactosylceramide C16:0; ceramide C24:1; ceramide C24:0; ceramide C23:0; ceramide C22:0; ceramide C20:0; ceramide C18:0; ceramide C16:0; ceramide C14:0; globotriaosylceramide C24:1; globotriaosylceramide C24:0; globotriaosylceramide C23:0; globotriaosylceramide C22:0; globotriaosylceramide C20:0; globotriaosylceramide C18:0; globotriaosylceramide C16:0; sphingomyelin C24: 1; sphingomyelin C24:0; sphingomyelin C23:0; sphingomyelin C22:0; sphingomyelin C20:0; sphingomyelin C18:0; sphingomyelin C16:0; galactosylceramide C24:1; galactosylceramide C24:0; galactosylceramide C23:0; galactosylceramide C22:0; galactosylceramide C20:0; galactosylceramide C18:0; galactosylceramide C16:0; glucosylceramide C24: 1; glucosylceramide C24:0; glucosylceramide C23:0; glucosylceramide C22:0; glucosylceramide C20:0; glucosylceramide C18:0; glucosylceramide C16:0; and glucosylsphingosine; and (c) determining the stage of the proteopathy in the subject from the at least one (e.g., 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or 51) level. Some embodiments of these methods further include a step of enriching the sample of (a) for lipids prior to the step of detecting the level(s) of the at least one sphingolipid.

In some embodiments of these methods, step (b) includes determining the level(s) of at least one (e.g., 2, 3, 4, 5, 6, 7, 8, 9, 10, 11, 12, 13, 14, 15, 16, 17, 18, 19, 20, 21, or 22) sphingolipid selected from the group of: dihexosylceramide C24:1, dihexosylceramide C24:0, dihexosylceramide C20:0, ceramide C24:0, ceramide C23:0, globotriaosylceramide C24:1, globotriaosylceramide C16:0, sphingomyelin C24:0, sphingomyelin C24:1, sphingomyelin C23:0, sphingomyelin C22:0, sphingomyelin C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0. In some embodiments of these methods, step (b) includes determining the levels of at least two (e.g., 3, 4, 5, 6, 7, 8, 9, 10, 11, 12, 13, 14, 15, 16, 17, 18, 19, 20, 21, or 22) sphingolipids selected from the group of: dihexosylceramide C24: 1, dihexosylceramide C24:0, dihexosylceramide C20:0, ceramide C24:0, ceramide C23:0, globotriaosylceramide C24:1, globotriaosylceramide C16:0, sphingomyelin C24:0, sphingomyelin C24:1, sphingomyelin C23:0, sphingomyelin C22:0, sphingomyelin C16:0, galactosylceramide C24:0, galactosylceramide C23:0, galactosylceramide C16:0, glucosylceramide C24: 1, glucosylceramide C24:0, glucosylceramide C23:0, glucosylceramide C22:0, glucosylceramide C20:0, glucosylceramide C18:0, and glucosylceramide C16:0, and the stage of the proteopathy in the subject is determined from at least one or both of the two levels.

In some embodiments of these methods, step (b) includes detecting the levels of at least three (e.g., 4, 5, 6, 7, 8, 9, 10, 11, 12, 13, 14, 15, 16, 17, 18, 19, 20, 21, or 22) sphingolipids selected from the group of: dihexosylceramide C24: 1, dihexosylceramide C24:0, dihexosylceramide C20:0, ceramide C24:0, ceramide C23:0, globotriaosylceramide C24:1, globotriaosylceramide C16:0, sphingomyelin C24:0, sphingomyelin C24:1, sphingomyelin C23:0, sphingomyelin C22:0, sphingomyelin C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0, and the stage of the proteopathy in the subject is determined from at least one, at least two, or all three of the three levels. In some embodiments of these methods, step (b) includes detecting the levels of at least four (e.g., 5, 6, 7, 8, 9, 10, 11, 12, 13, 14, 15, 16, 17, 18, 19, 20, 21, or 22) sphingolipids selected from the group of: dihexosylceramide C24: 1, dihexosylceramide C24:0, dihexosylceramide C20:0, ceramide C24:0, ceramide C23:0, globotriaosylceramide C24:1, globotriaosylceramide C16:0, sphingomyelin C24:0, sphingomyelin C24:1, sphingomyelin C23:0, sphingomyelin C22:0, sphingomyelin C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0, and the stage of the proteopathy in the subject is determined from at least one, at least two, at least three, or all four of the four levels.

Also described are methods of determining the stage of a proteopathy in a subject that include: (a) providing a sample including a biological fluid obtained from a subject suspected of having a proteopathy or identified as having a proteopathy; (b) determining at least one (e.g., 2, 3, 4, 5, 6, 7, or 8) of total dihexosylceramide level, total lactosylceramide level, total ceramide level, total globotriaosylceramide level, total sphingomyelin level, total galactosylceramide level, total glucosylceramide level, and total phosphatidylcholine level in the sample of step (a); and (c) determining the stage of the proteopathy in the subject from at least one (e.g., 2, 3, 4, 5, 6, 7, or 8) of the total dihexosylceramide level, the total lactosylceramide level, the total ceramide level, the total globotriaosylceramide level, the total sphingomyelin level, the total galactosylceramide level, the total glucosylceramide level, and the total phosphatidylcholine level. Some embodiments further include a step of enriching the sample of (a) for lipids prior to the step of determining at least one of total dihexosylceramide level, total lactosylceramide level, total ceramide level, total globotriaosylceramide level, total sphingomyelin level, total galactosylceramide level, total glucosylceramide level, and total phosphatidylcholine level. In some embodiments of these methods, step (b) includes determining at least one (e.g., 2, 3, or 4) of the total ceramide level, the total sphingomyelin level, the total galactosylceramide level, and the total glucosylceramide level. In some embodiments of these methods, step (b) includes determining at least two (e.g., 3 or 4) of the total ceramide level, the total sphingomyelin level, the total galactosylceramide level, and the total glucosylceramide level, and the stage of the proteopathy in the subject is determined from at least one or both of the two levels.

In some embodiments of these methods, step (b) includes determining at least three (e.g., 4) of the total ceramide level, the total sphingomyelin level, the total galactosylceramide level, and the total glucosylceramide level, and the stage of the proteopathy in the subject is determined from at least one, at least two, or all three of the three levels. In some embodiments of these methods, step (b) includes determining the total ceramide level, the total sphingomyelin level, the total galactosylceramide level, and the total glucosylceramide level, and the stage of the proteopathy in the subject is determined from at least one, at least two, at least three, or all four of the four levels.

In some embodiments of any of these methods, determining the stage can include comparing the level(s) of the at least one sphingolipid or the at least one of total dihexosylceramide level, total lactosylceramide level, total ceramide level, total globotriaosylceramide level, total sphingomyelin level, total galactosylceramide level, total glucosylceramide level, and total phosphatidylcholine level to a control level(s) of the at least one sphingolipid or the at least one of total dihexosylceramide level, total lactosylceramide level, total ceramide level, total globotriaosylceramide level, total sphingomyelin level, total galactosylceramide level, total glucosylceramide level, and total phosphatidylcholine level, respectively. The control level(s) can be, e.g., a threshold level(s) that is indicative of a subject having a specific stage of proteopathy when the measured level is above the threshold level. In some embodiments of any of these methods, the control level(s) can be a level(s) in a sample from a subject that has been diagnosed or identified as having a specific stage of a proteopathy. In some examples, the control level(s) can be a range of level(s) that represent the level(s) of the at least one sphingolipid or the at least one of total dihexosylceramide level, total lactosylceramide level, total ceramide level, total globotriaosylceramide level, total sphingomyelin level, total galactosylceramide level, total glucosylceramide level, and total phosphatidylcholine level that are present in a subject or a population of subjects determined or identified as having a specific stage of a proteopathy.

In these methods, the sample may include blood, serum, plasma, or cerebrospinal fluid. These methods may further include after (c): (d) administering a treatment for stage I, stage II, stage III, stage IV, or stage V of the proteopathy to a subject identified to have stage I, stage II, stage III, stage IV, or stage V of the proteopathy, respectively.

Non-limiting examples of treatments of early stages of Parkinson's disease (e.g., stages I, II, and/or III) can include, e.g., one or more of rasagiline, monoamine oxidase type B (MAO-B) inhibitors, dopamine agonists, and levodopa (with a decarboxylase inhibitor such as carbidopa). Non-limiting examples of treatments of later stages of Parkinson's disease (e.g., stages IV and/or V) can include: hospitalization or hospice care, and/or an increased dosage or frequency of administration of one or more of rasagiline, monoamine oxidase type B (MAO-B) inhibitors, dopamine agonists, and levodopa (with a decarboxylase inhibitor such as carbidopa) than was administered at an earlier stage of Parkinson's disease. In some examples, a treatment of later stages of Parkinson's disease (e.g., stages IV and/or V) do not include levodopa (optionally with carbidopa).

Non-limiting examples of treatments of early stages of Alzheimer's disease (e.g., stages I, II, III, and/or IV) can include, e.g., one or more of a cholinesterase inhibitor (e.g., donepezil (Aricept), rivastigmine (Exelon), and galantamine (Razadyne)). Non-limiting examples of treatments of moderate to severe stages of Alzheimer's disease (e.g., stage V, VI, or VII) can include, e.g., memantine (Namenda) or the combination of memantine (Namenda) and donepezil (Aricept).

Non-limiting examples of treatments of early stages of ALS (e.g., stages I, II, and/or III) can include, e.g., riluzole (Rilutek), baclofen (Lioresal), a NSAID (e.g., ibuprofen), lorazepam (Ativan), Amitriptyline, oxybutynin, omeprazole, prochlorperazine, and diphenylhydrazine. Non-limiting examples of treatments of late stages of ALS (e.g., stages IV, V, and/or VI) can include, e.g., mechanical ventilation and/or tracheostomy, and/or an increased dose of one or more of riluzole (Rilutek), baclofen (Lioresal), a NSAID (e.g., ibuprofen), lorazepam (Ativan), Amitriptyline, oxybutynin, omeprazole, prochlorperazine, and diphenylhydrazine as compared to the dose that would be administered to a subject having an early stage of ALS.

### Methods of Monitoring a Proteopathy

Also described are methods of monitoring a proteopathy in a subject that include: (a) providing a first sample including a biological fluid obtained from a subject having a proteopathy at a first time point; (b) determining the level at least one (e.g., 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, or 44) sphingolipid in the sample of (b), wherein the at least one sphingolipid is selected from the group of: dihexosylceramide C24:1; dihexosylceramide C24:0; dihexosylceramide C23:0; dihexosylceramide C22:0; dihexosylceramide C20:0; dihexosylceramide C18:0; dihexosylceramide C16:0; lactosylceramide C24:1; lactosylceramide C24:0; lactosylceramide C23:0; lactosylceramide C22:0; lactosylceramide C20:0; lactosylceramide C18:0; lactosylceramide C16:0; ceramide C24:1; ceramide C24:0; ceramide C23:0; ceramide C22:0; ceramide C20:0; ceramide C18:0; ceramide C16:0; ceramide C14:0; globotriaosylceramide C24:1; globotriaosylceramide C24:0; globotriaosylceramide C23:0; globotriaosylceramide C22:0; globotriaosylceramide C20:0; globotriaosylceramide C18:0; globotriaosylceramide C16:0; galactosylceramide C24: 1; galactosylceramide C24:0; galactosylceramide C23:0; galactosylceramide C22:0; galactosylceramide C20:0; galactosylceramide C18:0; galactosylceramide C16:0; glucosylceramide C24:1; glucosylceramide C24:0; glucosylceramide C23:0; glucosylceramide C22:0; glucosylceramide C20:0; glucosylceramide C18:0; glucosylceramide C16:0; and glucosylsphingosine; (c) providing a second sample including a biological fluid obtained from the subject at a second time point after the first time point, and performing step (b) on the second sample; and (d) identifying the subject as having improving or static proteopathy when the level(s) is not elevated at the second time point as compared to the level(s) at the first time point. Some embodiments of these methods further include a step of enriching the first or second sample for lipids prior to detecting the level(s) of the at least one sphingolipid.

In some embodiments of these methods, steps (b) and (c) include determining the level(s) of at least one (e.g., 2, 3, 4, 5, 6, 7, 8, 9, 10, 11, 12, 13, 14, 15, 16, or 17) sphingolipid selected from the group of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24: 1, glucosylceramide C24:0, glucosylceramide C23:0, glucosylceramide C22:0, glucosylceramide C20:0, glucosylceramide C18:0, and glucosylceramide C16:0. In some embodiments of these methods, steps (b) and (c) include determining the levels of at least two (e.g., 3, 4, 5, 6, 7, 8, 9, 10, 11, 12, 13, 14, 15, 16, or 17) sphingolipids selected from the group of: dihexosylceramide C24: 1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24: 1, glucosylceramide C24:0, glucosylceramide C23:0, glucosylceramide C22:0, glucosylceramide C20:0, glucosylceramide C18:0, and glucosylceramide C16:0, and the subject is identified as having improving or static proteopathy when at least one or both of the two levels is not elevated at the second time point as compared to the first time point. In some embodiments of these methods, steps (b) and (c) include detecting the levels of at least three (e.g., 4, 5, 6, 7, 8, 9, 10, 11, 12, 13, 14, 15, 16, or 17) sphingolipids selected from the group of: dihexosylceramide C24: 1, dihexosylceramide C24:0, dihexosylceramide C20:0, ceramide C24:0, ceramide C23:0, globotriaosylceramide C24: 1, globotriaosylceramide C16:0, galactosylceramide C24:0, galactosylceramide C23:0, galactosylceramide C16:0, glucosylceramide C24: 1, glucosylceramide C24:0, glucosylceramide C23:0, glucosylceramide C22:0, glucosylceramide C20:0, glucosylceramide C18:0, and glucosylceramide C16:0, and the subject is identified as having improving or static proteopathy when at least one, at least two, or all three of the three levels is not elevated at the second time point as compared to the first time point. In some embodiments of these methods, steps (b) and (c) include detecting the levels of at least four (e.g., 5, 6, 7, 8, 9, 10, 11, 12, 13, 14, 15, 16, or 17) sphingolipids selected from the of: dihexosylceramide C24: 1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24: 1, glucosylceramide C24:0, glucosylceramide C23:0, glucosylceramide C22:0, glucosylceramide C20:0, glucosylceramide C18:0, and glucosylceramide C16:0, and the subject is identified as having improving or static proteopathy when at least one, at least two, at least three, or all four of the four levels is not elevated at the second time point as compared to the first time point.

Also described are methods of monitoring a proteopathy in a subject that include: (a) providing a first sample including a biological fluid obtained from a subject having a proteopathy at a first time point; (b) determining the level at least one (e.g., 2, 3, 4, 5, 6, or 7) sphingolipid in the sample of (b), wherein the at least one sphingolipid is selected from the group of: sphingomyelin C24: 1; sphingomyelin C24:0; sphingomyelin C23:0; sphingomyelin C22:0; sphingomyelin C20:0; sphingomyelin C18:0; and sphingomyelin C16:0; (c) providing a second sample including a biological fluid obtained from the subject at a second time point after the first time point, and performing step (b) on the second sample; and (d) identifying the subject as having improving or static proteopathy when the level(s) is elevated at the second time point as compared to the level(s) at the first time point. Some embodiments of these methods further include a step of enriching the first or second sample for lipids prior to detecting the level(s) of the at least one sphingolipid.

Also described are methods of monitoring a proteopathy in a subject that include: (a) providing a first sample including a biological fluid obtained from a subject having a proteopathy at a first time point; (b) determining at least one (e.g., 2, 3, 4, 5, or 6) of total dihexosylceramide level, total lactosylceramide level, total globotriaosylceramide level, total galactosylceramide level, total glucosylceramide level, and total phosphatidylcholine level in the sample of step (a); (c) providing a second sample including a biological fluid obtained from the subject at a second time point after the first time point, and performing step (b) on the second sample; and (d) identifying the subject as having improving or static proteopathy when at least one (e.g., 2, 3, 4, 5, or 6) of the total dihexosylceramide level, the total lactosylceramide level, the total globotriaosylceramide level, the total galactosylceramide level, the total glucosylceramide level, and the total phosphatidylcholine level is not elevated at the second time point as compared to the first time point (e.g., the level(s) is the same or substantially the same as or is not increased as compared to the level(s) at the first time point). Some embodiments of these methods further include a step of enriching the first and second samples for lipids prior to determining the level(s) of at least one of the total dihexosylceramide level, the total lactosylceramide level, the total globotriaosylceramide level, the total galactosylceramide level, the total glucosylceramide level, and the total phosphatidylcholine level.

In some embodiments of these methods, steps (b) and (c) include determining one or both of the total galactosylceramide level and the total glucosylceramide level. In some embodiments of these methods, steps (b) and (c) include determining both of the total galactosylceramide level and the total glucosylceramide level, and the subject is identified as having improving or static proteopathy when at least one or both of the levels is not elevated at the second time point as compared to the first time point. In some embodiments of these methods, steps (b) and (c) include determining both the total galactosylceramide level and the total glucosylceramide level, and the subject is identified as having improving or static proteopathy if one or both of the levels is not elevated at the second time point as compared to the first time point.

Also described are methods of monitoring a proteopathy in a subject that include: (a) providing a first sample including a biological fluid obtained from a subject having a proteopathy at a first time point; (b) determining one or both of total ceramide level and total sphingomyelin level in the sample of step (a); (c) providing a second sample including a biological fluid obtained from the subject at a second time point after the first time point, and performing step (b) on the second sample; and (d) identifying the subject as having improving or static proteopathy when one or both of the total ceramide level and the total sphingomyelin level is elevated at the second time point as compared to the first time point (e.g., the level(s) is the same or substantially the same as or is increased as compared to the level(s) at the first time point). Some embodiments of these methods further include a step of enriching the first and second samples for lipids prior to determining the level(s) of one or both of the the total ceramide level and the total sphingomyelin level.

Also described are methods of monitoring a proteopathy in a subject that include: (a) providing a first sample including a biological fluid obtained from a subject having a proteopathy at a first time point; (b) determining the ratio of glucosylceramide C24:0 to sphingomyelin C24:0 in the sample of step (a); (c) providing a second sample including a biological fluid obtained from the subject at a second time point after the first time point, and performing step (b) on the second sample; and (d) identifying the subject as having improving or static proteopathy when the ratio of glucosylceramide C24:0 to sphingomyelin C24:0 is decreased at the second time point as compared to the first time point (e.g., the level(s) is the same or substantially the same as or is increased as compared to the level(s) at the first time point). Some embodiments of these methods further include a step of enriching the first and second samples for lipids prior to determining the level(s) of one or both of the the total ceramide level and the total sphingomyelin level. In any of the methods described herein, the first sample and the second sample include blood, plasma, blood, or cerebrosprinal fluid.

Some embodiments further include after (d): (e) administering the same treatment (e.g., any of the exemplary treatments of a proteopathy described herein or known in the art) to a subject identified as having improving or static proteopathy. For example, the administering in (e) can be, e.g., administration of a glucosyl ceramide synthase inhibitor (e.g., any of the glycosyl ceramide synthase inhibitors described herein) or a recombinant enzyme (e.g., any of the recombinant enzymes described herein). Non-limiting examples of glucosyl ceramide synthase inhibitors include: (i) eliglustat; (ii) miglustat; (iii) quinuclidin-3-yl (2-(4'-fluoro-[1,1'-biphenyl]-3-yl)propan-2-yl)carbamate; (iv) (S)-quinuclidin-3-yl (2-(2-(4-fluorophenyl)thiazol-4-yl)propan-2-yl)carbamate; (v) (S)-quinuclidin-3-yl (2-(4'-(2-methoxyethoxy)-[1,1'-biphenyl]-4-yl)propan-2-yl)carbamate; and pharmaceutically acceptable salt and prodrugs thereof. A non-limiting example of a recombinant enzyme is a recombinant glucocerebrosidase (e.g., imiglucerase, velaglucerase, or taliglucerase).

Some embodiments of any of the methods further include a step of selecting a subject having a proteopathy or diagnosing a subject as having a proteopathy (prior to step (a)) (e.g., using any of the exemplary methods of diagnosing a proteopathy described herein). The subject in any of these methods can be any of the subjects described herein. Some embodiments of any of the methods further include obtaining the first and/or second samples from a subject having a proteopathy.

Some embodiments further include recording the improving or static proteopathy status of the subject in the subject's medical record (e.g., a computer readable medium). Some examples further include informing the subject, the subject's family, and/or the subject's primary care physician or attending physician of improving or static proteopathy status of the subject. Some embodiments further include authorization of a refill of a treatment administered to the subject between the first and second time points, when the subject has been identified as having improving or static proteopathy. Some embodiments include discharging a subject from an inpatient facility (e.g., hospital) based on identification of the subject as having improving or static proteopathy.

The difference in time between the first and second time points can be, e.g., between 1 week and 40 weeks, between 1 week and 30 weeks, between 1 week and 20 weeks, between 1 week and 12 weeks, between 1 week and 8 weeks, between 1 week and 4 weeks, between 1 week and 2 weeks, between 2 weeks and 12 weeks, between 2 weeks and 8 weeks, or between 2 weeks and 4 weeks.

In some embodiments of any of the methods, monitoring a proteopathy can include comparing the level(s) of the at least one sphingolipid or the at least one of total dihexosylceramide level, total lactosylceramide level, total globotriaosylceramide level, total galactosylceramide level, total glucosylceramide level, and total phosphatidylcholine level to a control level(s). The control level(s) can be, e.g., a level(s) in a sample from a subject or a population of subjects having a specific stage of a proteopathy (e.g., stage I, stage II, stage III, or stage IV of a proteopathy, e.g., any of the proteopathies described herein). In some examples, the control level(s) can be a range of level(s) that represent the level(s) of the at least one sphingolipid or the at least one of total dihexosylceramide level, total lactosylceramide level, total globotriaosylceramide level, total galactosylceramide level, total glucosylceramide level, and total phosphatidylcholine level that are present in a subject or a population of subjects determined or identified as having a specific stage of a proteopathy (e.g., stage I, stage II, stage III, or stage IV of a proteopathy).

### Methods of Selecting a Treatment for a Proteopathy for a Subject

Also described herein are methods of selecting a treatment for a proteopathy for a subject that include: (a) providing a sample including a biological fluid obtained from a subject; (b) determining the level of at least one (e.g., 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, or 44) sphingolipid in the sample of (a), wherein the at least one sphingolipid is selected from the group of: dihexosylceramide C24:1; dihexosylceramide C24:0; dihexosylceramide C23:0; dihexosylceramide C22:0; dihexosylceramide C20:0; dihexosylceramide C18:0; dihexosylceramide C16:0; lactosylceramide C24:1; lactosylceramide C24:0; lactosylceramide C23:0; lactosylceramide C22:0; lactosylceramide C20:0; lactosylceramide C18:0; lactosylceramide C16:0; ceramide C24:1; ceramide C24:0; ceramide C23:0; ceramide C22:0; ceramide C20:0; ceramide C18:0; ceramide C16:0; ceramide C14:0; globotriaosylceramide C24:1; globotriaosylceramide C24:0; globotriaosylceramide C23:0; globotriaosylceramide C22:0; globotriaosylceramide C20:0; globotriaosylceramide C18:0; globotriaosylceramide C16:0; galactosylceramide C24:1; galactosylceramide C24:0; galactosylceramide C23:0; galactosylceramide C22:0; galactosylceramide C20:0; galactosylceramide C18:0; galactosylceramide C16:0; glucosylceramide C24:1; glucosylceramide C24:0; glucosylceramide C23:0; glucosylceramide C22:0; glucosylceramide C20:0; glucosylceramide C18:0; glucosylceramide C16:0; and glucosylsphingosine; and (c) selecting a treatment for a proteopathy for a subject when the level(s) of the at least one (e.g., 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, or 44) sphingolipid is elevated as compared to a control level(s). Some embodiments further include a step of enriching the sample of (a) for lipids prior to the step of detecting the level(s) of the at least one sphingolipid.

In some embodiments of these methods, step (b) includes determining the level(s) of at least one (e.g., 2, 3, 4, 5, 6, or 7) sphingolipid selected from the group of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0. In some embodiments of these methods, step (b) includes determining the levels of at least two (e.g., 3, 4, 5, 6, or 7) sphingolipids selected from the group of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0, and a treatment for a proteopathy is selected for a subject when at least one or both of the two levels is elevated as compared to control level(s).

In some embodiments of these methods, step (b) includes detecting the levels of at least three (e.g., 4, 5, 6, or 7) sphingolipids selected from the group of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0, and a treatment for a proteopathy is selected for a subject when at least one, at least two, or all three of the three levels is elevated as compared to control level(s). In some embodiments of these methods, step (b) includes detecting the levels of at least four (e.g., 5, 6, or 7) sphingolipids selected from the group of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0, and a treatment for a proteopathy is selected for the subject when at least one, at least two, at least three, or all four of the four levels is elevated as compared to control level(s).

Also described herein are methods of selecting a treatment for a proteopathy for a subject that include: (a) providing a sample including a biological fluid obtained from a subject; (b) determining the level of at least one (e.g., 2, 3, 4, 5, 6, or 7) sphingolipid in the sample of (a), wherein the at least one sphingolipid is selected from the group of sphingomyelin C24:1; sphingomyelin C24:0; sphingomyelin C23:0; sphingomyelin C22:0; sphingomyelin C20:0; sphingomyelin C18:0; and sphingomyelin C16:0; and (c) selecting a treatment for a proteopathy for a subject when the level(s) of the at least one (e.g., 2, 3, 4, 5, 6, or 7) sphingolipid is decreased as compared to a control level(s). Some embodiments further include a step of enriching the sample of (a) for lipids prior to the step of detecting the level(s) of the at least one sphingolipid.

Also described herein are methods of selecting a treatment for a proteopathy for a subject that include: (a) providing a sample including a biological fluid obtained from a subject; (b) determining at least one (e.g., 2, 3, 4, 5, or 6) of total dihexosylceramide level, total lactosylceramide level, total globotriaosylceramide level, total galactosylceramide level, total glucosylceramide level, and total phosphatidylcholine level in the sample of step (a); and (c) selecting a treatment for a proteopathy for a subject when at least one (e.g., 2, 3, 4, 5, or 6) of the total dihexosylceramide level, the total lactosylceramide level, the total globotriaosylceramide level, the total galactosylceramide level, the total glucosylceramide level, and the total phosphatidylcholine level is elevated as compared to a control level(s). Some embodiments of these methods further include enriching the sample of (a) for lipids prior to determining the level(s) of at least one of total dihexosylceramide level, total lactosylceramide level, total globotriaosylceramide level, total galactosylceramide level, total glucosylceramide level, and total phosphatidylcholine level.

In some embodiments of these methods, step (b) includes determining one or both of the total galactosylceramide level and the total glucosylceramide level. In some embodiments of these methods, step (b) includes determining both the total galactosylceramide level and the total glucosylceramide level, and a treatment for a proteopathy is selected for the subject when one or both of the levels is increased as compared to control level(s). In some embodiments of these methods, step (b) includes determining the total galactosylceramide level and the total glucosylceramide level, and a treatment for a proteopathy is selected for the subject when both levels are increased as compared to control level(s).

Also described herein are methods of selecting a treatment for a proteopathy for a subject that include: (a) providing a sample including a biological fluid obtained from a subject; (b) determining one or both of total ceramide level and total sphingomyelin level in the sample of step (a); and (c) selecting a treatment for a proteopathy for a subject when one or both of the total ceramide level and the total sphingomyelin level is decreased as compared to a control level(s). Some embodiments of these methods further include enriching the sample of (a) for lipids prior to determining the level(s) of one or both of total ceramide level and total sphingomyelin level.

Also described herein are methods of selecting a treatment for a proteopathy for a subject that include: (a) providing a sample including a biological fluid obtained from a subject; (b) determining the ratio of glucosylceramide C24:0 to sphingomyelin C24:0 in the sample of step (a); and (c) selecting a treatment for a proteopathy for a subject when the ratio of glucosylceramide C24:0 to sphingomyelin C24:0 is increased as compared to a control level(s). Some embodiments of these methods further include enriching the sample of (a) for lipids prior to determining the ratio of glucosylceramide C24:0 to sphingomyelin C24:0.In some embodiments of any of the methods described herein, the sample includes blood, serum, plasma, or cerebrospinal fluid. Some embodiments of any of these methods further include detecting a mutation in a glucocerebrosidase (GBA) gene in a sample including genomic DNA from the subject, and further selecting a treatment for a proteopathy for a subject having a mutation in a GBA gene. Non-limiting mutations in a GBA gene can result in the expression of a GBA having, e.g., one or more of the following mutations: V15L, C16S, Δ36T, F37V, E41K, G46E, R48W, L66P, K79N, A90T, S107L, N117D, I119T, R120W, R120Q, P122S, M123V, D127V, R131C, R131L, T134I, D140H, K156Q, P159T, P159L, R170C, R170P, P178S, P182L, N188S, G189V, A190T, A190E, G195W, L197F, K198E, G202R, M3611, F213I, F216Y, T231R, E233 Stop, insertion of M between amino acids 241 and 242, S237P, F251L, H255Q, D409H, R257Q, P266A, P266R, P266L, S271N, R285H, P289L, Y304C, Y304Stop, H311R, W312C, G325R, E326K, A341T, C342G, C342Y, V352L, R353G, G355D, R359Q, S364R, S364T, S366G, N370S, L371V, V375L, G377S, D380A, G389E, G390R, N392I, V394L, N396T, F397S, V398L, V398F, D399N, P401L, I402F, I402T, L444P, A456P, D409V, D409G, K413Q, Q414R, P415R, K425E, R433G, L444R, A446P, N462K, R463C, T491I, R496C, and R496H. In some embodiments of these methods, the mutation in a GBA gene results in the expression of a GBA protein having one or more of the following mutations: S12I, insertion of SY between amino acids 13 and 14, frameshift mutation at amino acid 14, L157Q, V460M, and K416Q. In some embodiments of these methods, the mutation in a GBA gene results in the expression of a GBA protein having one or more of the following mutations: K(-27)R, 2(-4)X, G10S, V15M, C16S, D24N, G35S, S42N, T43I, R47X, R48W, R48Q, Q73X, K74X, V78A, M85T, L105R, S107L, F109V, E111K, G113E, G113A, I119S, V121A, P122L, M123T, T134P, Y135X, A136E, K157Q, K157N, I161N, I161S, H162P, R163X, Q169X, R170P, S173X, L174F, A176D, W179X, T180P, P182T, W184R, L185F, N188K, V191G, V191E, G195E, S196P, L197P, G202E, Y205C, W209R, A210V, Y212H, F213C, Y220C, E233X, E233D, G239V, G243V, Y244H, P245H, R257X, F259L, G265D, P266A, S271N, L279P, R285C, K303I, Y304X, V305L, A309V, W312R, Y313H, D315H, A318D, P319A, T323I, L324P, G325W, R329C, F331S, L336P, C342R, W348G, G349K, Q350X, R353W, S356F, R359X, Y363C, S364N, S366N, S366T, T369M, N370K, W378G, W378X, D380N, D380H, W381X, N382K, L383R, L385P, P387L, E388X, P391L, W393R, W393L, V394L, R395C, R395P, V398I, D399Y, F411I, Y412H, Q414X, M416V, F417V, Y418C, R433S, H451R, L461P, N462S, R463P, D474Y, G478S, L480P, I489T. In some embodiments of these methods, the mutation in a GBA gene includes one or more of the following insertion mutations: 84GG, 122CC, c.153-154insTACAGC, 155-156insACAGCT, D127X, 500insT, c.8410842insTGA, 1093-1094insG, 1098insA, c.1122-1123insTG, c1326insT, c.1515_1516ins AGTGAGGGCAAT, and 1562-1585ins. In some embodiments of these methods, the mutation in a GBA gene results in the expression of a GBA protein having one or more of the following deletion mutations: c.42-65del24, 72delC, 203Cdel, del205-209ACCTT, c.222-224delTAC, del255-257GCG, c.330delA, 344delA, c.533delC, 534delT, 595-596delCT, c.708delC, V214X, 793delC, 898delG, 914Cdel, c.953delT, g5255delT, L354X, c.1214delGC, 1324-1326delATT, c.1439-1445del7, 1450del2, 1447-1466del20 insTG, and c.1510delT,C,T. In some embodiments of these methods, the mutation in a GBA gene is one or more of the following splice junction mutations: IVS2 +1G>A, IVS2+1G>T, IVS4 +1G>A, IVS5 +1G>T, g.4252C>G, g.4426A>G, IVS6-1G>C, g.5230G>A, IVS8+1, IVS8(-11delC)(-14T>A), IVS9-3C>G, IVS10-1G>A R463Q, IVS10+2T>A, and IVS10(+2). In some embodiments of these methods, the mutation in a GBA gene includes a IVS2+1 mutation. In some embodiments of these methods, the mutation in a GBA gene results in the expression of a GBA protein having one or more of the following mutations: c.(-203)A>G + IVS4-2a>g, S448P, c.1379G>A c.1469A>G, g.7319T>C + g.7741T>C, c.203-204insC, RecTL. In some embodiments of these methods, the mutation in a GBA gene results in the expression of a GBA protein having a Rec1 mutation (L444P, A456P, and V460M). Additional examples of mutations in a GBA gene and methods of detecting mutations in a GBA gene are described in, e.g., Beutler et al., Blood Cells, Molecules, and Diseases 35 (2005) 355-364; Gan-Or et al., Neurology 70(24):2277-83, 2008; Hruska et al., Human Mutation 29(5), 567-583, 2008; and Tsuang D., et al., Neurology 6; 79(19): 1944-1950, 2012.

Additional exemplary mutations in a GBA gene and exemplary methods for detecting a mutation in a GBA gene are described, e.g., in Hruska et al., Human Mutation 29(5):567-583, 2008; and Beautler et al., Blood Cells, Molecules, and Diseases 35:355-364, 2005. Non-limiting examples of methods for detection a mutation in a GBA gene include the techniques of restriction fragment length polymorphism (RFLP); amplification refractory mutation system (ARMS) PCR; allele-specific amplification (ASA); multiplex PCR; nested PCR; reverse transcriptase (RT) PCR; real-time PCR; multiplex ligation-dependent probe amplification (MLPA); denaturing gradient gel electrophoresis (DGGE); denaturing high-performance liquid chromatography (DHPLC); temperature gradient gel electrophoresis (TGGE); single strand conformational polymorphism (SSCP); heteroduplex analysis (HET); single nucleotide primer extension (i.e., mini sequencing); chemical cleavage of mismatch (CCM); TaqMan and molecular beacons; enzyme mismatch cleavage (EMC); protein truncation test (PTT); oligonucleotide ligation assay (OLA); fluorescence in situ hybridization (FISH); cleavage fragment length polymorphism (CFLP); mismatch binding proteins (e.g., MutS); allele-specific oligonucleotide (ASO) hybridization; differential reactivity with sodium bisulfite; base excision sequencing scanning (BESS); and ribonuclease mismatch cleavage (e.g., NIRCA). Additional methods for detecting a mutation in a GBA gene are described in, e.g., Mahdieh et al., Iran J. Pediatr. 23(4):375-388, 2013.

Some embodiments of any of the methods described herein further include detecting a level of one or more (e.g., 2, 3, 4, 5, or 6) of acid beta-glucocerebrosidase, acid sphingomyelinase, acid alpha-glucosidase, galactocerebrosidase, alpha-galactosidase A, and alpha-L-iduronidase, and further selecting a treatment for a proteopathy for a subject having a decrease in the level of at least one (e.g., 2, 3, 4, 5, or 6) of acid beta-glucocerebrosidase, acid sphingomyelinase, acid alpha-glucosidase, galactocerebrosidase, alpha-galactosidase A, and alpha-L-iduronidase, as compared to control level(s). Exemplary methods for detecting the levels of acid beta-glucocerebrosidase, acid sphingomyelinase, acid alpha-glucosidase, galactocerebrosidase, alpha-galactosidase A, and alpha-L-iduronidase in a sample including a biological fluid are described in, e.g., U.S. Patent Application Publication No. 2008/0248513 and WO 13/070953. Kits for detecting the level of acid beta-glucocerebrosidase, acid sphingomyelinase, acid alpha-glucosidase, galactocerebrosidase, alpha-galactosidase A, and alpha-L-iduronidase in a sample including a biological fluid are also commercially available.

The methods described herein may further include after step (c): (d) administering the selected treatment to the subject. In some examples, the selected treatment is a glucosyl ceramide synthase inhibitor (e.g., any of the glucosyl ceramide synthase inhibitors described herein or known in the art) or a recombinant enzyme (e.g., a recombinant glucocerebrosidase, e.g., imiglucerase, velaglucerase, or taliglucerase). In some embodiments of these methods, the selected treatment is a glucosyl ceramide synthase inhibitor selected from the group of (i) eliglustat; (ii) miglustat; (iii) quinuclidin-3-yl (2-(4'-fluoro-[1,1'-biphenyl]-3-yl)propan-2-yl)carbamate; (iv) (S)-quinuclidin-3-yl (2-(2-(4-fluorophenyl)thiazol-4-yl)propan-2-yl)carbamate; (v) (S)-quinuclidin-3-yl (2-(4'-(2-methoxyethoxy)-[1,1'-biphenyl]-4-yl)propan-2-yl)carbamate; and the pharmaceutically acceptable salts and prodrugs thereof.

In any of these methods, a control level(s) can be, e.g., a level(s) in a subject not presenting with one or more symptoms of a proteopathy and/or not diagnosed as having a proteopathy, a level(s) in a subject that has no history of a proteopathy, a level(s) in a healthy subject or a population of healthy subjects, or a threshold level(s) (e.g., a level(s) above which indicates that the subject has a proteopathy or an increased risk of developing a proteopathy). The control level(s) can be, e.g., a level(s) in a sample from a subject or a population of subjects that has/have no history of a proteopathy and, optionally, also does/do not have a genetically-related family member diagnosed or identified as having a proteopathy. The control level(s) can be, e.g., a threshold level(s) that is indicative of a subject having a proteopathy or a subject having an increased risk of developing a proteopathy when the measured level is above the threshold level. Additional exemplary control level(s) of any of the sphingolipid(s) are described herein.

### Methods of Selecting a Subject for a Clinical Trial

Also described are methods of selecting a subject for a clinical trial that includes the administration of a treatment for a proteopathy that includes: (a) providing a sample including a biological fluid obtained from a subject; (b) determining the level of at least one (e.g., 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, or 44) sphingolipid in the sample of (a), wherein the at least one sphingolipid is selected from the group of: dihexosylceramide C24:1; dihexosylceramide C24:0; dihexosylceramide C23:0; dihexosylceramide C22:0; dihexosylceramide C20:0; dihexosylceramide C18:0; dihexosylceramide C16:0; lactosylceramide C24:1; lactosylceramide C24:0; lactosylceramide C23:0; lactosylceramide C22:0; lactosylceramide C20:0; lactosylceramide C18:0; lactosylceramide C16:0; ceramide C24:1; ceramide C24:0; ceramide C23:0; ceramide C22:0; ceramide C20:0; ceramide C18:0; ceramide C16:0; ceramide C14:0; globotriaosylceramide C24:1; globotriaosylceramide C24:0; globotriaosylceramide C23:0; globotriaosylceramide C22:0; globotriaosylceramide C20:0; globotriaosylceramide C18:0; globotriaosylceramide C16:0; galactosylceramide C24:1; galactosylceramide C24:0; galactosylceramide C23:0; galactosylceramide C22:0; galactosylceramide C20:0; galactosylceramide C18:0; galactosylceramide C16:0; glucosylceramide C24:1; glucosylceramide C24:0; glucosylceramide C23:0; glucosylceramide C22:0; glucosylceramide C20:0; glucosylceramide C18:0; glucosylceramide C16:0; and glucosylsphingosine; and (c) selecting a subject for participation in the clinical trial when the level(s) of the at least one (e.g., 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44) sphingolipid is elevated as compared to a control level(s). Some embodiments of these methods further include a step of enriching the sample of (a) for lipids prior to the step of determining the level(s) of the at least one sphingolipid.

In some embodiments of these methods, step (b) includes determining a level(s) of at least one (e.g., 2, 3, 4, 5, 6, 7, 8, 9, 10, 11, 12, 13, 14, 15, 16, or 17) sphingolipid selected from the group of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0. In some embodiments of these methods, step (b) includes determining the levels of at least two (e.g., 3, 4, 5, 6, 7, 8, 9, 10, 11, 12, 13, 14, 15, 16, or 17) sphingolipids selected from the group of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0, and the subject is selected for participation in the clinical trial when at least one or both of the two levels is elevated as compared to control level(s).

In some embodiments of these methods, step (b) includes detecting the levels of at least three (e.g., 4, 5, 6, 7, 8, 9, 10, 11, 12, 13, 14, 15, 16, or 17) sphingolipids selected from the group of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylceramide C16:0, and the subject is selected for participation in the clinical trial when at least one, at least two, or all three of the three levels is elevated as compared to control level(s). In some embodiments of these methods, step (c) includes detecting the levels of at least four (e.g., 5, 6, 7, 8, 9, 10, 11, 12, 13, 14, 15, 16, or 17) sphingolipids selected from the group of: dihexosylceramide C24:1, dihexosylceramide C24:0, dihexosylceramide C20:0, ceramide C24:0, ceramide C23:0, globotriaosylceramide C24:1, globotriaosylceramide C16:0, galactosylceramide C24:0, galactosylceramide C23:0, galactosylceramide C16:0, glucosylceramide C24:1, glucosylceramide C24:0, glucosylceramide C23:0, glucosylceramide C22:0 glucosylceramide C20:0, glucosylceramide C18:0, and glucosylceramide C16:0, and the subject is selected for participation in the clinical trial when at least one, at least two, at least three, or all four of the four levels is elevated as compared to control level(s).

Also described are methods of selecting a subject for a clinical trial that includes the administration of a treatment for a proteopathy that includes: (a) providing a sample including a biological fluid obtained from a subject; (b) determining the level of at least one (e.g., 2, 3, 4, 5, 6, or 7) sphingolipid in the sample of (a), wherein the at least one sphingolipid is selected from the group of: sphingomyelin C24:1; sphingomyelin C24:0; sphingomyelin C23:0; sphingomyelin C22:0; sphingomyelin C20:0; sphingomyelin C18:0; and sphingomyelin C16:0; and (c) selecting a subject for participation in the clinical trial when the level(s) of the at least one (e.g., 2, 3, 4, 5, 6, or 7) sphingolipid is decreased as compared to a control level(s). Some embodiments of these methods further include a step of enriching the sample of (a) for lipids prior to the step of determining the level(s) of the at least one sphingolipid.

Also described are methods of selecting a subject for a clinical trial that includes the administration of a treatment for a proteopathy that include: (a) providing a sample including a biological fluid obtained from a subject; (b) determining at least one (e.g., 2, 3, 4, 5, or 6) of total dihexosylceramide level, total lactosylceramide level, total globotriaosylceramide level, total galactosylceramide level, total glucosylceramide level, and total phosphatidylcholine level in the sample of step (a); (c) selecting a subject for participation in the clinical trial when at least one (e.g., 2, 3, 4, 5, or 6) of the total dihexosylceramide level, the total lactosylceramide level, the total globotriaosylceramide level, the total galactosylceramide level, the total glucosylceramide level, and the total phosphatidylcholine level is elevated as compared to a control level(s). Some embodiments of these methods further include a step of enriching the sample of (a) for lipids before the step of detecting the level(s) of at least one of the total dihexosylceramide level, the total lactosylceramide level, the total globotriaosylceramide level, the total galactosylceramide level, the total glucosylceramide level, and the total phosphatidylcholine level.

In some embodiments of these methods, step (b) includes determining one or both of the total galactosylceramide level and the total glucosylceramide level. In some embodiments of these methods, step (b) includes determining both the total galactosylceramide level and the total glucosylceramide level, and the subject is selected for participation in the clinical trial when one or both of the levels is increased as compared to control level(s).

In some embodiments of these methods, step (b) includes determining both of the total galactosylceramide leveland the total glucosylceramide level, and the subject is selected for participation in the clinical trial when one or both of the levels is increased as compared to control level(s). In some embodiments of these methods, step (b) includes determining both the total galactosylceramide level and the total glucosylceramide level, and the subject is selected for participation in the clinical trial when both of the levels is increased as compared to control level(s).

Also described are methods of selecting a subject for a clinical trial that includes the administration of a treatment for a proteopathy that include: (a) providing a sample including a biological fluid obtained from a subject; (b) determining one or both of total ceramide level and total sphingomyelin level in the sample of step (a); (c) selecting a subject for participation in the clinical trial when one or both of the total ceramide level and the total sphingomyelin level is decreased as compared to a control level(s). Some embodiments of these methods further include a step of enriching the sample of (a) for lipids before the step of detecting the level(s) of one or both of the total ceramide level and the total sphingomyelin level.

Also described are methods of selecting a subject for a clinical trial that includes the administration of a treatment for a proteopathy that include: (a) providing a sample including a biological fluid obtained from a subject; (b) determining the ratio of glucosylceramide C24:0 to sphingomyelin C24:0 in the sample of step (a); (c) selecting a subject for participation in the clinical trial when the ratio of glucosylceramide C24:0 to sphingomyelin C24:0 is increased as compared to a control level(s). Some embodiments of these methods further include a step of enriching the sample of (a) for lipids before the step of determining the ratio of glucosylceramide C24:0 to sphingomyelin C24:0.In some embodiments of any of the methods described herein, the sample includes blood, serum, plasma, or cerebrospinal fluid. Some embodiments further include detecting a mutation in a glucocerebrosidase (GBA) gene in a sample including genomic DNA from the subject, and further selecting a subject having a mutation in a GBA gene for participation in the clinical trial. Non-limiting mutations in a GBA gene can result in the expression of a GBA having, e.g., one or more of the following mutations: V15L, C16S, Δ36T, F37V, E41K, G46E, R48W, L66P, K79N, A90T, S107L, N117D, I119T, R120W, R120Q, P122S, M123V, D127V, R131C, R131L, T134I, D140H, K156Q, P159T, P159L, R170C, R170P, P178S, P182L, N188S, G189V, A190T, A190E, G195W, L197F, K198E, G202R, M361I, F213I, F216Y, T231R, E233 Stop, insertion of M between amino acids 241 and 242, S237P, F251L, H255Q, D409H, R257Q, P266A, P266R, P266L, S271N, R285H, P289L, Y304C, Y304Stop, H311R, W312C, G325R, E326K, A341T, C342G, C342Y, V352L, R353G, G355D, R359Q, S364R, S364T, S366G, N370S, L371V, V375L, G377S, D380A, G389E, G390R, N392I, V394L, N396T, F397S, V398L, V398F, D399N, P401L, I402F, I402T, L444P, A456P, D409V, D409G, K413Q, Q414R, P415R, K425E, R433G, L444R, A446P, N462K, R463C, T491I, R496C, and R496H. In some embodiments of these methods, the mutation in a GBA gene results in the expression of a GBA protein having one or more of the following mutations: S12I, insertion of SY between amino acids 13 and 14, frameshift mutation at amino acid 14, L157Q, V460M, and K416Q. In some embodiments of these methods, the mutation in a GBA gene results in the expression of a GBA protein having one or more of the following mutations: K(-27)R, 2(-4)X, G10S, V15M, C16S, D24N, G35S, S42N, T43I, R47X, R48W, R48Q, Q73X, K74X, V78A, M85T, L105R, S107L, F109V, E111K, G113E, G113A, I119S, V121A, P122L, M123T, T134P, Y135X, A136E, K157Q, K157N, I161N, I161S, H162P, R163X, Q169X, R170P, S173X, L174F, A176D, W179X, T180P, P182T, W184R, L185F, N188K, V191G, V191E, G195E, S196P, L197P, G202E, Y205C, W209R, A210V, Y212H, F213C, Y220C, E233X, E233D, G239V, G243V, Y244H, P245H, R257X, F259L, G265D, P266A, S271N, L279P, R285C, K303I, Y304X, V305L, A309V, W312R, Y313H, D315H, A318D, P319A, T323I, L324P, G325W, R329C, F331S, L336P, C342R, W348G, G349K, Q350X, R353W, S356F, R359X, Y363C, S364N, S366N, S366T, T369M, N370K, W378G, W378X, D380N, D380H, W381X, N382K, L383R, L385P, P387L, E388X, P391L, W393R, W393L, V394L, R395C, R395P, V398I, D399Y, F411I, Y412H, Q414X, M416V, F417V, Y418C, R433S, H451R, L461P, N462S, R463P, D474Y, G478S, L480P, I489T. In some embodiments of these methods, the mutation in a GBA gene includes one or more of the following insertion mutations: 84GG, 122CC, c.153-154insTACAGC, 155-156insACAGCT, D127X, 500insT, c.8410842insTGA, 1093-1094insG, 1098insA, c.1122-1123insTG, c1326insT, c.1515_1516ins AGTGAGGGCAAT, and 1562-1585ins. In some embodiments of these methods, the mutation in a GBA gene results in the expression of a GBA protein having one or more of the following deletion mutations: c.42-65del24, 72delC, 203Cdel, del205-209ACCTT, c.222-224delTAC, del255-257GCG, c.330delA, 344delA, c.533delC, 534delT, 595-596delCT, c.708delC, V214X, 793delC, 898delG, 914Cdel, c.953delT, g5255delT, L354X, c.1214delGC, 1324-1326delATT, c.1439-1445del7, 1450del2, 1447-1466del20 insTG, and c.1510delT,C,T. In some embodiments of these methods, the mutation in a GBA gene is one or more of the following splice junction mutations: IVS2 +1G>A, IVS2+1G>T, IVS4 +1G>A, IVS5 +1G>T, g.4252C>G, g.4426A>G, IVS6-1G>C, g.5230G>A, IVS8+1, IVS8(-11delC)(-14T>A), IVS9-3C>G, IVS10-1G>A R463Q, IVS10+2T>A, and IVS10(+2). In some embodiments of these methods, the mutation in a GBA gene includes a IVS2+1 mutation. In some embodiments of these methods, the mutation in a GBA gene results in the expression of a GBA protein having one or more of the following mutations: c.(-203)A>G + IVS4-2a>g, S448P, c.1379G>A c.1469A>G, g.7319T>C + g.7741T>C, c.203-204insC, RecTL. In some embodiments of these methods, the mutation in a GBA gene results in the expression of a GBA protein having a Rec1 mutation (L444P, A456P, and V460M). Additional examples of mutations in a GBA gene and methods of detecting mutations in a GBA gene are described in, e.g., Beutler et al., Blood Cells, Molecules, and Diseases 35 (2005) 355-364; Gan-Or et al., Neurology 70(24):2277-83, 2008; Hruska et al., Human Mutation 29(5), 567-583, 2008; and Tsuang D., et al., Neurology 6; 79(19): 1944-1950, 2012.

Additional exemplary mutations in a GBA gene and exemplary methods for detecting a mutation in a GBA gene are described, e.g., in Hruska et al., Human Mutation 29(5):567-583, 2008; and Beautler et al., Blood Cells, Molecules, and Diseases 35:355-364, 2005. Non-limiting examples of methods for detection a mutation in a GBA gene include the techniques of restriction fragment length polymorphism (RFLP); amplification refractory mutation system (ARMS) PCR; allele-specific amplification (ASA); multiplex PCR; nested PCR; reverse transcriptase (RT) PCR; real-time PCR; multiplex ligation-dependent probe amplification (MLPA); denaturing gradient gel electrophoresis (DGGE); denaturing high-performance liquid chromatography (DHPLC); temperature gradient gel electrophoresis (TGGE); single strand conformational polymorphism (SSCP); heteroduplex analysis (HET); single nucleotide primer extension (i.e., mini sequencing); chemical cleavage of mismatch (CCM); TaqMan and molecular beacons; enzyme mismatch cleavage (EMC); protein truncation test (PTT); oligonucleotide ligation assay (OLA); fluorescence in situ hybridization (FISH); cleavage fragment length polymorphism (CFLP); mismatch binding proteins (e.g., MutS); allele-specific oligonucleotide (ASO) hybridization; differential reactivity with sodium bisulfite; base excision sequencing scanning (BESS); and ribonuclease mismatch cleavage (e.g., NIRCA). Additional methods for detecting a mutation in a GBA gene are described in, e.g., Mahdieh et al., Iran J. Pediatr. 23(4):375-388, 2013.

Some embodiments of any of the methods described herein further include detecting a level of one or more (e.g., 2, 3, 4, 5, or 6) of acid beta-glucocerebrosidase, acid sphingomyelinase, acid alpha-glucosidase, galactocerebrosidase, alpha-galactosidase A, and alpha-L-iduronidase, and further selecting a subject having a decrease in the level of at least one (e.g., 2, 3, 4, 5, or 6) of acid beta-glucocerebrosidase, acid sphingomyelinase, acid alpha-glucosidase, galactocerebrosidase, alpha-galactosidase A, and alpha-L-iduronidase, as compared to control level(s), for participation in the clinical trial. Exemplary methods for detecting the levels of acid beta-glucocerebrosidase, acid sphingomyelinase, acid alpha-glucosidase, galactocerebrosidase, alpha-galactosidase A, and alpha-L-iduronidase in a sample including a biological fluid are described in, e.g., U.S. Patent Application Publication No. 2008/0248513 and WO 13/070953. Kits for detecting the level of acid beta-glucocerebrosidase, acid sphingomyelinase, acid alpha-glucosidase, galactocerebrosidase, alpha-galactosidase A, and alpha-L-iduronidase in a sample including a biological fluid are also commercially available.

In the methods described herein, the treatment for a proteopathy may be administering a glucosyl ceramide synthase inhibitor (e.g., any of the glucosyl ceramide synthase inhibitors described herein or known in the art) or a recombinant enzyme (e.g., a recombinant glucocerebrosidase, e.g., imiglucerase, velaglucerase, or taliglucerase). In some embodiment sof any of the methods described herein, the treatment for a proteopathy is administering a glucosyl ceramide synthase inhibitor selected from the group of: (i) eliglustat; (ii) miglustat; (iii) quinuclidin-3-yl (2-(4'-fluoro-[1,1'-biphenyl]-3-yl)propan-2-yl)carbamate; (iv) (S)-quinuclidin-3-yl (2-(2-(4-fluorophenyl)thiazol-4-yl)propan-2-yl)carbamate; (v) (S)-quinuclidin-3-yl (2-(4'-(2-methoxyethoxy)-[1,1'-biphenyl]-4-yl)propan-2-yl)carbamate; and the pharmaceutically acceptable salts and prodrugs thereof.

In any of these methods, a control level(s) can be, e.g., a level(s) in a subject not presenting with one or more symptoms of a proteopathy and/or not diagnosed as having a proteopathy, a level(s) in a subject that has no history of a proteopathy, a level(s) in a healthy subject or a population of healthy subjects, or a threshold level(s) (e.g., a level(s) above which indicates that the subject has a proteopathy or an increased risk of developing a proteopathy). The control level(s) can be, e.g., a level(s) in a sample from a subject or a population of subjects that has/have no history of a proteopathy and, optionally, also does/do not have a genetically-related family member diagnosed or identified as having a proteopathy. The control level(s) can be, e.g., a threshold level(s) that is indicative of a subject having a proteopathy or a subject having an increased risk of developing a proteopathy when the measured level is above the threshold level. Additional exemplary control level(s) of any of the sphingolipid(s) are described herein.

### Kits

Also described herein are kits that include one or more organic solvents (e.g., any of the organic solvents described herein) for use in enriching a sample for lipids (e.g., for use in the step of extracting lipids from a sample including a biological fluid from a subject). Some examples of the kits can optionally further include a control sample including a predetermined concentration of one or more phospholipids (for use as a control in LC-MS-MS analysis). Some examples of the kits can also optionally further include a sample from a subject having a proteinopathy. Some examples of the kits can further include instructions for performing any of the methods described herein.

The control sample described herein can be a sample including any of the control level(s) of the at least one sphingolipid or the at least one of total dihexosylceramide level, total lactosylceramide level, total ceramide level, total globotriaosylceramide level, total sphingomyelin level, total galactosylceramide level, total glucosylceramide level, and total phosphatidylcholine level described herein.

The disclosure is further described in the following examples, which do not limit the scope of the invention described in the claims. The following Examples may relate to aspects of the present disclosure which are not claimed. It will be appreciated that these examples may be useful for understanding the invention and are included for reference purposes.

### EXAMPLES

### Example 1. Detection of Sphingolipids in Samples from Subjects Having a Proteopathy

A set of experiments was performed to determine the level of sphingolipids in samples from healthy subjects and subjects having Parkinson's disease.

### Materials and Methods

### Blood Collection and Plasma Processing

Peripheral venous blood was collected into purple top EDTA-BD Vacutainer tubes (BD Franklin Lakes, NJ) using standard phlebotomy procedures. Tubes were centrifuged at room temperature (25 °C) in order to avoid platelet activation through cooling, at 2,000 rcf for 5 minutes. In order to reduce freeze-thaw cycles, immediately after centrifugation, plasma was aliquoted into 500-µL aliquots into low-retention in 1.5 mL-tubes (Fisher Scientific, CA) using low-retention pipette tips. Aliquots were bar-coded, electronically tracked, and stored ready-to-use at -80° within 4 hours of blood draw. Time of blood draw, time of last meal prior to phlebotomy, time to centrifugation, and time to freezing were monitored for quality-control purposes for all samples.

### Lumbar Puncture Collection

Lumbar puncture (LP) was performed using the atraumatic technique. Cerebrospinal fluid (CSF) is collected into two 10 mL-syringes at room temperature, the 20 mL were transferred into one 50 mL-Falcon tube, mixed gently by inverting 3-4 times. CSF is immediately centrifuged at 400 g for 10 min at room temperature, immediately aliquoted into pre-cooled, siliconized polypropylene aliquot tubes and immediately frozen on dry ice and then stored frozen at -80 within two hours of LP.

### Lipid Detection

The following lipids were detected in the biofluid (e.g. plasma, serum, cerebrospinal fluid, urine, etc.).

| **Abbreviation** | **Name** |
|---|---|
| TotalDiHexCer | Total Dihexosylceramide |
| DiHexCer_C24.1 | Dihexosylceramide C24:1 |
| DiHexCer_C24 | Dihexosylceramide C24:0 |
| DiHexCer_C23 | Dihexosylceramide C23:0 |
| DiHexCer_C20 | Dihexosylceramide C22:0 |
| DiHexCer_C18 | Dihexosylceramide C20:0 |
| DiHexCer_C16 | Dihexosylceramide C18:0 |
| DiHexCe_C22 | Dihexosylceramide C16:0 |
| TotalGL2 | Total Lactosylceramide |
| GL2_C24.1 | Lactosylceramide C24:1 |
| GL2_C24 | Lactosylceramide C24:0 |
| GL2_C23 | Lactosylceramide C23:0 |
| GL2_C22 | Lactosylceramide C22:0 |
| GL2_C20 | Lactosylceramide C20:0 |
| GL2_C18 | Lactosylceramide C18:0 |
| GL2_C16 | Lactosylceramide C16:0 |
| TotalCer | Total Ceramide |
| Cer_C24.1 | Ceramide C24:1 |
| Cer_C24 | Ceramide C24:0 |
| Cer_C23 | Ceramide C23:0 |
| Cer_C22 | Ceramide C22:0 |
| Cer_C20 | Ceramide C20:0 |
| Cer_C18 | Ceramide C18:0 |
| Cer_C16 | Ceramide C16:0 |
| Cer_C14 | Ceramide C14:0 |
| TotalGL3 | Total Globotriaosylceramide |
| GL3_C24.1 | Globotriaosylceramide C24:1 |
| GL3_C24 | Globotriaosylceramide C24:0 |
| GL3_C23 | Globotriaosylceramide C23:0 |
| GL3_C22 | Globotriaosylceramide C22:0 |
| GL3_C20 | Globotriaosylceramide C20:0 |
| GL3_C18 | Globotriaosylceramide C18:0 |
| GL3_C16 | Globotriaosylceramide C16:0 |
| TotalSM | Total Sphingomyelin |
| SM_C24.1 | Sphingomyelin C24:1 |
| SM_C24 | Sphingomyelin C24:0 |
| SM_C23 | Sphingomyelin C23:0 |
| SM_C22 | Sphingomyelin C22:0 |
| SM_C20 | Sphingomyelin C20:0 |
| SM_C18 | Sphingomyelin C18:0 |
| SM_C16 | Sphingomyelin C16:0 |
| TotalGalCer | Total Galactosylceramide |
| GalCer_C24.1 | Galactosylceramide C24:1 |
| GalCer_C24 | Galactosylceramide C24:0 |
| GalCer_C23 | Galactosylceramide C23:0 |
| GalCer_C22 | Galactosylceramide C22:0 |
| GalCer_C20 | Galactosylceramide C20:0 |
| GalCer_C18 | Galactosylceramide C18:0 |
| GalCer_C16 | Galactosylceramide C16:0 |
| TotalGL1 | Total Glucosylceramide |
| GL1_C24.1 | Glucosylceramide C24:1 |
| GL1_C24 | Glucosylceramide C24:0 |
| GL1_C23 | Glucosylceramide C23:0 |
| GL1_C22 | Glucosylceramide C22:0 |
| GL1_C20 | Glucosylceramide C20:0 |
| GL1_C18 | Glucosylceramide C18:0 |
| GL1_C16 | Glucosylceramide C16:0 |
| Lyso_GL1 | Glucosylsphingosine |
| TotalPC | Total Phosphatidilcholine |

### LC/MS/MS

Quantitative analysis of sphingolipids was performed by liquid chromatography and tandem mass spectrometry (LC/MS/MS). Briefly, various aliquots of human plasma or CSF were extracted with 1 mL of an organic solvent mixture as follows:
For glucosylceramide (GL1) analysis, 10 µl of homogenate was extracted with 1 ml of 90% mobile phase A (MPA) and 10% mobile phase B (MPB). MPA is consisted of 96:2:1:1 acetonitrile/methanol/acetic acid/water (v/v/v/v) and MPB of 98:1:1 methanol/acetic acid/water (v/v/v); both contained 5 mM ammonium acetate. The samples were placed on a VX-2500 tube vortexer (VWR International, LLC) for 5 min and then centrifuged for 4 min at 8, 400 rpm (Beckman Coulter, Inc.). The resultant supernatant was transferred into HPLC vials for analysis. GL1 and galactosylceramide (GalCer) were separated using a Waters Acquity UPLC and Atlantis HILIC Silica column (2.1 mm x 150 mm, 3 µm particles, Waters Corp., Milford, MA) and analyzed by an API 5000 triple quadrupole mass spectrometer in MRM mode (Applied Biosystems, Foster City, CA).

For LysoGL1 analysis, 30 µl of human plasma were extracted with 1 ml of 50% of MPA and 50% of MPB. After extraction, the supernatant was transferred into HPLC vials. LysoGL1 and psychosine were separated using an Agilent 1290 Infinity LC system and a Waters BEH HILIC column (2.1 mm x 100 mm, 1.7 µm particles), and analyzed by an Agilent 6490 triple quadrupole mass spectrometer in MRM mode (Agilent Technologies, Santa Clara, CA). The mobile phases used for lysoGL1 and psychosine separation consisted of 95:5 acetonitrile/200mM ammonium acetate (v/v) and 95:5 methanol/200mM ammonium acetate (v/v) at pH 9.0.

For Lactosylceramide (GL2), Dihexosylceramide (DiHexCer), and Trihexosylceramide (GL3) analyses, 30 µl of human plasma were extracted with 1 ml of 50% of MPA and 50% of MPB. After extraction, the supernatant was transferred into HPLC vials. GL2 and DiHexCer were separated using a Waters Acquity UPLC and BEH HILIC column (2.1 mm x 100 mm, 1.7 µm particles, Waters Corp., Milford, MA) and analyzed by an API 5000 triple quadrupole mass spectrometer in MRM mode (Applied Biosystems, Foster City, CA). GL3 was also analyzed using a Waters Acquity UPLC and BEH HILIC column (2.1 mm x 100 mm, 1.7 µm particles, Waters Corp., Milford, MA) and analyzed by an API 5000 triple quadrupole mass spectrometer in MRM mode (Applied Biosystems, Foster City, CA).

For Phosphatidylcholine (PC) and Sphingomyelin (SM) analysis, 10 µl of extract for GL1 analysis was diluted into 600 µl of MPA and analyzed using a Waters Acquity UPLC (Waters Corp., Milford, MA) and Luna HILIC column (2.0 mm x 100 mm, 3 µm particles, Phenomenex, Torrance, CA) and analyzed by an API 4000 triple quadrupole mass spectrometer in precursor mode (Applied Biosystems, Foster City, CA).

For Ceramide (Cer) analysis, 30 µl of human plasma were extracted with 1 ml of 85:10:5 = methanol:acetonitrile:water (v/v/v). After extraction, the supernatant was transferred into HPLC vials. Cer was analyzed using a Waters Acquity UPLC and BEH C8 column (2.1 mm x 100 mm, 1.7 µm particles, Waters Corp., Milford, MA) and analyzed by an API 5000 triple quadrupole mass spectrometer in MRM mode (Applied Biosystems, Foster City, CA).

GL1, GalCer, GL2, and GL3 standards were purchased from Matreya, LLC (Pleasant Gap, PA) and lysoGL1, psychosine, and Cer standards were purchased from Avanti Polar Lipids, Inc. (Alabaster, AL).

### Results

The abundance of these lipids were individually and comparatively analyzed, e.g., 1) levels of one of the sphingolipids alone; or 2) in combination with levels of other sphingolipids; and/or 3) in combination with the activity of the enzymes responsible for the synthesizing of hydrolyzing sphingolipids measured in biofluids; and/or 4) presence of mutations in the glucocerebrosidase *GBA* gene.

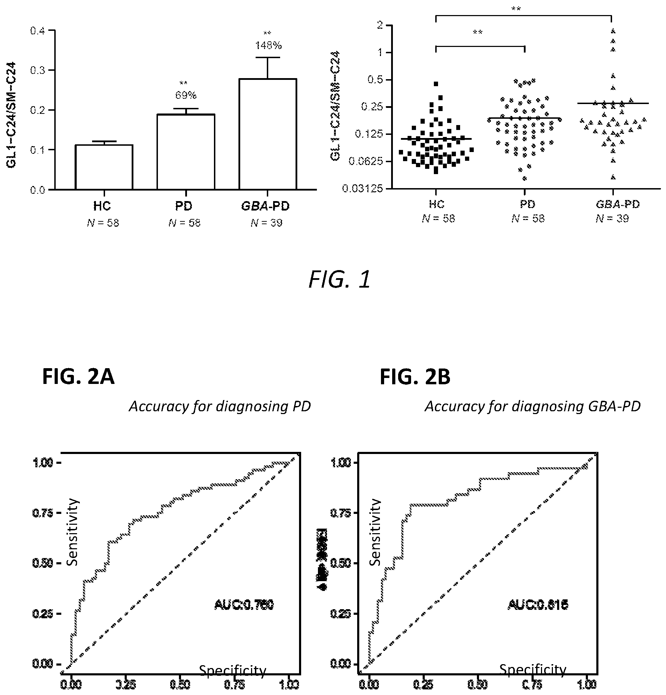

As seen in FIG. 1, the combination of glucosylceramide and sphingomyelin levels in the biofluids provided a biomarker that distinguished PD samples from healthy control samples (HC). Additionally the combination provided a biomarker to distinguish diseases associated with a mutation in the *GBA* gene (GBA-PD) from both PD and healthy control samples (FIG. 1).

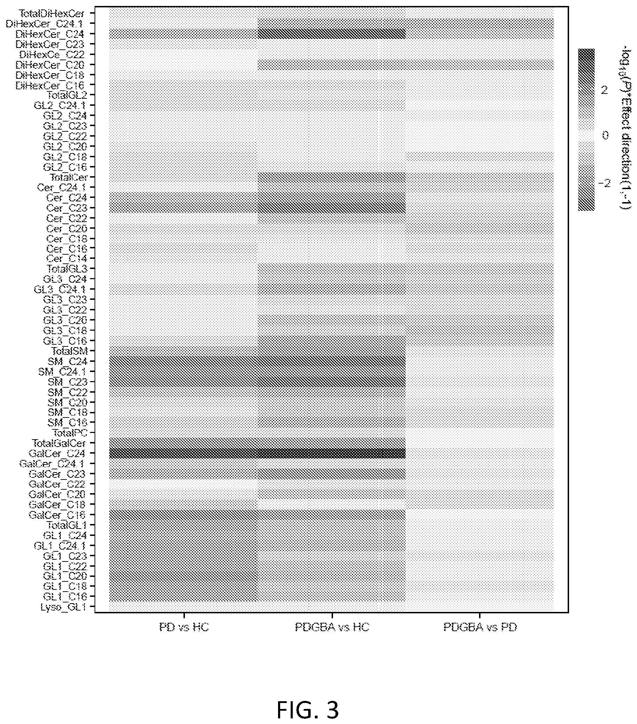

The diagnostic accuracy of using sphingolipid panels for diagnosing PD and also for diagnosing PD carrying a *GBA* mutation is shown in FIG. 2. The heatmap shown in FIG. 3 illustrates the both simple and complex patterns of combinations of these sphingolipids. These patterns can be used as "molecular barcodes" to distinguish patients with PD and patients with PD with a *GBA* mutation (GBA-PD) from healthy controls (HC). (Abbreviations for FIG. 3 indicated above).

### Example 2. Levels of Specific Sphingolipids in Samples from Healthy and Parkinson's Disease Patients

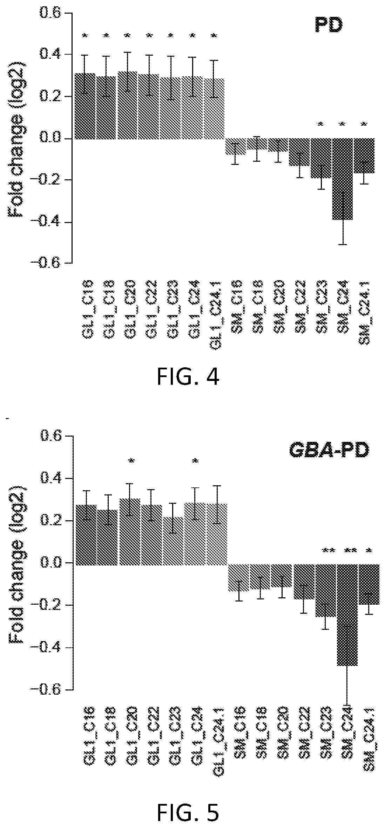

The levels of specific sphingolipid isoforms were determined in plasma samples from healthy controls and sporadic Parkinson's disease patients and Parkinson's disease patients having a mutation in GBA as described in Example 1. The data show that the levels of several specific sphingolipid isoforms differ (are elevated or decreased) in both patients having sporadic Parkinson's disease and patients having Parkinson's disease that carry a GBA mutation, as compared to healthy controls (Figures 4 and 5). It is noted that specific sphingomyelins are decreased in sporadic Parkinson's disease patients and Parkinson's disease patients carrying a GBA mutation as compared to healthy controls (Figures 4 and 5, respectively).

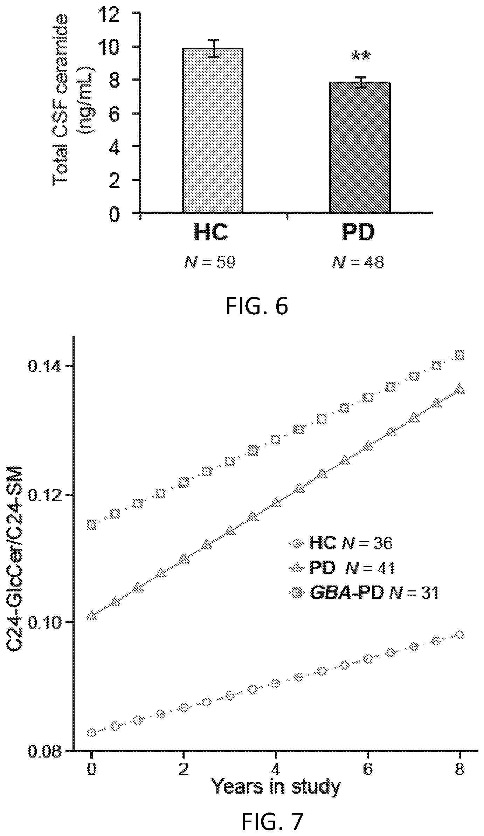

A separate experiments was performed to determine the total ceramide level in cerebrospinal fluid obtained from either healthy controls or patients having Parkinson's disease. The levels of total ceramide were determined as described in Example 1. The data show that the level of total ceramide in the cerebrospinal fluid of patients having Parkinson's disease were decreased as compared to the level of total ceramide in the cerebrospinal fluid in age- and sex-matched healthy controls (Figure 6).

A further study was performed to determine the ratio of glucosylceramide C24:0 to sphingomyelin C24:0 in plasma samples from Parkinson's disease patients without a GBA mutation (sporadic Parkinson's disease), Parkinson's disease patients having a GBA mutation, and control subjects over a period of 8 years. The ratio of glucosylceramide C24:0 to sphingomyelin C24:0 in each plasma sample was determined as described in Example 1. The data show that the ratio of glucosylceramide C24:0 to sphingomyelin C24:0 increases in plasma samples of the Parkinson's patients having or not having a GBA mutation over time (Figure 7). These data show that specific sphingolipid isoforms can be used to diagnose, monitor, and track Parkinson's disease in patients.

It is to be understood that while the invention has been described in conjunction with the detailed description thereof, the foregoing description is intended to illustrate and not limit the scope of the invention, which is defined by the scope of the appended claims.

## Claims

1. A method of determining the efficacy of a glucosyl ceramide synthase inhibitor treatment for Parkinson's disease in a subject having Parkinson's disease, the method comprising:
(a) providing a first sample comprising plasma obtained from a subject having Parkinson's disease at a first time point;
(b) determining the level at least one sphingolipid in the sample of (a), wherein the at least one sphingolipid is selected from the group consisting of:
sphingomyelin C24:1;
sphingomyelin C24:0; and
sphingomyelin C23:0;
(c) providing a second sample comprising plasma obtained from the subject at a second time point, wherein the subject has been administered said treatment between the first time point and the second time point, and performing step (b) on the second sample; and
(d) identifying the administered treatment as being effective when the level(s) of the at least one sphingolipid is increased at the second time point as compared to the first time point.

2. The method of claim 1, wherein the method further comprises:
determining the level of at least one sphingolipid selected from the group consisting of: dihexosylceramide C24:1, dihexosylceramide C24:0, dihexosylceramide C23:0, dihexosylceramide C22:0, dihexosylceramide C20:0, dihexosylceramide C18:0, dihexosylceramide C16:0, lactosylceramide C24:1, lactosylceramide C24:0, lactosylceramide C23:0, lactosylceramide C22:0, lactosylceramide C20:0, lactosylceramide C18:0, ceramide C24:1, ceramide C24:0, ceramide C23:0, ceramide C22:0, ceramide C20:0, ceramide C18:0, ceramide C16:0, ceramide C14:0, globotriaosylceramide C24:1, globotriaosylceramide C24:0, globotriaosylceramide C23:0, globotriaosylceramide C22:0, globotriaosylceramide C20:0, globotriaosylceramide C18:0, globotriaosylceramide C16:0, galactosylceramide C24:1, galactosylceramide C24:0, galactosylceramide C23:0, galactosylceramide C22:0, galactosylceramide C20:0, galactosylceramide C18:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylsphingosine; and
further identifying the administered treatment as being effective when the level(s) of the at least one sphingolipid selected from the group consisting of dihexosylceramide C24:1, dihexosylceramide C24:0, dihexosylceramide C23:0, dihexosylceramide C22:0, dihexosylceramide C20:0, dihexosylceramide C18:0, dihexosylceramide C16:0, lactosylceramide C24:1, lactosylceramide C24:0, lactosylceramide C23:0, lactosylceramide C22:0, lactosylceramide C20:0, lactosylceramide C18:0, ceramide C24:1, ceramide C24:0, ceramide C23:0, ceramide C22:0, ceramide C20:0, ceramide C18:0, ceramide C16:0, ceramide C14:0, globotriaosylceramide C24:1, globotriaosylceramide C24:0, globotriaosylceramide C23:0, globotriaosylceramide C22:0, globotriaosylceramide C20:0, globotriaosylceramide C18:0, globotriaosylceramide C16:0, galactosylceramide C24:1, galactosylceramide C24:0, galactosylceramide C23:0, galactosylceramide C22:0, galactosylceramide C20:0, galactosylceramide C18:0, galactosylceramide C16:0, glucosylceramide C24:1, glucosylceramide C24:0, glucosylceramide C23:0, glucosylceramide C22:0, glucosylceramide C20:0, glucosylceramide C18:0, and glucosylsphingosine is decreased as compared to level(s) of the at least one sphingolipid present in a healthy subject.

3. The method of claim 1, wherein the method further comprises:
further identifying the administered treatment as being effective when the level(s) of the at least one sphingolipid selected from the group consisting of sphingomyelin C24:1, sphingomyelin C24:0, and sphingomyelin C23:0 is also increased as compared to level(s) of the at least one sphingolipid present in a healthy subject.

4. The method of claim 1, wherein the glucosyl ceramide synthase inhibitor is selected from the group consisting of:
(i) eliglustat;
(ii) miglustat;
(iii) quinuclidin-3-yl (2-(4'-fluoro-[1,1'-biphenyl]-3-yl)propan-2-yl)carbamate;
(iv) (S)-quinuclidin-3-yl (2-(2-(4-fluorophenyl)thiazol-4-yl)propan-2-yl)carbamate; and
(v) (S)-quinuclidin-3-yl (2-(4'-(2-methoxyethoxy)-[1,1'-biphenyl]-4-yl)propan-2-yl)carbamate; and the pharmaceutically acceptable salts and prodrugs thereof.

## Patentansprüche

1. Verfahren zur Bestimmung der Wirksamkeit einer Glucosylceramidsynthaseinhibitor-Behandlung für Parkinson-Krankheit bei einem Individuum mit Parkinson-Krankheit, wobei das Verfahren umfasst:
(a) Bereitstellen einer ersten Probe, die Plasma umfasst, das von einem Individuum mit Parkinson-Krankheit zu einem ersten Zeitpunkt erhalten wurde;
(b) Bestimmen des Spiegels wenigstens eines Sphingolipids in der Probe von (a), wobei das wenigstens eine Sphingolipid ausgewählt ist aus der Gruppe bestehend aus:
Sphingomyelin C24:1;
Sphingomyelin C24:0; und
Sphingomyelin C23:0;
(c) Bereitstellen einer zweiten Probe, die Plasma umfasst, das von dem Individuum zu einem zweiten Zeitpunkt erhalten wurde, wobei dem Individuum zwischen dem ersten Zeitpunkt und dem zweiten Zeitpunkt die Behandlung verabreicht worden ist, und Durchführen von Schritt (b) an der zweiten Probe; und
(d) Identifizieren der verabreichten Behandlung als wirksam, wenn der oder die Spiegel des wenigstens einen Sphingolipids zu dem zweiten Zeitpunkt im Vergleich zu dem ersten Zeitpunkt erhöht sind.

2. Verfahren nach Anspruch 1, wobei das Verfahren ferner umfasst:
Bestimmen des Spiegels wenigstens eines Sphingolipids ausgewählt aus der Gruppe bestehend aus: Dihexosylceramid C24:1, Dihexosylceramid C24:0, Dihexosylceramid C23:0, Dihexosylceramid C22:0, Dihexosylceramid C20:0, Dihexosylceramid C18:0, Dihexosylceramid C16:0, Lactosylceramid C24:1, Lactosylceramid C24:0, Lactosylceramid C23:0, Lactosylceramid C22:0, Lactosylceramid C20:0, Lactosylceramid C18:0, Ceramid C24:1, Ceramid C24:0, Ceramid C23:0, Ceramid C22:0, Ceramid C20:0, Ceramid C18:0, Ceramid C16:0, Ceramid C14:0, Globotriaosylceramid C24:1, Globotriaosylceramid C24:0, Globotriaosylceramid C23:0, Globotriaosylceramid C22:0, Globotriaosylceramid C20:0, Globotriaosylceramid C18:0, Globotriaosylceramid C16:0, Galactosylceramid C24:1, Galactosylceramid C24:0, Galactosylceramid C23:0, Galactosylceramid C22:0, Galactosylceramid C20:0, Galactosylceramid C18:0, Galactosylceramid C16:0, Glucosylceramid C24:1, Glucosylceramid C24:0, Glucosylceramid C23:0, Glucosylceramid C22:0, Glucosylceramid C20:0, Glucosylceramid C18:0 und Glucosylsphingosin; und
ferner Identifizieren der verabreichten Behandlung als wirksam, wenn der oder die Spiegel des wenigstens einen Sphingolipids ausgewählt aus der Gruppe bestehend aus Dihexosylceramid C24:1, Dihexosylceramid C24:0, Dihexosylceramid C23:0, Dihexosylceramid C22:0, Dihexosylceramid C20:0, Dihexosylceramid C18:0, Dihexosylceramid C16:0, Lactosylceramid C24:1, Lactosylceramid C24:0, Lactosylceramid C23:0, Lactosylceramid C22:0, Lactosylceramid C20:0, Lactosylceramid C18:0, Ceramid C24:1, Ceramid C24:0, Ceramid C23:0, Ceramid C22:0, Ceramid C20:0, Ceramid C18:0, Ceramid C16:0, Ceramid C14:0, Globotriaosylceramid C24:1, Globotriaosylceramid C24:0, Globotriaosylceramid C23:0, Globotriaosylceramid C22:0, Globotriaosylceramid C20:0, Globotriaosylceramid C18:0, Globotriaosylceramid C16:0, Galactosylceramid C24:1, Galactosylceramid C24:0, Galactosylceramid C23:0, Galactosylceramid C22:0, Galactosylceramid C20:0, Galactosylceramid C18:0, Galactosylceramid C16:0, Glucosylceramid C24:1, Glucosylceramid C24:0, Glucosylceramid C23:0, Glucosylceramid C22:0, Glucosylceramid C20:0, Glucosylceramid C18:0 und Glucosylsphingosin im Vergleich zu dem oder den Spiegeln des wenigstens einen Sphingolipids, das bei einem gesunden Individuum vorhanden ist, verringert ist.

3. Verfahren nach Anspruch 1, wobei das Verfahren ferner umfasst:
ferner Identifizieren der verabreichten Behandlung als wirksam, wenn der oder die Spiegel des wenigstens einen Sphingolipids ausgewählt aus der Gruppe bestehend aus Sphingomyelin C24:1, Sphingomyelin C24:0 und Sphingomyelin C23:0, im Vergleich zu dem oder den Spiegeln des wenigstens einen Sphingolipids, das in einem gesunden Individuum vorhanden ist, erhöht ist.

4. Verfahren nach Anspruch 1, wobei der Glucosylceramidsynthaseinhibitor ausgewählt ist aus der Gruppe bestehend aus:
(i) Eliglustat;
(ii) Miglustat;
(iii) Chinuclidin-3-yl-(2-(4'-fluor-[1,1'-biphenyl]-3-yl)propan-2-yl)carbamat;
(iv) (S)-Chinuclidin-3-yl-(2-(2-(4-fluorphenyl)thiazol-4-yl)propan-2-yl)carbamat; und
(v) (S)-Chinuclidin-3-yl-(2-(4'-(2-methoxyethoxy)-[1,1'-biphenyl]-4-yl)propan-2-yl)carbamat; und den pharmazeutisch akzeptablen Salzen und Prodrugs davon.

## Revendications

1. Procédé de détermination de l'efficacité d'un traitement par un inhibiteur de la glucosylcéramide synthase contre la maladie de Parkinson chez un sujet atteint de la maladie de Parkinson, le procédé comprenant :
(a) la mise à disposition d'un premier échantillon comprenant du plasma obtenu à partir d'un sujet atteint de la maladie de Parkinson à un premier point temporel ;
(b) la détermination du niveau d'au moins un sphingolipide dans l'échantillon de (a), ledit au moins un sphingolipide étant choisi dans le groupe constitué par :
la sphingomyéline C24:1 ;
la sphingomyéline C24:0 ; et
la sphingomyéline C23:0 ;
(c) la mise à disposition d'un second échantillon comprenant du plasma obtenu à partir du sujet à un second point temporel, le sujet ayant reçu ledit traitement entre le premier point temporel et le second temps temporel, et la mise en œuvre de l'étape (b) sur le second échantillon ; et
(d) l'identification du fait que le traitement administré est efficace lorsque le(s) niveau(x) dudit au moins un sphingolipide est/sont augmenté(s) au second point temporel par rapport au premier point temporel.

2. Procédé selon la revendication 1, le procédé comprenant en outre :
la détermination du niveau d'au moins un sphingolipide choisi dans le groupe constitué par : le dihexosylcéramide C24:1, le dihexosylcéramide C24:0, le dihexosylcéramide C23:0, le dihexosylcéramide C22:0, le dihexosylcéramide C20:0, le dihexosylcéramide C18:0, le dihexosylcéramide C16:0, le lactosylcéramide C24:1, le lactosylcéramide C24:0, le lactosylcéramide C23:0, le lactosylcéramide C22:0, le lactosylcéramide C20:0, le lactosylcéramide C18:0, le céramide C24:1, le céramide C24:0, le céramide C23:0, le céramide C22:0, le céramide C20:0, le céramide C18:0, le céramide C16:0, le céramide C14:0, le globotriaosylcéramide C24:1, le globotriaosylcéramide C24:0, le globotriaosylcéramide C23:0, le globotriaosylcéramide C22:0, le globotriaosylcéramide C20:0, le globotriaosylcéramide C18:0, le globotriaosylcéramide C16:0, le galactosylcéramide C24:1, le galactosylcéramide C24:0, le galactosylcéramide C23:0, le galactosylcéramide C22:0, le galactosylcéramide C20:0, le galactosylcéramide C18:0, le galactosylcéramide C16:0, le glucosylcéramide C24:1, le glucosylcéramide C24:0, le glucosylcéramide C23:0, le glucosylcéramide C22:0, le glucosylcéramide C20:0, le glucosylcéramide C18:0 et la glucosylsphingosine ; et
l'identification supplémentaire du fait que le traitement administré est efficace lorsque le(s) niveau(x) dudit au moins un sphingolipide choisi dans le groupe constitué par le dihexosylcéramide C24:1, le dihexosylcéramide C24:0, le dihexosylcéramide C23:0, le dihexosylcéramide C22:0, le dihexosylcéramide C20:0, le dihexosylcéramide C18:0, le dihexosylcéramide C16:0, le lactosylcéramide C24:1, le lactosylcéramide C24:0, le lactosylcéramide C23:0, le lactosylcéramide C22:0, le lactosylcéramide C20:0, le lactosylcéramide C18:0, le céramide C24:1, le céramide C24:0, le céramide C23:0, le céramide C22:0, le céramide C20:0, le céramide C18:0, le céramide C16:0, le céramide C14:0, le globotriaosylcéramide C24:1, le globotriaosylcéramide C24:0, le globotriaosylcéramide C23:0, le globotriaosylcéramide C22:0, le globotriaosylcéramide C20:0, le globotriaosylcéramide C18:0, le globotriaosylcéramide C16:0, le galactosylcéramide C24:1, le galactosylcéramide C24:0, le galactosylcéramide C23:0, le galactosylcéramide C22:0, le galactosylcéramide C20:0, le galactosylcéramide C18:0, le galactosylcéramide C16:0, le glucosylcéramide C24:1, le glucosylcéramide C24:0, le glucosylcéramide C23:0, le glucosylcéramide C22:0, le glucosylcéramide C20:0, le glucosylcéramide C18:0 et la glucosylsphingosine est/sont diminué(s) par rapport au(x) niveau(x) dudit au moins sphingolipide présent chez un sujet sain.

3. Procédé selon la revendication 1, le procédé comprenant en outre :
l'identification supplémentaire du fait que le traitement administré est efficace lorsque le(s) niveau(x) dudit au moins un sphingolipide sélectionné dans le groupe constitué par la sphingomyéline C24:1, la sphingomyéline C24:0 et la sphingomyéline C23:0 est/sont également augmenté(s) par rapport au(x) niveau(x) dudit au moins un sphingolipide présent chez un sujet sain.

4. Procédé selon la revendication 1, l'inhibiteur de la glucosylcéramide synthase étant choisi dans le groupe constitué par :
(i) l'éliglustat ;
(ii) le miglustat ;
(iii) le (2-(4'-fluoro-[1,1'-biphényl]-3-yl)propan-2-yl)carbamate de quinuclidin-3-yle ;
(iv) le (2-(2-(4-fluorophényl)thiazol-4-yl)propan-2-yl)carbamate de (S)-quinuclidin-3-yle ; et
(v) le (2-(4'-(2-méthoxyéthoxy)-[1,1'-biphényl]-4-yl)propan-2-yl)carbamate de (S)-quinuclidin-3-yle ; et les sels et promédicaments pharmaceutiquement acceptables correspondants.
